# EUROPEAN PATENT SPECIFICATION

(11) **EP 3 383 418 B1**
(45) Date of publication and mention of the grant of the patent: **20.10.2021**
(21) Application number: 16871668.6
(22) Date of filing: 02.12.2016
(51) Int. Cl.: A61K 38/00, A61K 39/00, A61P 35/00, C07K 11/00, C07K 14/435

(54) **SLC45A2 PEPTIDES FOR IMMUNOTHERAPY**
SLC45A2-PEPTIDE FÜR IMMUNTHERAPIE
PEPTIDES SLC45A2 POUR L'IMMUNOTHÉRAPIE

(30) Priority: 04.12.2015 US 201562263189 P; 07.12.2015 US 201562263835 P
(43) Date of publication of application: 10.10.2018
(73) Proprietor: Board of Regents, The University of Texas System, Austin, TX 78701 (US)
(72) Inventor: LIZEE, Gregory, Houston, TX 77054 (US); YEE, Cassian, Houston, TX 77054 (US); HWU, Patrick, Houston, TX 77005 (US); ROSZIK, Janos, Houston, TX 77054 (US)
(74) Representative: Vossius & Partner Patentanwälte Rechtsanwälte mbB
(86) International application number: PCT/US2016/064825
(87) International publication number: WO 2017/096304

(56) References cited:
- WO-A1-2017/089768
- FR-A1- 2 907 681
- US-A1- 2011 086 805
- US-A1- 2011 177 042
- US-A1- 2013 217 116
- US-A1- 2014 206 574
- HARADA MAMORU ET AL: "Use of an in vitro immunoselected tumor line to identify shared melanoma antigens recognized by HLA-A*0201-restricted T cells", CANCER RESEARCH, AMERICAN ASSOCIATION FOR CANCER RESEARCH, US, vol. 61, no. 3, 1 February 2001 (2001-02-01), pages 1089-1094, XP002486099, ISSN: 0008-5472
- GUEDJ MICKAEL ET AL: "Variants of the MATP/SLC45A2 gene are protective for melanoma in the French population", HUMAN MUTATION, JOHN WILEY & SONS, INC, US, vol. 29, no. 9, 1 September 2008 (2008-09-01), pages 1154-1160, XP002520649, ISSN: 1059-7794, DOI: 10.1002/HUMU.20823 [retrieved on 2008-08-06]
- KONDO TAISUKE ET AL: "Oculocutaneous albinism: Developing novel antibodies targeting the proteins associated with OCA2 and OCA4", JOURNAL OF DERMATOLOGICAL SCIENCE, ELSEVIER, AMSTERDAM, NL, vol. 77, no. 1, 20 November 2014 (2014-11-20), pages 21-27, XP029185975, ISSN: 0923-1811, DOI: 10.1016/J.JDERMSCI.2014.11.006

### SEQUENCE LISTING

<110> BOARD OF REGENTS, THE UNIVERSITY OF TEXAS SYSTEM
<120> SLC45A2 PEPTIDES FOR IMMUNOTHERAPY
<130> UTFC.P1278WO
<150> US 62/263,835
   <151> 2015-12-07
<150> US 62/263,189
   <151> 2015-12-04
<160> 18
<170> PatentIn version 3.5
<210> 1
   <211> 9
   <212> PRT
   <213> Artificial sequence
<220>
   <223> Synthetic peptide
<400> 1
   Ser Leu Tyr Ser Tyr Phe Gln Lys Val
   1               5
<210> 2
   <211> 10
   <212> PRT
   <213> Artificial sequence
<220>
   <223> Synthetic peptide
<400> 2
   Ser Tyr Ile Gly Leu Lys Gly Leu Tyr Phe
   1               5                   10
<210> 3
   <211> 21
   <212> DNA
   <213> Artificial sequence
<220>
   <223> Synthetic primer
<400> 3
   ctggccgcca catctataaa t 21
<210> 4
   <211> 22
   <212> DNA
   <213> Artificial sequence
<220>
   <223> Synthetic primer
<400> 4
   gtagcagaac tctcttccga ac 22
<210> 5
   <211> 22
   <212> DNA
   <213> Artificial sequence
<220>
   <223> Synthetic primer
<400> 5
   acagtgatcc tgggagtctt ac 22
<210> 6
   <211> 22
   <212> DNA
   <213> Artificial sequence
<220>
   <223> Synthetic primer
<400> 6
   ttgaagagac actttgctgt cc 22
<210> 7
   <211> 19
   <212> DNA
   <213> Artificial sequence
<220>
   <223> Synthetic primer
<400> 7
   aggtgccttt ctccgtgag 19
<210> 8
   <211> 20
   <212> DNA
   <213> Artificial sequence
<220>
   <223> Synthetic primer
<400> 8
   gcttcagcca gatagccact 20
<210> 9
   <211> 21
   <212> DNA
   <213> Artificial sequence
<220>
   <223> Synthetic primer
<400> 9
   gcaaagcata ccatcagctc a 21
<210> 10
   <211> 21
   <212> DNA
   <213> Artificial sequence
<220>
   <223> Synthetic primer
<400> 10
   gcagtgcatc cattgacaca t 21
<210> 11
   <211> 20
   <212> DNA
   <213> Artificial sequence
<220>
   <223> Synthetic primer
<400> 11
   aatcccatca ccatcttcca 20
<210> 12
   <211> 20
   <212> DNA
   <213> Artificial sequence
<220>
   <223> Synthetic primer
<400> 12
   tggactccac gacgtactca 20
<210> 13
   <211> 9
   <212> PRT
   <213> Artificial sequence
<220>
   <223> Synthetic peptide
<400> 13
   Arg Leu Leu Gly Thr Glu Phe Gln Val
   1               5
<210> 14
   <211> 10
   <212> PRT
   <213> Artificial sequence
<220>
   <223> Synthetic peptide
<400> 14
   Val Trp Phe Leu Ser Pro Ile Leu Gly Phe
   1               5                   10
<210> 15
   <211> 9
   <212> PRT
   <213> Artificial sequence
<220>
   <223> Synthetic peptide
<400> 15
   Ala Leu Ile Ala Asn Pro Arg Arg Lys
   1               5
<210> 16
   <211> 9
   <212> PRT
   <213> Artificial sequence
<220>
   <223> Synthetic peptide
<400> 16
   Ser Gly Gln Ala Gly Arg His Ile Tyr
   1               5
<210> 17
   <211> 9
   <212> PRT
   <213> Artificial sequence
<220>
   <223> Synthetic peptide
<400> 17
   Ala Ala Gly Ile Gly Ile Leu Thr Val
   1               5
<210> 18
   <211> 9
   <212> PRT
   <213> Artificial sequence
<220>
   <223> Synthetic peptide
<400> 18
   Lys Thr Trp Gly Gln Tyr Trp Gln Val
   1               5

## Description

### BACKGROUND

The present disclosure relates generally to the field of immunology and medicine. More particularly, peptide fragments are disclosed which are recognized by T cells and may be used to treat a cancer.

### 2. Description of Related Art

Adoptive T cell therapy (ACT; also referred to as an "adoptive cell transfer") has shown significant promise as a method for treating melanoma; unfortunately, this approach has also been hindered by limitations including toxicity towards non-cancerous tissues. ACT generally involves which involves infusing a large number of autologous activated tumor-specific T cells into a patient, *e.g.,* to treat a cancer. ACT has resulted in therapeutic clinical responses in melanoma patients (Yee 2002; Dudley 2002; Yee 2014; Chapuis 2016). Generally, to develop effective anti-tumor T cell responses, the following three steps are normally required: priming and activating antigen-specific T cells, migrating activated T cells to tumor site, and recognizing and killing tumor by antigen-specific T cells. The choice of target antigen is important for induction of effective antigen-specific T cells.

Several antigens selected for treating melanoma with ACT have displayed significant adverse autoimmune side effects. The choice of target antigen is also important for induction of effective antigen-specific T cells. In the last decades, MART-1, gp100, and tyrosinase have been identified as human melanoma differentiation antigens (MDAs) recognized by T cell derived from human PBLs or TILs (Yee 2002, Chapuis 2012; Coulie 1994; Kawakami 1995; Harada 2001)). MDAs are also expressed by normal tissue such as melanocytes in skin and eye and by inner ear cells. Unfortunately, according to outcomes from a recent clinical trial, ACT with T cells specific for these MDAs induced unwanted autoimmune responses by destruction of normal tissues, leading to vitiligo, vision loss, and inner ear toxicity (Yee C 2000; Brichard V 1993; Seaman 2012). Identification of new target antigens for melanoma with less toxicity and optimal efficacy would be desirable. Clearly, there is a need for new antigen targets and peptides that may be used in adoptive T cell therapies.

### SUMMARY

The invention relates to the embodiments as defined in the claims. In particular, the invention relates to an isolated peptide 35 amino acids in length or less for use in the treatment of melanoma excluding uveal melanoma, wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402.

In addition, the invention relates to a cell culture medium comprising a peptide 35 amino acids in length or less, wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402.

Furthermore, the invention relates to a pharmaceutical composition comprising a peptide 35 amino acids in length or less for use in the treatment of melanoma excluding uveal melanoma, wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402 and an excipient.

The invention also relates to an *in vitro* method for inducing a population of T cells to proliferate, comprising contacting T cells *in vitro* with a peptide in an amount sufficient to bind a HLA-A*0201 or a HLA-A2 in the T cells and promote proliferation of one or more of the T cells, preferably wherein the T cells are cytotoxic T lymphocytes (CTL) and/or CD8+ T cells, wherein the peptide is 35 amino acids in length or less and wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402.

The invention further relates to an antibody that selectively binds to a peptide or that selectively binds to a peptide - HLA-A2 complex for use in the treatment of melanoma excluding uveal melanoma, preferably wherein said antibody is a monoclonal antibody, is comprised in polyclonal antisera, is an antibody fragment, is a human or humanized antibody, or is comprised in a fusion construct, a soluble fusion construct, an ImmTAC, or an immunotoxin, wherein the peptide is 35 amino acids in length or less and wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402.

Disclosed are MHC class I epitopes of SLC45A2. The antigenic SLC45A2 peptides may be used in a cancer therapy, e.g., as a cancer vaccine or in an adoptive T cell therapy. Antibodies, such as therapeutic humanized antibodies, may also be generated that selectively bind one or more of the SLC45A2 peptides or the complex formed by the binding of a SLC45A2 peptide and (HLA-A2 or HLA-A24). The SLC45A2 peptides may be used to treat a melanoma such as, *e.g.,* a cutaneous melanoma, uveal melanoma, a mucosal melanoma, or a metastatic melanoma. The present disclosure is based, in part, on the discovery that peptides of the intracellular protein SLC45A2 are provided by MHC I (HLA-A2 or HLA-A24) on the surface of tumor cells that are recognized by T-cell receptors on T cells. , SLC45A2 peptides are provided herein that can bind MHC I (HLA-A2 or HLA-A24) and can be recognized by T-cell receptors on T cells. The SLC45A2 peptides may be therapeutically used to treat a cancer, such as a melanoma. Methods for expanding a population of T cells that target SLC45A2 are also provided. Also SLC45A2 peptides are provided that can be used to generate CD8 T cells that effectively kill melanoma cells without destruction of normal melanocytes. This reduction in toxicity towards non-cancerous cells may be particularly useful for the treatment of melanomas.

As shown in the below examples, expression of SLC45A2 in melanomas and normal tissues was characterized, and SLC45A2 peptides SLC45A2₃₈₂₋₃₉₀ (SEQ ID NO:1) and SLC45A2₃₉₃₋₄₀₂ (SEQ ID NO:2) were identified as immunogenic epitopes that can selectively bind to HLA-A*0201 (HLA-A2) and HLA-A*2402 (HLA-A24), respectively, and it was observed that cytotoxic T lymphocytes (CTL) proliferated using these peptides efficiently killed a variety of melanoma cells, including multiple cutaneous melanomas, uveal melanomas, mucosal melanomas, and metastatic melanomas. Additional SLC45A2 peptides that may be used are provided in Table 4.. As shown in the below examples, these SLC45A2 peptides were shown to display antigen specific and HLA-A*0201 or HLA A*2402-restricted responses of SLC45A2-specific CD8 T cells. SLC45A2₃₈₂₋₃₉₀ (SEQ ID NO:1) and/or SLC45A2₃₉₃₋₄₀₂ (SEQ ID NO:2) may be used in various immunotherapy approaches (e.g., as a therapeutic vaccine, in an adoptive T cell therapy) to treat a melanoma.

Disclosed is an isolated peptide 35 amino acids in length or less and comprising the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402. The peptide may be 30 or less, 25 or less, 20 or less, or 15 or less amino acids in length. The peptide comprises or consists of SLC45A2₃₈₂₋₃₉₀ (SEQ ID NO:1), wherein the peptide selectively binds HLA-A2 or HLA-A*0201The peptide may comprise or consist of SLC45A2₃₉₃₋₄₀₂ (SEQ ID NO:2),wherein the peptide selectively binds HLA-A24 or HLA-A*2402. The peptide may be comprised in a pharmaceutical preparation. The pharmaceutical preparation may be formulated for parenteral administration, intravenous injection, intramuscular injection, inhalation, or subcutaneous injection. The peptide may be comprised in a liposome, lipid-containing nanoparticle, or in a lipid-based carrier. The pharmaceutical preparation may be formulated for injection or inhalation as a nasal spray. The peptide may be comprised in a cell culture media.

Provided is also a cell culture media comprising the peptide as described above.

Further disclosed is a pharmaceutical composition comprising the peptide as described above and an excipient. The pharmaceutical preparation may be formulated for parenteral administration, intravenous injection, intramuscular injection, inhalation, or subcutaneous injection. , The peptide may be comprised in a liposome, lipid-containing nanoparticle, or in a lipid-based carrier.

Also disclosed is a composition comprising a peptide as described above, for use in therapeutic treatment. The composition may be for use in the treatment of a melanoma. The peptide may be 25 or less, 20 or less, or 15 or less amino acids in length. The peptide may comprise or consist of SLC45A2₃₈₂₋₃₉₀ (SEQ ID NO: 1). The peptide may comprise or consist of SLC45A2₃₉₃₋₄₀₂ (SEQ ID NO:2). The peptide may be comprised in a pharmaceutical preparation. The pharmaceutical preparation may be formulated for parenteral administration, intravenous injection, intramuscular injection, inhalation, or subcutaneous injection. The peptide may be comprised in a liposome, lipid-containing nanoparticle, or in a lipid-based carrier. The pharmaceutical preparation may be formulated for injection or inhalation as a nasal spray. The peptide may be produced via peptide synthesis. The peptide may be recombinantly produced. The melanoma may be a cutaneous melanoma, a uveal melanoma, a mucosal melanoma, or a metastatic melanoma.

Also disclosed is a method of treating a melanoma in a mammalian subject, comprising administering to the subject an effective amount of the peptide as described herein. The peptide may be comprised in a pharmaceutical preparation. The pharmaceutical preparation may be formulated for parenteral administration, intravenous injection, intramuscular injection, inhalation, or subcutaneous injection. The subject may be a human. The melanoma may be a cutaneous melanoma, an uveal melanoma, a mucosal melanoma, or a metastatic melanoma. The subject may be administered a second anti-cancer therapy. The second anti-cancer therapy may be selected from the group consisting of chemotherapy, a radiotherapy, an immunotherapy, or a surgery. The peptide may be administered to the subject in an amount effective to promote cytotoxic T lymphocytes (CTL) in the subject to lyse or kill cancerous cells in the subject.

Further provided is an *in vitro* method for inducing a population of T cells to proliferate, comprising contacting T cells *in vitro* with a peptide of any one of claims 1-12 in an amount sufficient to bind a HLA-A*0201 or a HLA-A2 in the T cells and promote proliferation of one or more of the T cells. The T cells may be cytotoxic T lymphocytes (CTL). The T cells may be CD8+ T cells. The method may further comprise administering the T cells to a subject after said proliferation. The subject may be a mammalian subject such as, *e.g.,* a human.

Further disclosed is a method of promoting an immune response in a subject against SLC45A2, comprising administering to the subject a peptide as described herein in an amount effective to cause proliferation of T cells that selectively target SLC45A2. The T cells may be cytotoxic T lymphocytes. The subject may be a human. The subject may have a melanoma. The melanoma may be a cutaneous melanoma, a uveal melanoma, a mucosal melanoma, or a metastatic melanoma. The subject may not have cancer.

Also disclosed is an isolated nucleic acid encoding a peptide as described above. The nucleic acid may be a DNA or an RNA. Avector comprising a contiguous sequence consisting of the nucleic acid segment is also disclosed. The vector may further comprise a heterologous promoter. The nucleic acid or vector may be comprised in a minigene, a plasmid, or an RNA; for example, the nucleic acid or vector may be used, *e.g.,* to engineer expression of the epitope in an antigen-presenting cells *(e.g.,* a dendritic cell, an artificial APC, or a T cell).

Also disclosed is an isolated antibody that selectively binds to a peptide as described herein. The antibody may be a monoclonal antibody, is comprised in polyclonal antisera, or is an antibody fragment. The antibody may be a human or humanized antibody. The antibody may be comprised in a fusion construct, a soluble fusion construct, an ImmTAC, or an immunotoxin; for example a variety of moieties may be attached to the antibody to achieve an additional therapeutic effect, *e.g.,* as described in Oates *et al.* (2015) and Liddy *et al.* (2012). For example the antibody may be fused to a humanized cluster of differentiation 3 (CD3)-specific single-chain antibody fragment (scFv).

Further disclosed is an isolated antibody that selectively binds to a peptide - HLA-A2 complex, wherein the peptide - HLA-A2 complex comprises the peptide of any one of claims 1-12 bound to a HLA-A2. The antibody may be a monoclonal antibody, may be comprised in polyclonal antisera, or may be an antibody fragment. The antibody may be a human or humanized antibody.

Also disclosed is a kit comprising a peptide as described above in a container. The peptide may be comprised in a pharmaceutical preparation. The pharmaceutical preparation may be formulated for parenteral administration or inhalation. The peptide may be comprised in a cell culture media.

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.05%, preferably below 0.01%. Most preferred is a composition in which no amount of the specified component can be detected with standard analytical methods.

HLA-A2 refers to the human leukocyte antigen serotype A2 and is also referred to as HLA-A*02. Several serotypes of the gene products of many HLA-A*02 alleles are well known, including HLA-A*0201, *0202, *0203, *0206, *0207, and *0211 gene products.

HLA-A24 refers to the human leukocyte antigen serotype A24 and is also referred to as HLA-A*24. Several serotypes of the gene products of many HLA-A*24 alleles are well known, including HLA-A*2402 and *2403 gene products.

The terms "inhibiting," "reducing," or "prevention," or any variation of these terms, when used in the claims and/or the specification includes any measurable decrease or complete inhibition to achieve a desired result.

The term "effective," as that term is used in the specification and/or claims, means adequate to accomplish a desired, expected, or intended result.

The use of the word "a" or "an" when used in conjunction with the term "comprising" in the claims and/or the specification may mean "one," but it is also consistent with the meaning of "one or more," "at least one," and "one or more than one."

It is contemplated that any disclosure discussed in this specification can be implemented with respect to any method or composition disclosed herein, and vice versa. Furthermore, disclosed compositions can be used to achieve disclosed methods..

Throughout this application, the term "about" is used to indicate that a value includes the inherent variation of error for the device, the method being employed to determine the value, or the variation that exists among the study subjects.

The use of the term "or" in the claims is used to mean "and/or" unless explicitly indicated to refer to alternatives only or the alternatives are mutually exclusive, although the disclosure supports a definition that refers to only alternatives and "and/or."

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

Other objects, features and advantages disclosed herein will become apparent from the following detailed description. It should be understood, however, that the detailed description and the specific examples are given by way of illustration only.

### BRIEF DESCRIPTION OF THE DRAWINGS

The following drawings form part of the present specification. The disclosure may be better understood by reference to one or more of these drawings in combination with the detailed description.

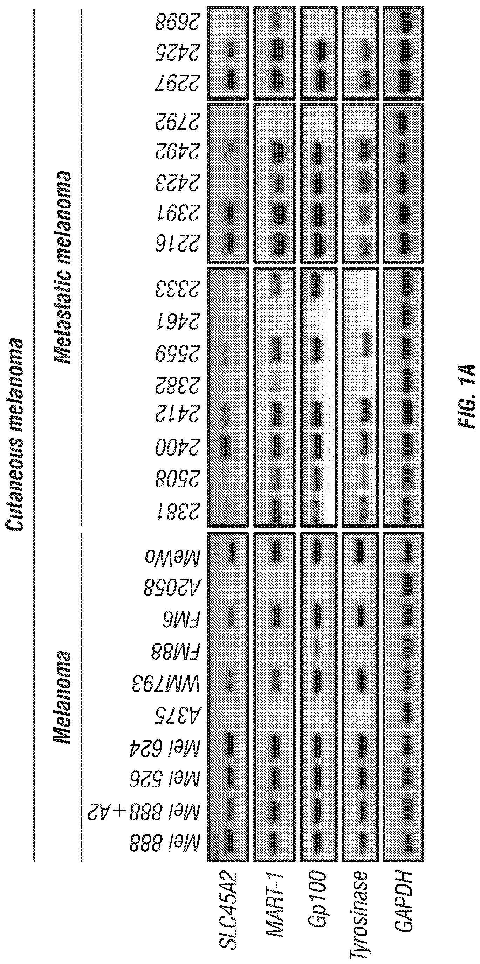

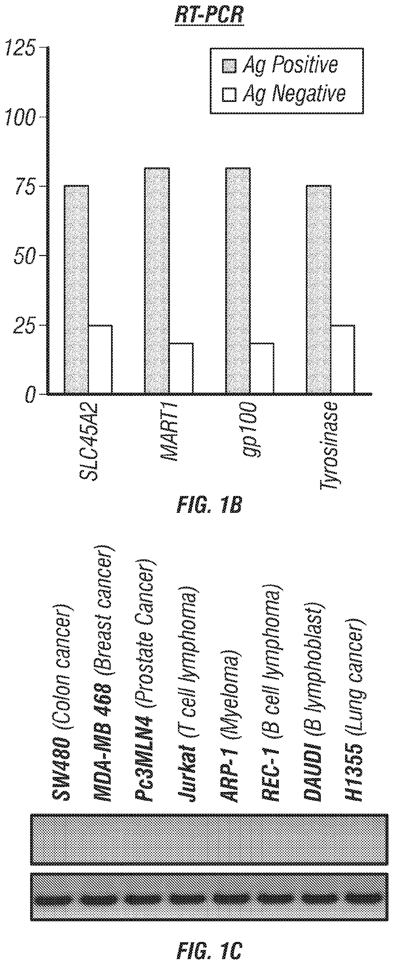

**FIGS. 1A****-C:** Expression of SLC45A2 in cutaneous melanoma cell lines and the high-restricted tissue. **FIG. 1A****,** Summary of results showing MDA expression in 24 cutaneous melanoma cell lines, as determined by RT-PCR. **FIG. 1B****,** SLC45A2 mRNA expression in cutaneous melanoma cells. SLC45A2 mRNA was detected in most melanoma cells including metastatic melanoma cells originated from different sites by RT-PCR analysis as other melanocyte differentiation antigens such as MART-1, gp100 and tyrosinase. **FIG. 1C****,** No SLC45A2 mRNA expression in various tumor type cells by RT-PCR analysis. Tumor cells of different types except melanomas didn't express SLC45A2.

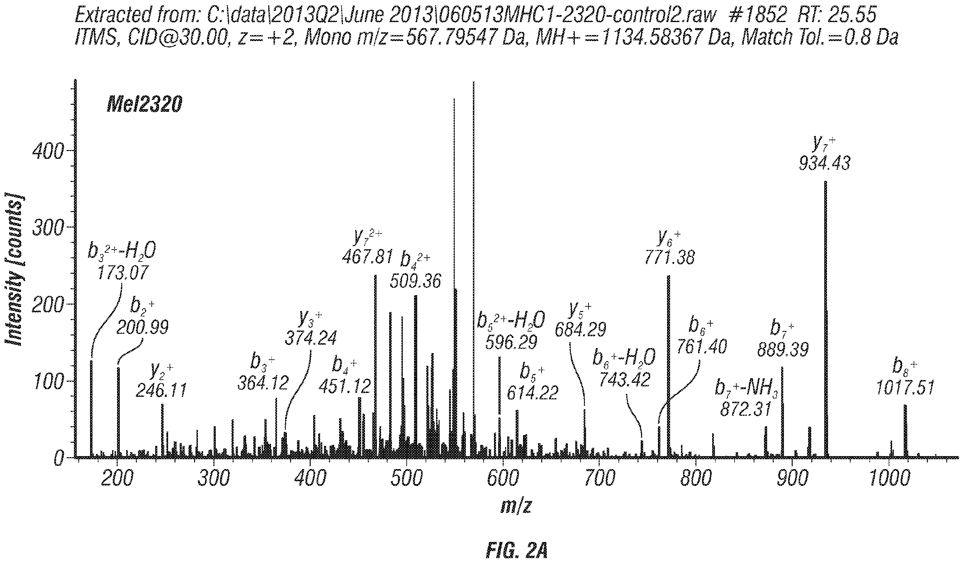

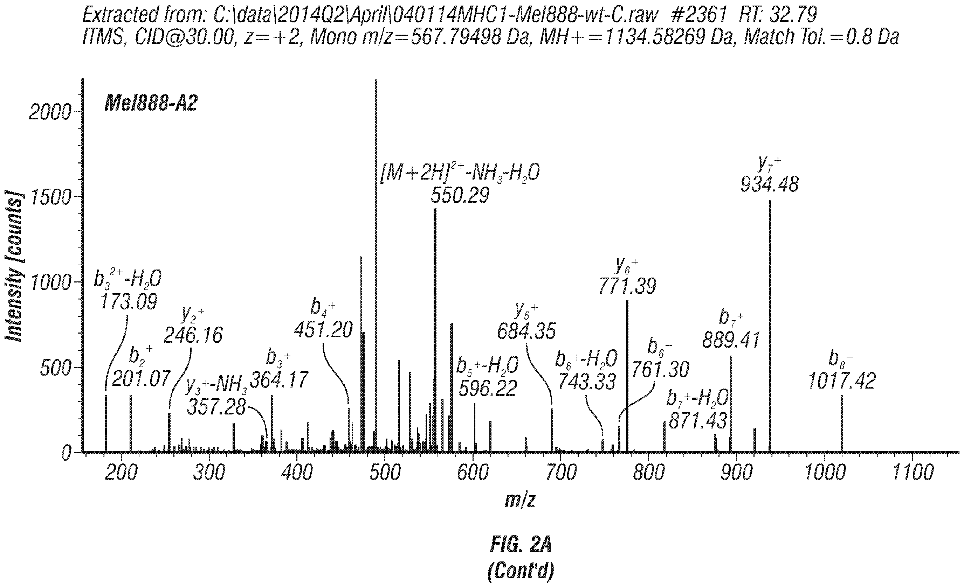

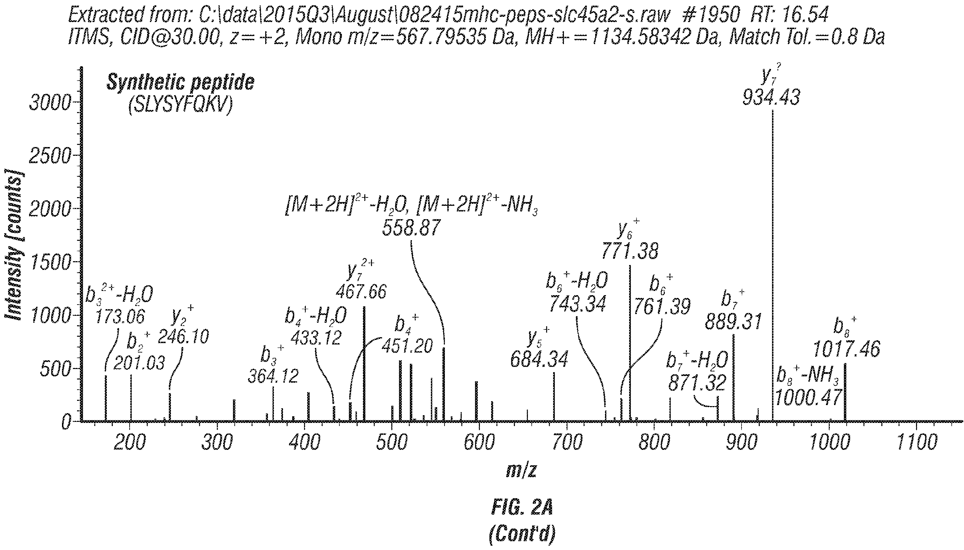

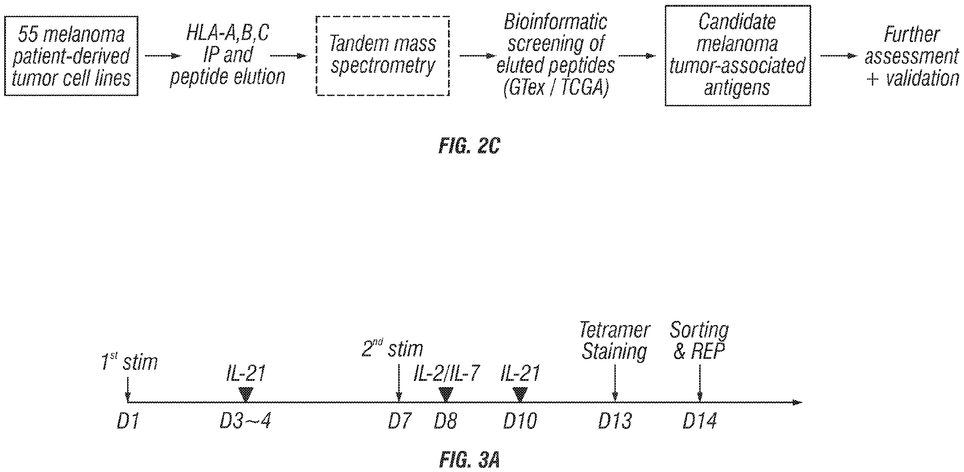

**FIGS. 2A****-B:** Mass spectra of tumor-derived and synthetic SLC45A2-derived peptides. **FIG: 2A****,** HLA-A*0201 restricted SLC45A2 peptide. **FIG: 2B****,** HLA-A*2402 restricted peptide. **FIG. 2C****,** Experimental strategy to identify melanoma tumor-specific peptides from melanoma cell lines.

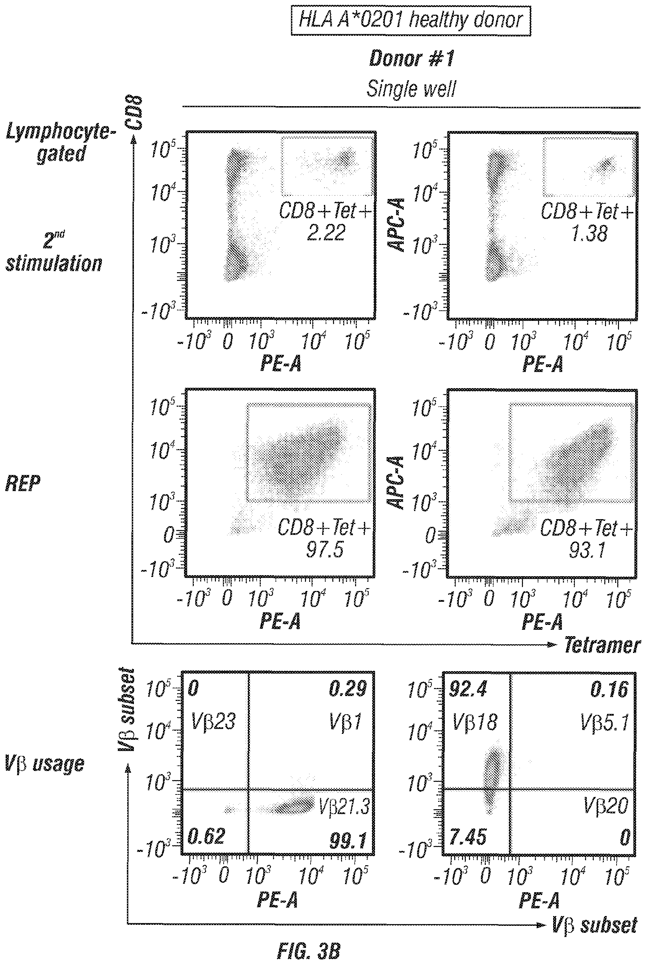

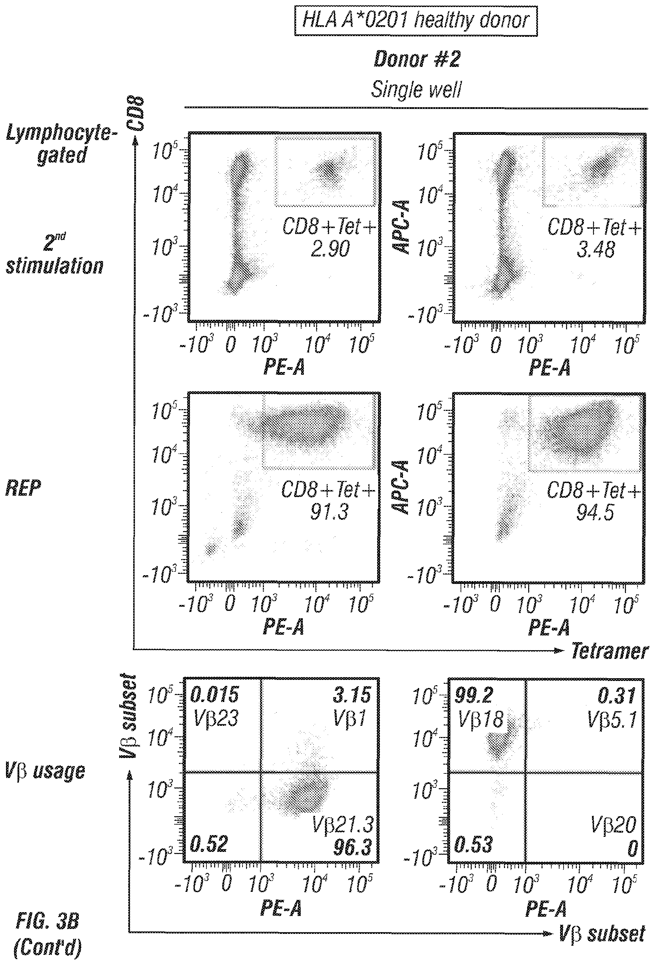

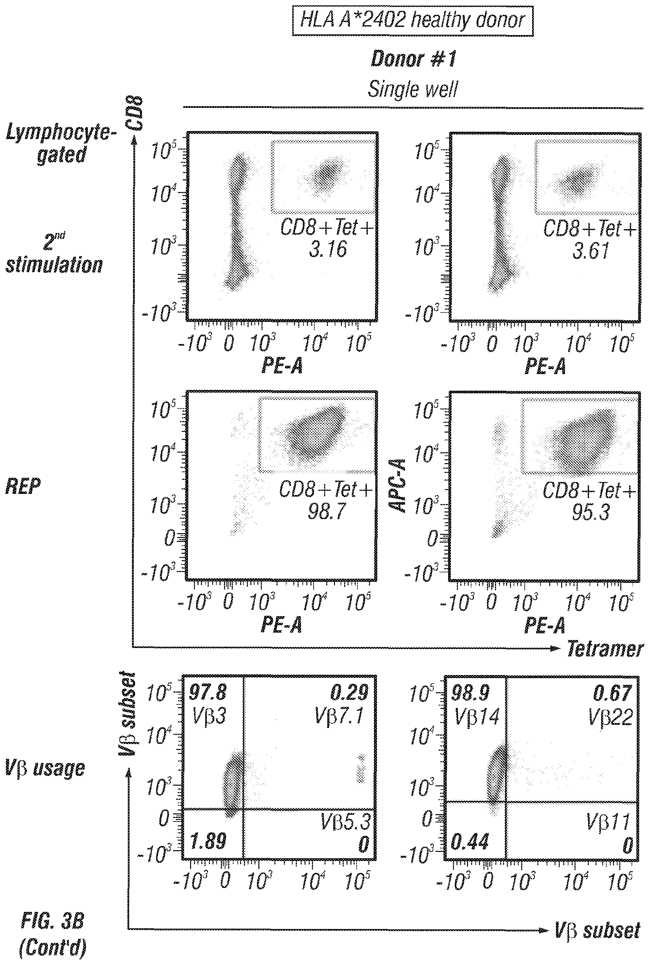

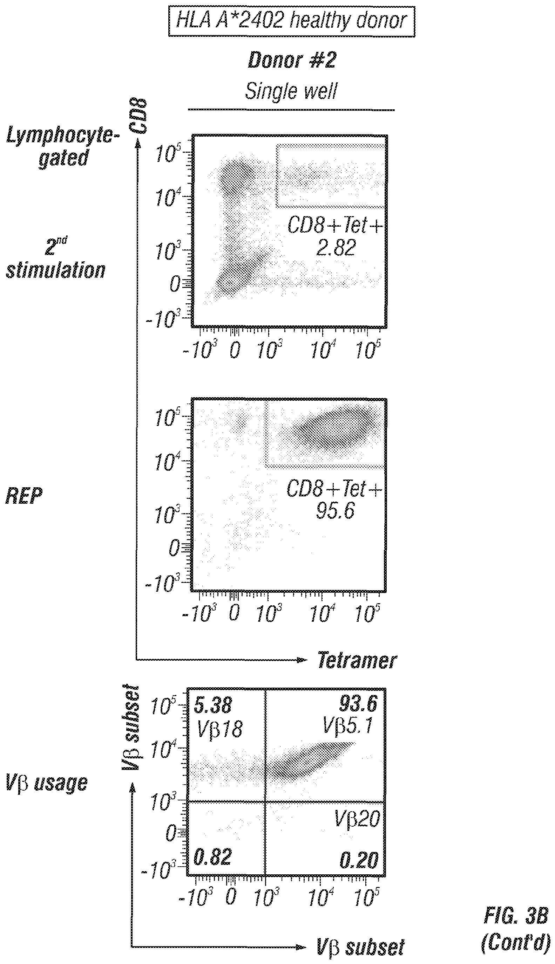

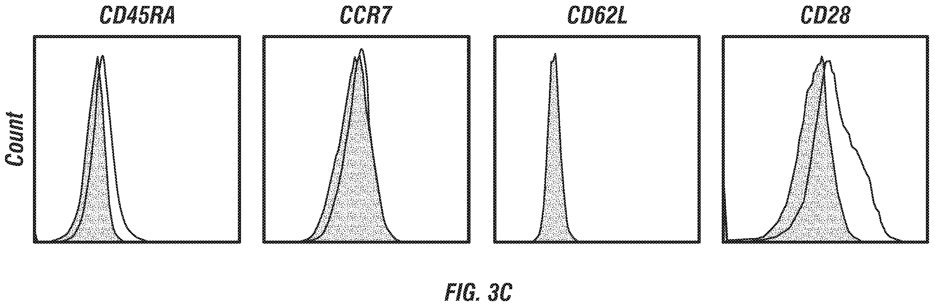

**FIGS. 3A****-C:** Generation of SLC45A2-specific CD8 T cells in PBMCs of HLA A*0201 or A*2402 -restricted healthy donors. **FIG. 3A****,** The schedule for generation of SLC45A2-specific CD8 T cells. **FIG. 3B****,** Induction of SLC45A2-tetramer positive CD8 T cells. PBMC from HLA A*0201 or A*2402 restricted healthy donors was stimulated with autologous SLC45A2₃₈₂₋₃₉₀ peptide or SLC45A2₃₉₃₋₄₀₂ peptide -pulsed DC respectively. SLC45A2 tetramer-positive CD8 T cells were sorted by ARIA sorter after 2 times stimulation (top panel) and the sorted SLC45A2-tetramer positive CD8 T cells were expanded according to rapid expansion protocol (REP). The expanded cells were then used as SLC45A2-specific CD8 T cells (middle panel). TCR repertoire analysis of SLC45A2-specific CD8 T cells was performed using the IOTest Beta Mark TCR-Vβ repertoire kit with Vβ antibodies corresponding to 24 different specificities (bottom panel). **FIG. 3C****,** Phenotype of SLC45A2-specific CD8 T cells. 14 days after REP, phenotype was tested using antibodies for CD45RA, CCR7, CD62L and CD28 by flow cytometry.

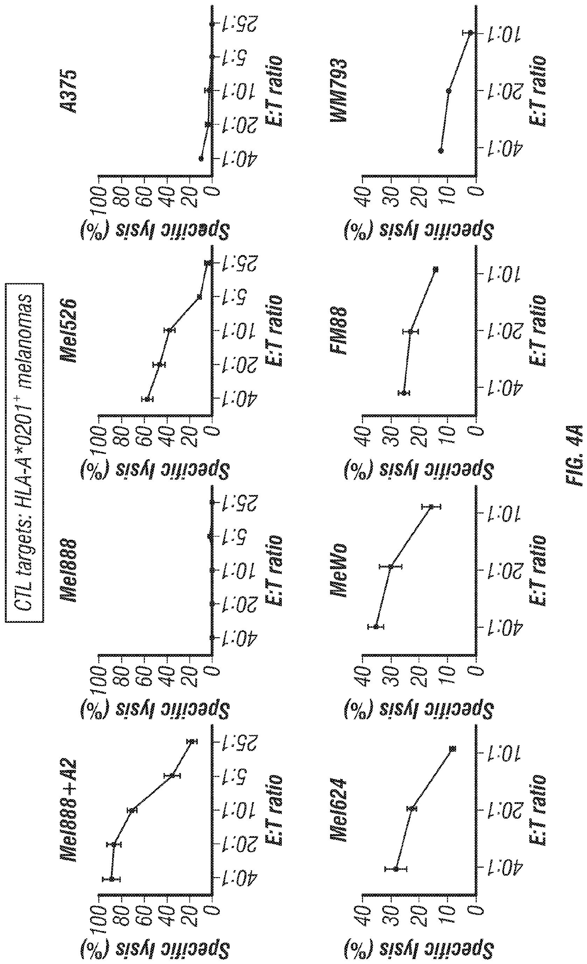

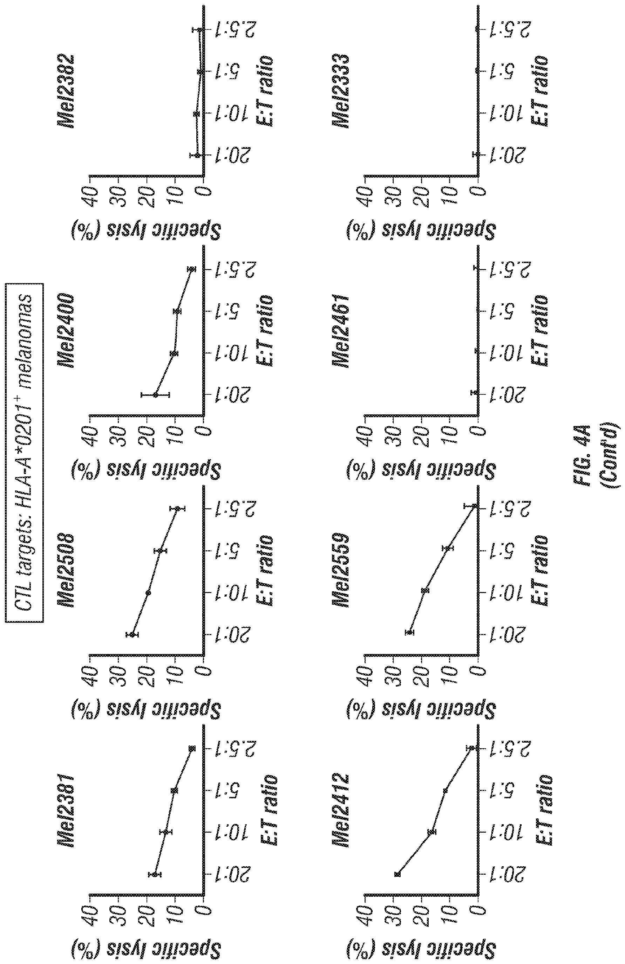

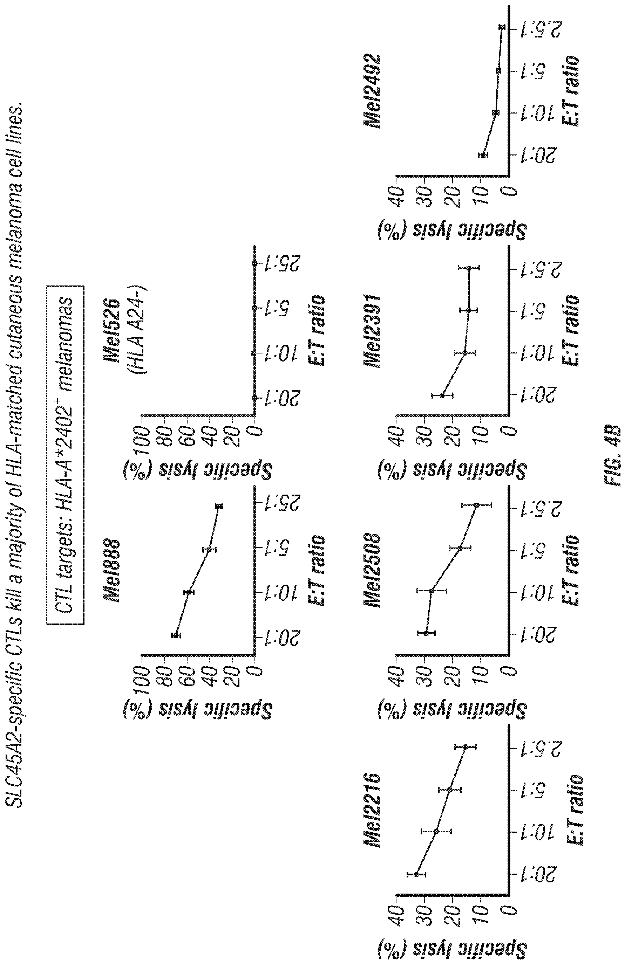

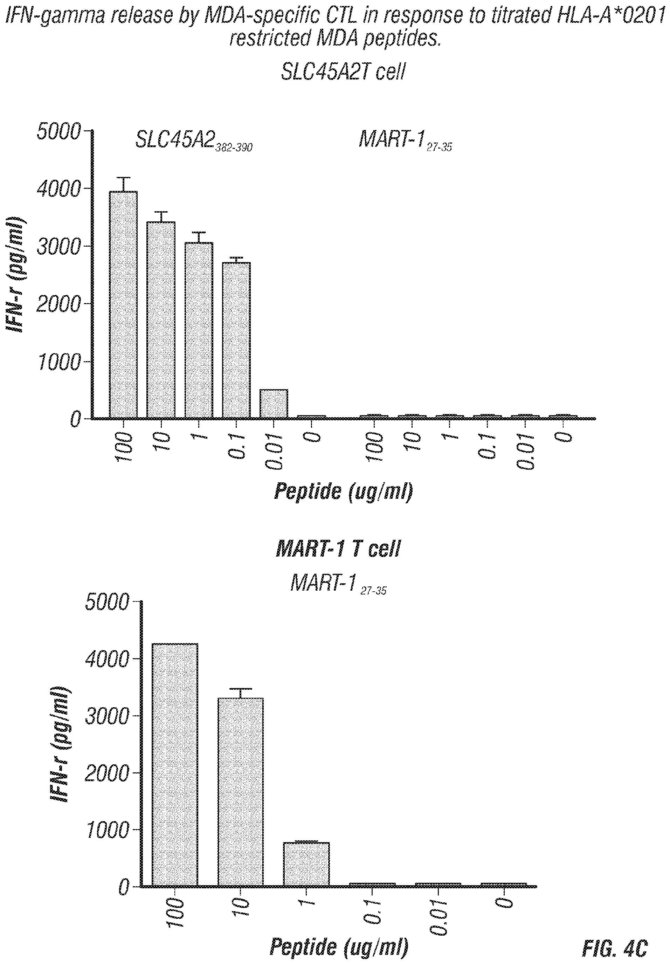

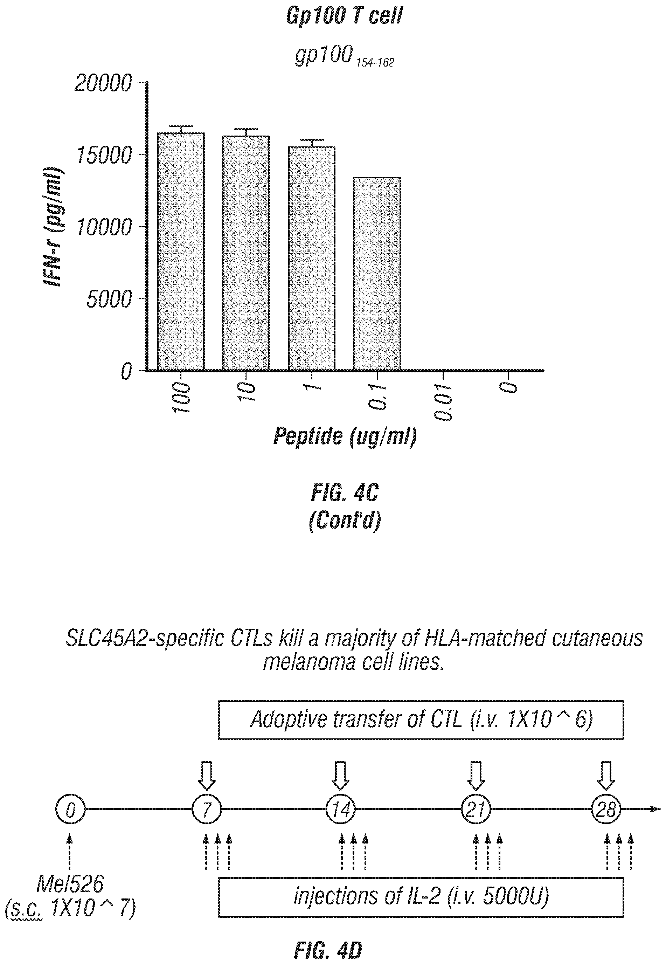

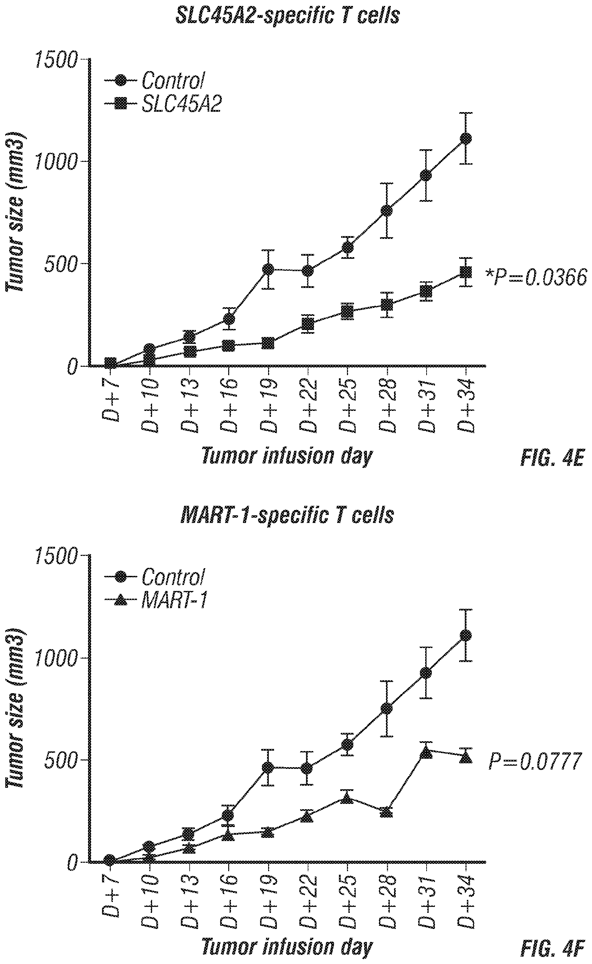

**FIGS. 4A****-F:** Effector function of SLC45A2-specific CD8 T cells. **FIG. 4A****,** The killing effect of SLC45A2-specific CD8 T cells restricted A*0201 on cutaneous melanoma cells. SLC45A-specific CD8 T cells recognized and killed HLA A*0201 restricted melanoma cells endogenously expressing SLC45A2 (HLA-A*0201+/SLC45A2+: Mel888 transduced with A2, Mel526, Mel624 and MeWo). SLC45A2-specific CD8 T cells did not kill Mel888 which expressed SLC45A2 but did not express HLA A*0201(HLA-A*0201-/SLC45A2+) and A375 which expressed HLA A*0201 but not SLC45A2 (HLA-A*0201+/SLC45A2-). SLC45A2-specific CD8 T cells showed killing effect against metastatic melanomas expressing HLA-A*0201 and SLC45A2+. SLC45A2-specific CD8 T cells from donor #1 were used. Standard ⁵¹Cr release assay for cytotoxic activity was performed in different E:T ratio. Results of 1 representative experiment of at least 3 performed was shown. **FIG. 4B****,** The killing effect of SLC45A-specific CD8 T cells restricted A*2402 on cutaneous melanoma cells. SLC45A-specific CD8 T cells restricted A*2402 killed cutaneous melanoma cells expressing SLC45A2 and HLA A*2402. Standard ⁵¹Cr release assay for cytotoxic activity was performed in different E:T ratio. Results of 1 representative experiment of at least 2 performed was shown. **FIG. 4C****,** Functional avidity of SLC45A-specific CD8 T cells. SLC45A2-specific CD8 T cells were cultured with T2 cells pre-incubated with SLC45A2₃₈₂₋₃₉₀ peptide and unmatched peptide, MART-1₂₇₋₃₅ at various concentrations (100, 10, 1, 0.1, 0.01, 0 nM) (upper panel). MART-1 or gp100 -specific CD8 T cells were cultured with T2 cells pre-incubated with M₂₇₋₃₅ or G₁₅₄₋₁₆₂ peptide at the indicated concentration (bottom panel). 48 hours after incubation, IFN-γ production was measured by ELISA assay. Peptide dose threshold of SLC45A2, MART-1-and gp100-specific CD8 T cells was measured for comparison of peptide sensitivity of antigen-specific CD8 T cells. **FIG. 4D****,** Schematic showing the experimental timeline of adoptive T-cell transfer using a melanoma xenograft model. **FIGS. 4E-F****,** Tumor growth curves showing the therapeutic effect of adoptively transferred (**FIG. 4E**) SLC45A2- or (**FIG. 4F**) MART1-specific CTLs against human melanoma Mel526 xenografts. Nude mice were inoculated subcutaneously with 1x10⁷ Mel526 cells. Seven days following tumor challenge, SLC45A2-specific or MART-1-specific CTLs (1x10⁷) were injected intravenously once per week for 4 weeks and tumor growth was monitored.

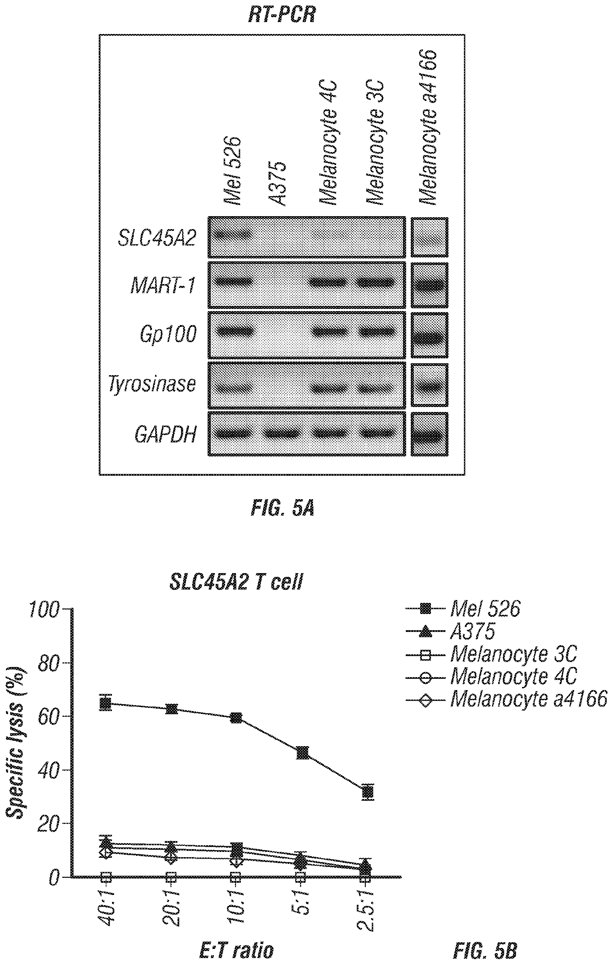

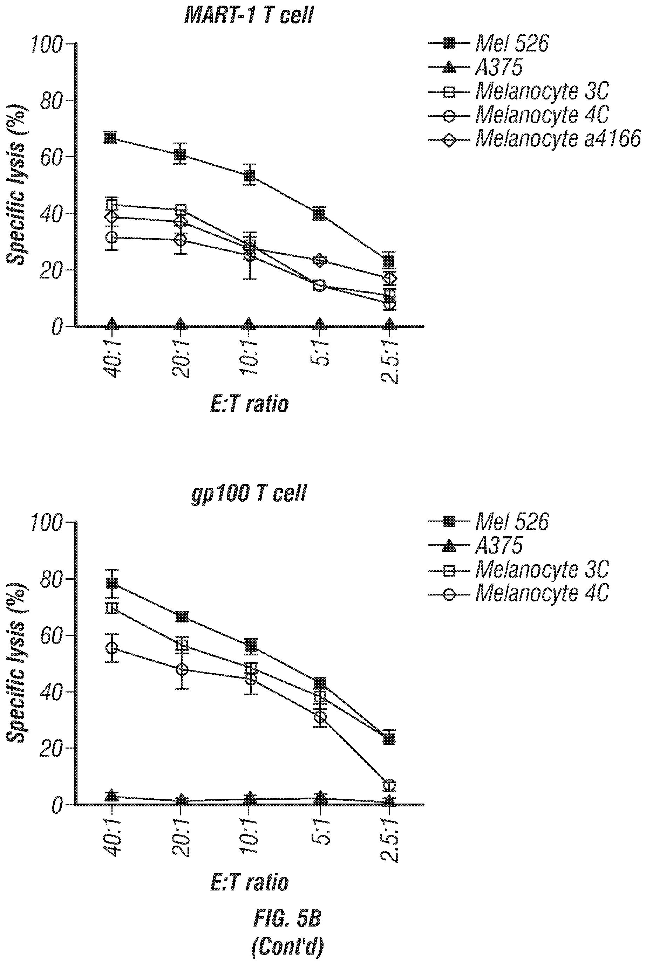

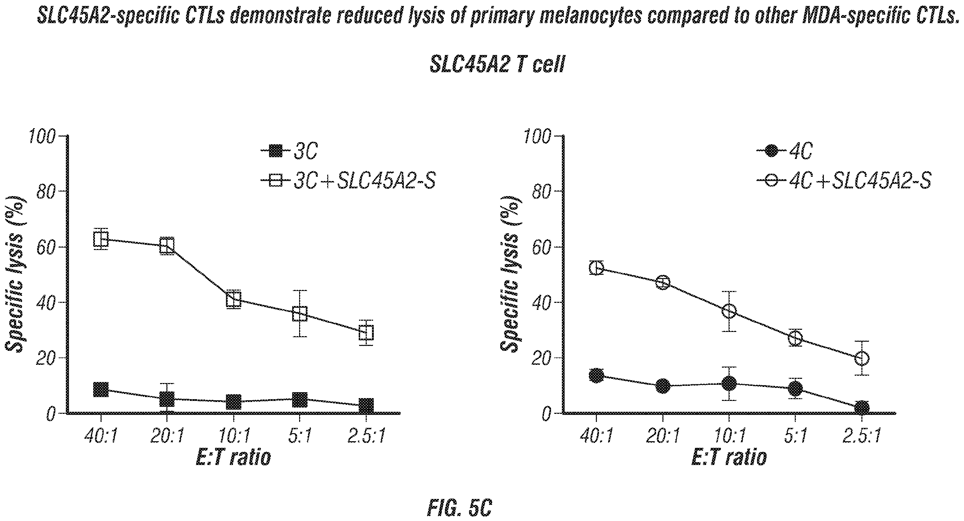

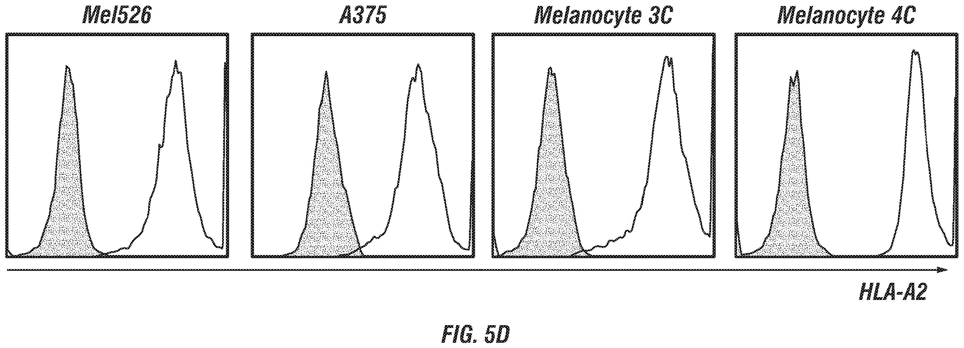

**FIGS. 5A****-D:** Expression of SLC45A2 and cytotoxic activity of SLC45A2-specific CD8 T cells against melanocytes. **FIG. 5A****,** Expression of melanoma differentiation antigen in melanocytes. mRNA expression of SLC45A2, MART-1, gp100, and tyrosinase were tested in two different melanocytes, 4C0197 (4C) and 3C0661 (3C), by RT-PCR. SLC45A2 mRNA was expressed on both melanocytes, but it was very low level compared with melanoma cells, whereas other melanoma differentiation antigens such as MART-1, gp100 and tyrosinase were expressed in melanocytes with similar level to melanoma. Results of 1 representative experiment of at least 3 performed are shown. **FIG. 5B****,** Cytotoxic activity of HLA-A*0201-restricted SLC45A2-specific CD8 T cells against melanocytes. SLC45A2-specific CD8 T cells did not kill two kinds of melanocytes (HLA-A*0201+) well but kill melanoma cells with high cytotoxic effect. On the contrary, MART-1 and gp100-specific CD8 T cells killed melanocyte as well as melanoma cells. Standard ⁵¹Cr release assay was performed using HLA-A*0201-restricted SLC45A2-, MART-1, and gp100 -specific CD8 T cells at various E:T ratio. Mel526 (HLA-A*0201+/SLC45A2+) and A375 (HLA-A*0201+/SLC45A2-) was used as positive and negative control respectively. **FIG. 5C****,** Cytotoxic activity of HLA-A*2402-restricted SLC45A2-specific CD8 T cells against melanocytes. HLA-A*2402-restricted SLC45A2-specific CD8 T cells did not kill melanocyte, 4C0197 expressing HLA-A*2402 well but did kill melanoma cells expressing HLA-A*2402+/SLC45A2+. Mel526 (HLA-A*2402-/SLC45A2+) is used as negative control. Primary melanocyte lines 3C and 4C were pulsed with 1ug/ml SLYSYFQKV (SEQ ID NO:1) peptide and used as targets for HLA-A*0201-restricted SLC45A2-specific CTLs in standard ⁵¹Cr release assay. **FIG. 5D****,** Comparison of surface HLA-A*0201 expression in Mel526, A375, and primary melanocytes 3C and 4C following staining with mAb BB7.2 and flow cytometric analysis.

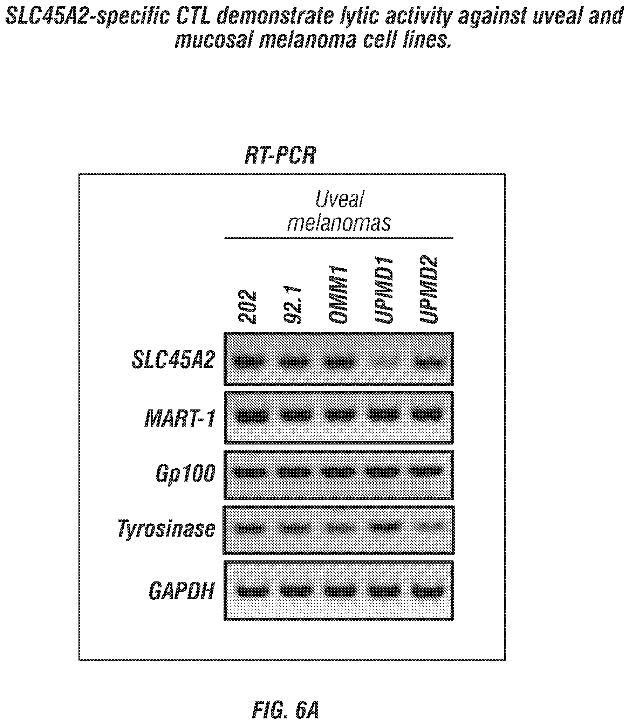

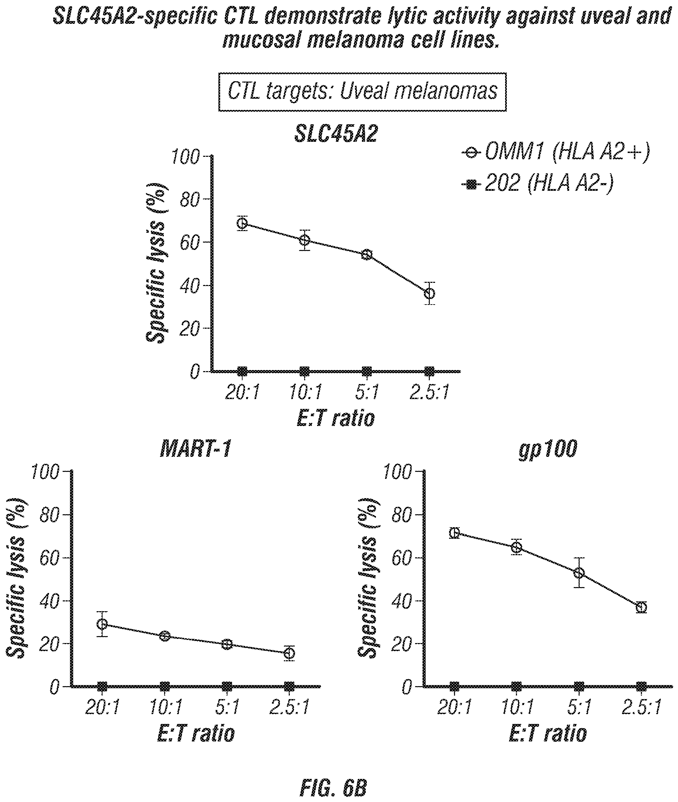

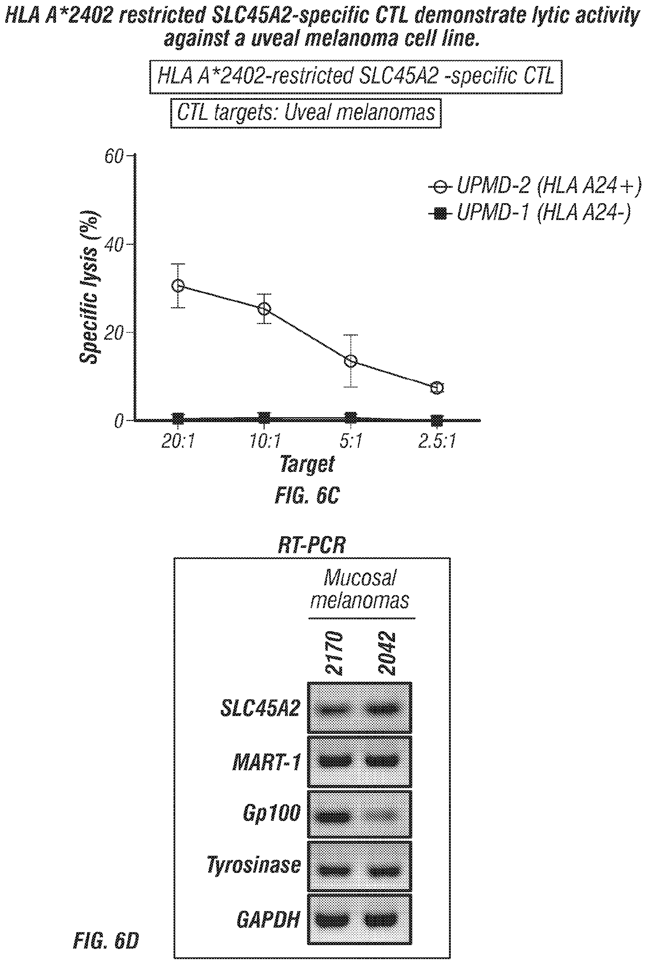

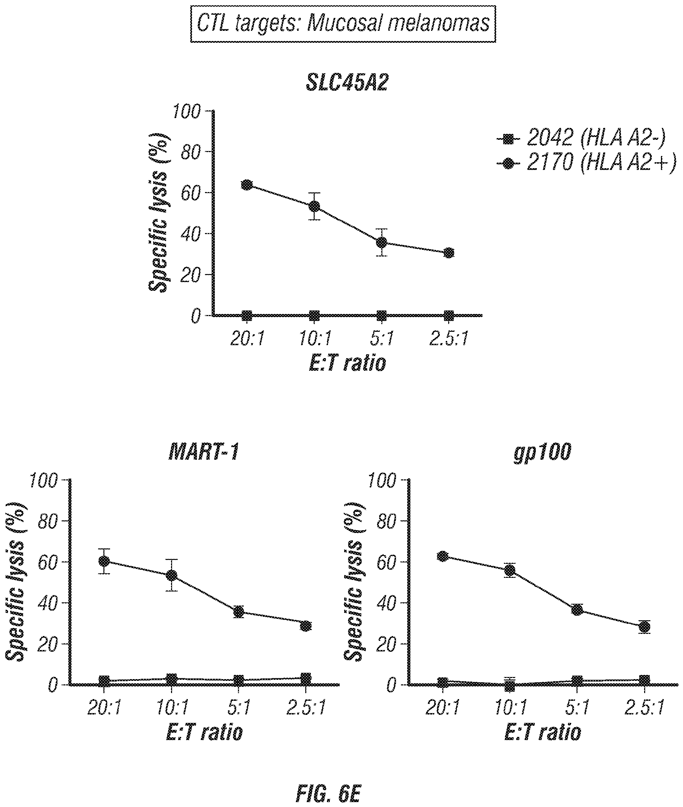

**FIGS. 6A****-E:** SLC45A2 expression and cytotoxic activity of SLC45A2-specific CD8 T cells against uveal and mucosal melanoma cells. **FIG. 6A****,** SLC45A2 expression on uveal melanoma cells. SLC45A2 expression was analyzed by RT-PCR and all uveal melanoma cells used in this study expressed SLC45A2. **FIG. 6B****,** Cytotoxic effect of SLC45A-specific CD8 T cells against uveal melanoma cells. HLA A*0201 -restricted SLC45A-specific CD8 T cells lysed OMM1, uveal melanoma cells expressing SLC45A2 and HLA A*0201 but not lysed 202, uveal melanoma cells expressing SLC45A2 but not HLA A*0201. HLA A*2402-restricted SLC45A-specific CD8 T cells showed killing effect against UPMD2 expressing SLC45A2 and HLA A*2402. When UPMD2 pulsed with S₉₃₃₋₄₀₂ peptide, higher cytotoxicity was shown by HLA A*2402-restricted SLC45A-specific CD8 T cells. UPMD1 expressing SLC45A2+ and HLA A*2402- was not killed by HLA A*2402-restricted SLC45A-specific CD8 T cells. Standard ⁵¹Cr release assay for cytotoxic activity are performed in different E:T ratio. Results of 1 representative experiment of at least 2 performed are shown. **FIG. 6C****,** HLA A*2402 restricted SLC45A2-specific CTL demonstrate lytic activity against a uveal melanoma cell line. **FIG. 6D****,** SLC45A2 expression on mucosal melanoma cells. Two kinds of mucosal melanoma cells expressed SLC45A2. SLC45A2 expression was analyzed by RT-PCR. **FIG.** 6E, Cytotoxic effect of SLC45A-specific CD8 T cells against mucosal melanoma cells. SLC45A-specific CD8 T cells killed 2170, mucosal melanoma cells expressing SLC45A2 and HLA A*0201 but did not kill 2042 expressing SLC45A2 but not HLA A*0201. Standard ⁵¹Cr release assay for cytotoxic activity are performed in different E:T ratio. Results of 1 representative experiment of at least 2 performed are shown.

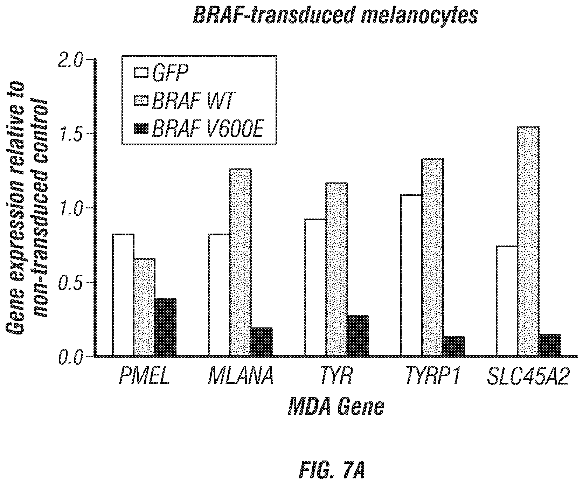

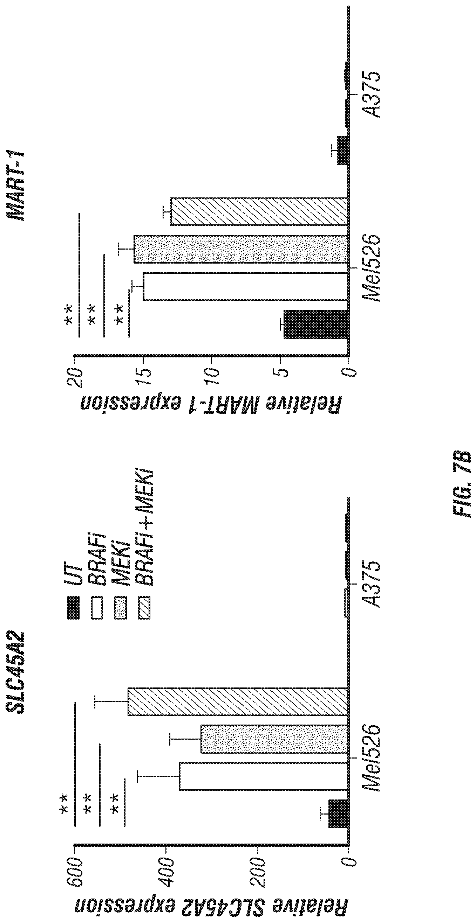

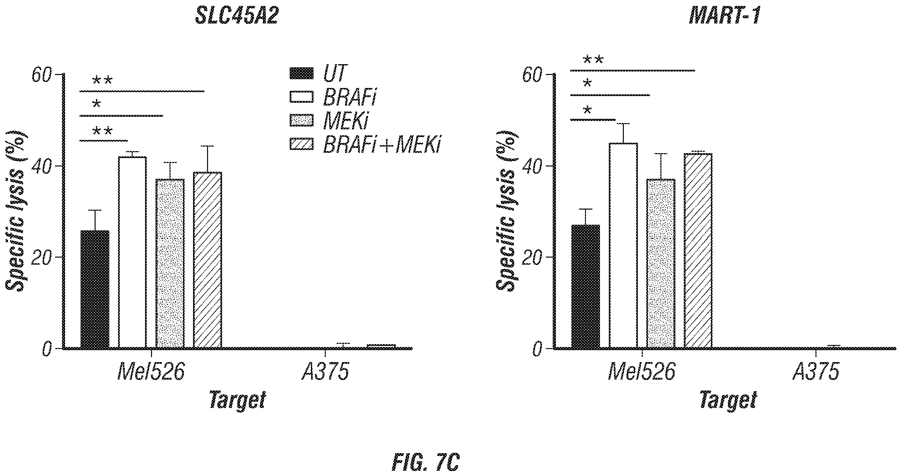

**FIGS. 7A****-C:** Control of SLC45A2 expression by the MAPK pathway and the enhanced melanoma cell CTL killing following MAPK inhibitor treatment. **FIG. 7A****,** Melanocyte differentiation antigen expression in primary melanocytes transduced to express GFP, wild-type BRAF or mutant BRAF(V600E), as assessed by gene expression microarray analysis. Relative expression of SLC45A2, MART-1, gp100, tyrosinase-related protein and tyrosinase compared to non-transduced cells is shown. **FIG. 7B****,** BRAF(V600E)-positive melanoma cell lines Mel526 or A375 were treated with the BRAF(V600E)-specific inhibitor dabrafenib (50nM), the MEK inhibitor Trametinib (50nM), or both inhibitors. Forty-eight hours later, mRNA expression of SLC45A2 and MART-1 was analyzed by quantitative RT-PCR. Untreated melanoma cells were used as controls. **FIG. 7C****,** SLC45A2-specific T-cell mediated cytotoxic killing of melanoma cell lines Mel526 or A375 following 48h treatment with BRAFi, MEKi or both inhibitors. Cytotoxic activity of SLC45A2-specific CTLs in a standard ⁵¹Cr release assay (E:T ratio = 20:1) against drug-treated targets is shown in comparison with untreated targets.

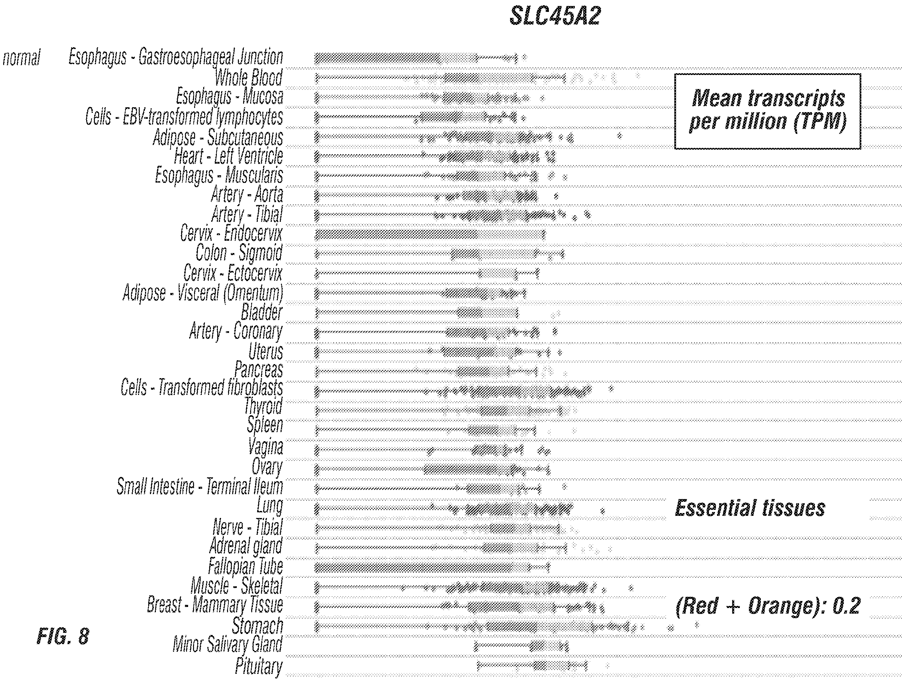

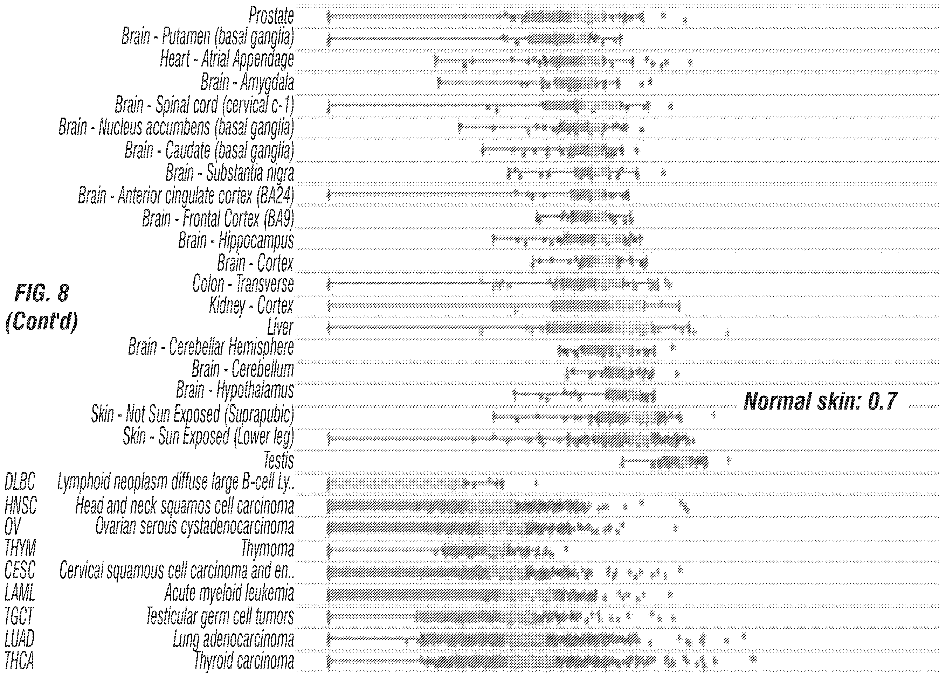

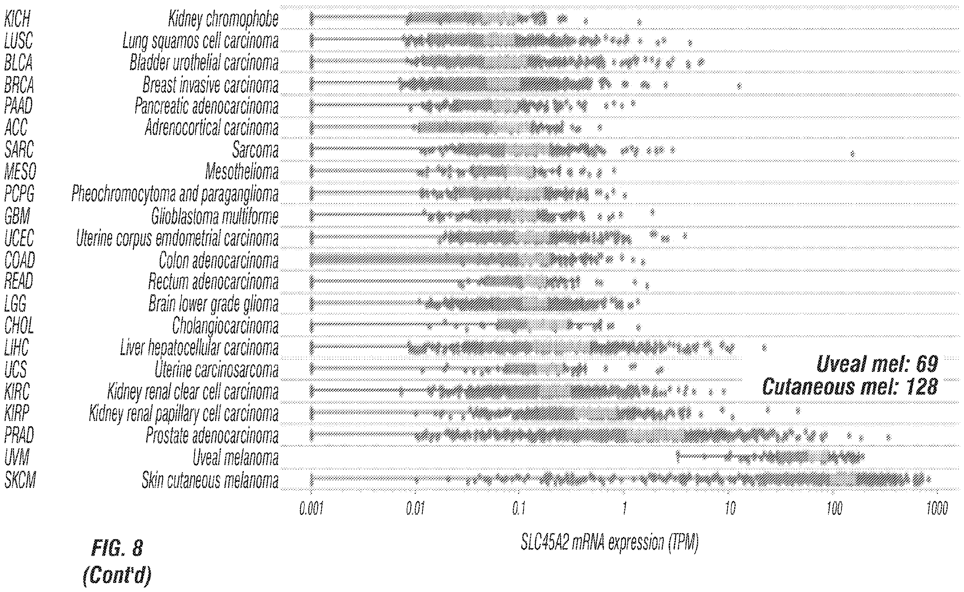

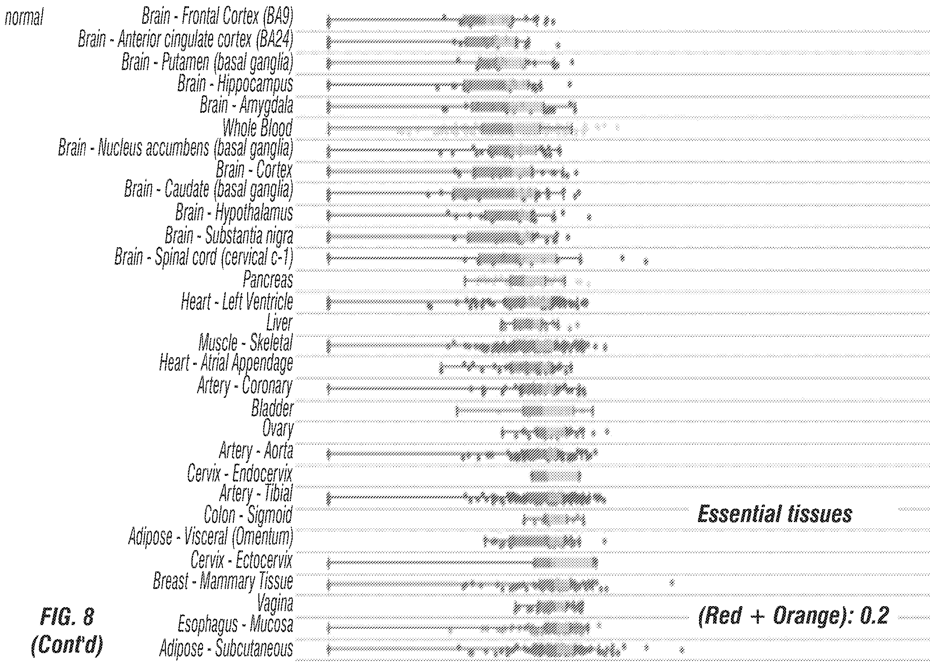

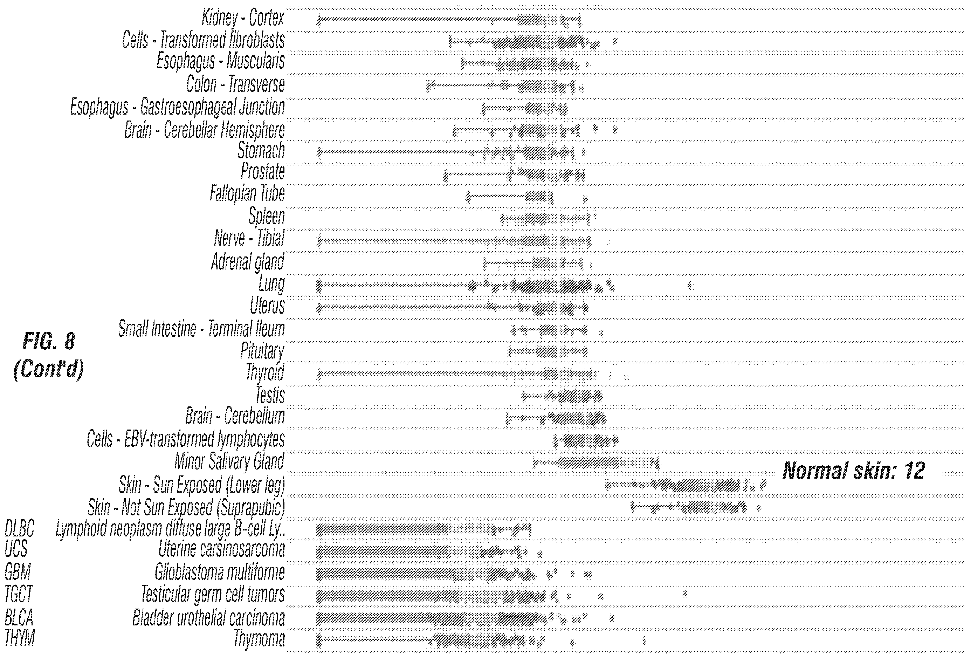

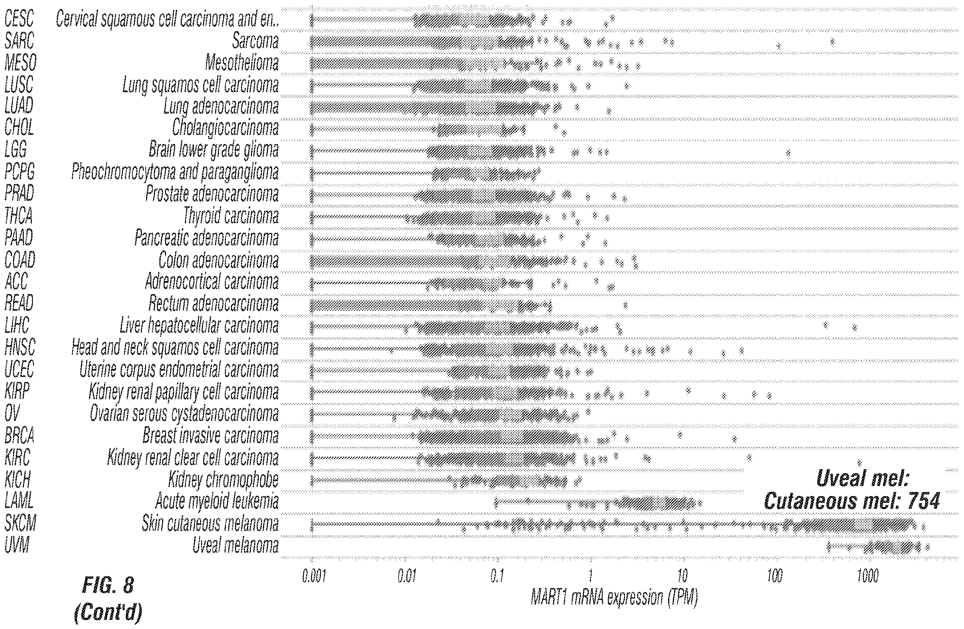

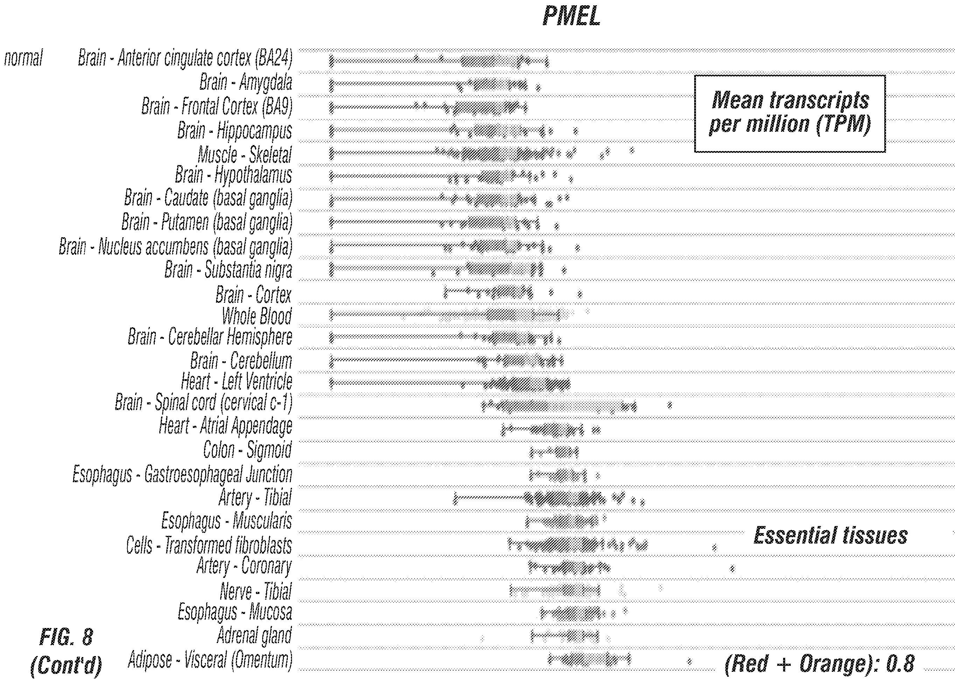

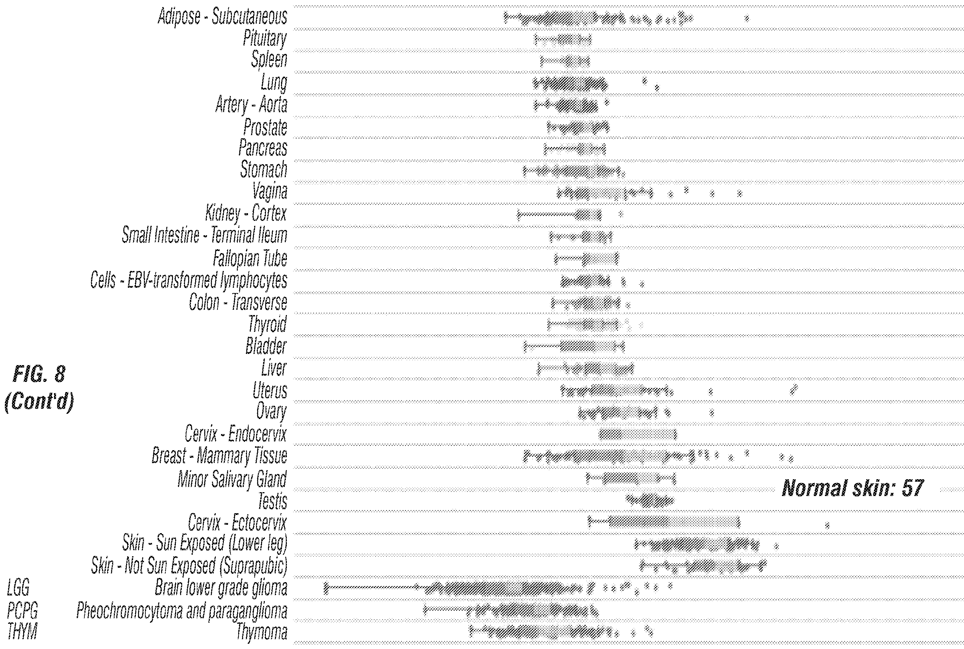

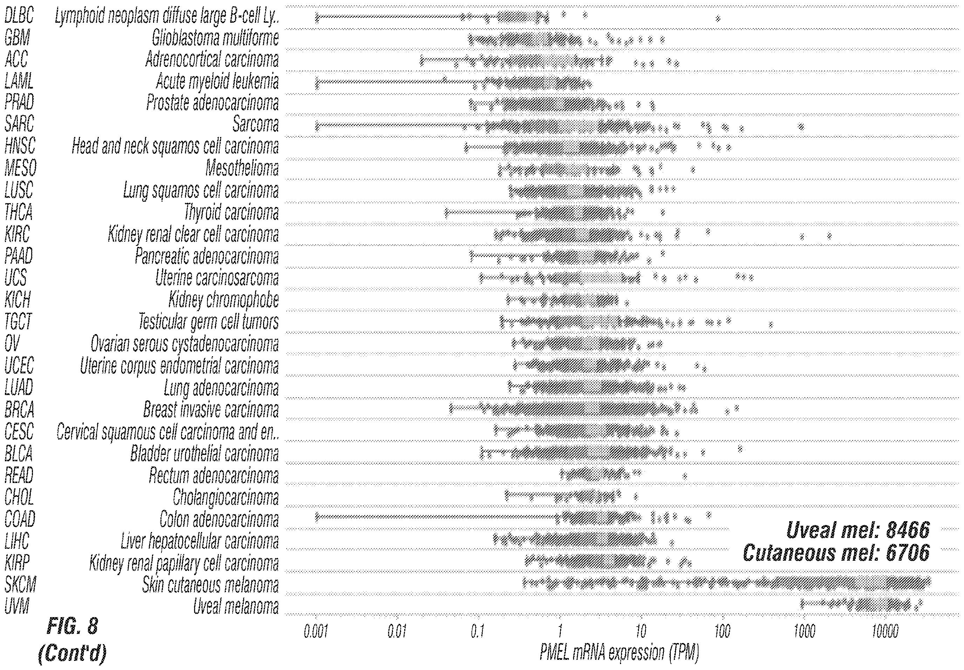

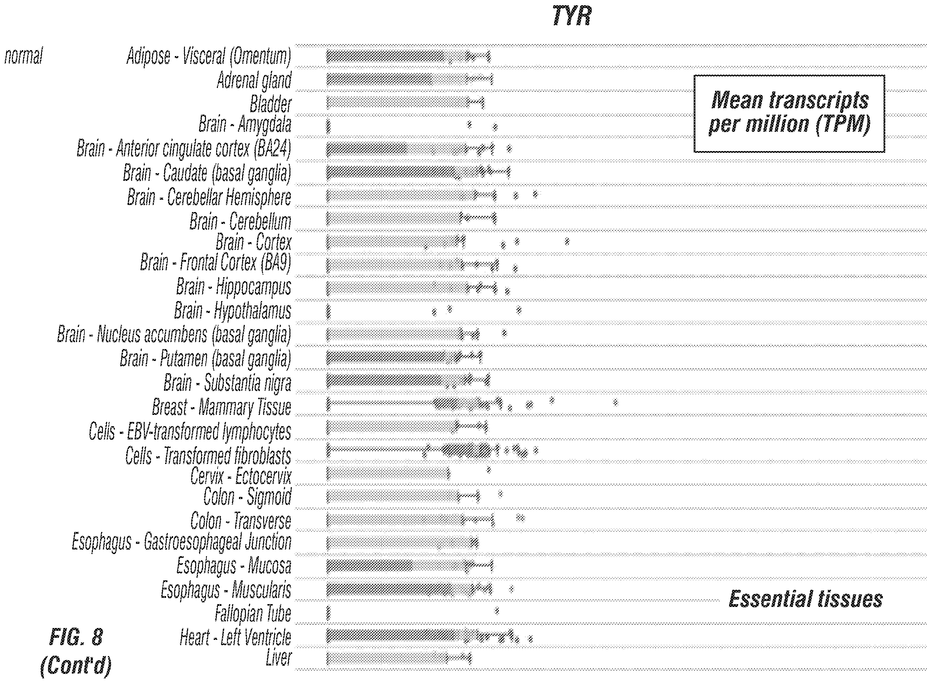

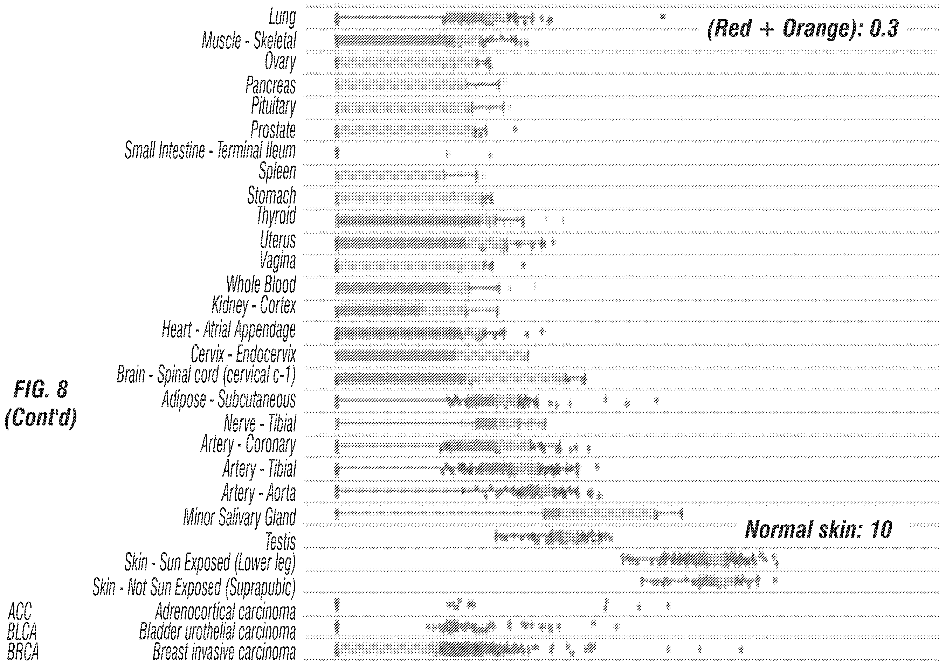

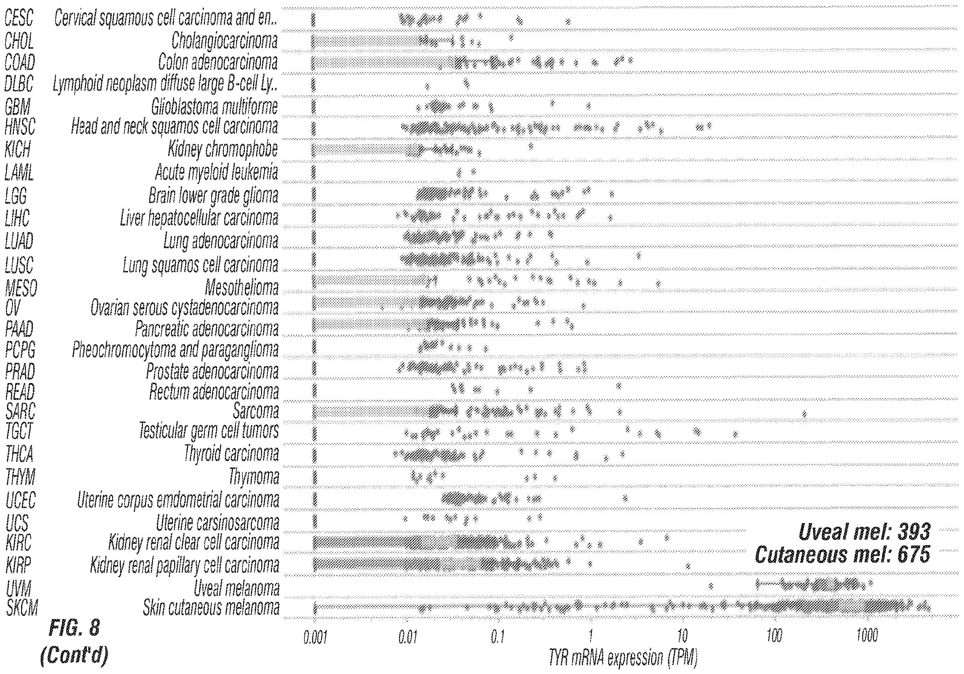

**FIG. 8****:** Expression of SLC45A2 in normal tissues and cancer tissues. Relative gene expression of melanocyte differentiation antigen in normal tissues and cancer tissues. Gene expression of melanocyte differentiation antigen in normal tissues and in cancer patients samples was analyzed using the Genotype-Tissue Expression (GTEx) portal data and the Cancer Genome Atlas (TCGA) portal data respectively. SLC45A2 was barely expressed in many normal tissue, whereas MART-1 and gp100 gene expression was observed in most normal tissue even it was low level. SLC45A2, MART-1, tyrosinase, and gp100 gene expression showed high expression in cutaneous melanoma and uveal melanoma tissue compared with in other cancer tissues.

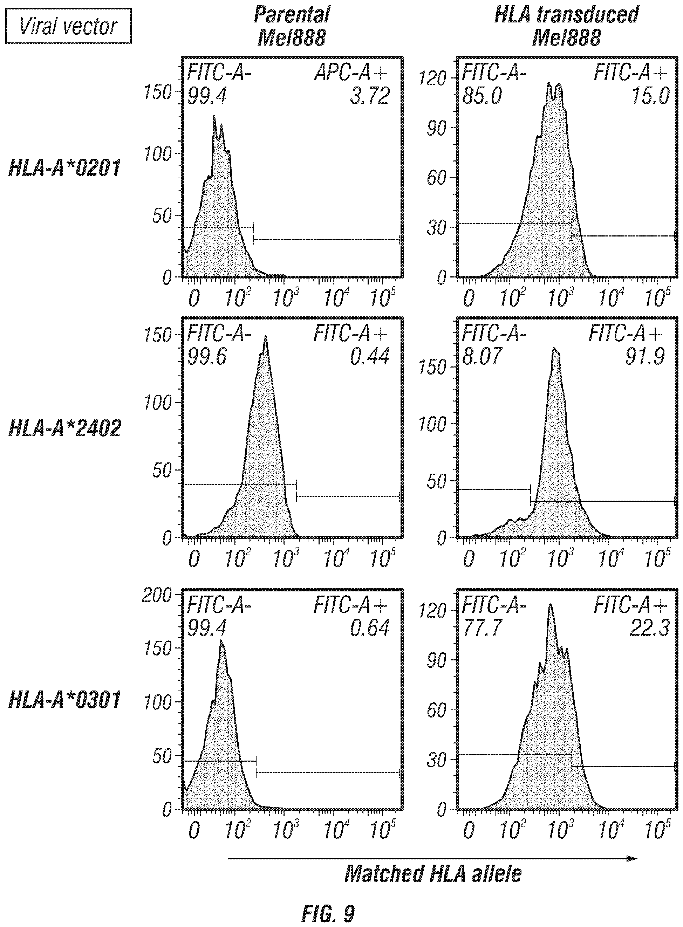

**FIG. 9****:** Ectopic expression of HLA in SLC45A2+ melanoma cell line Mel888.

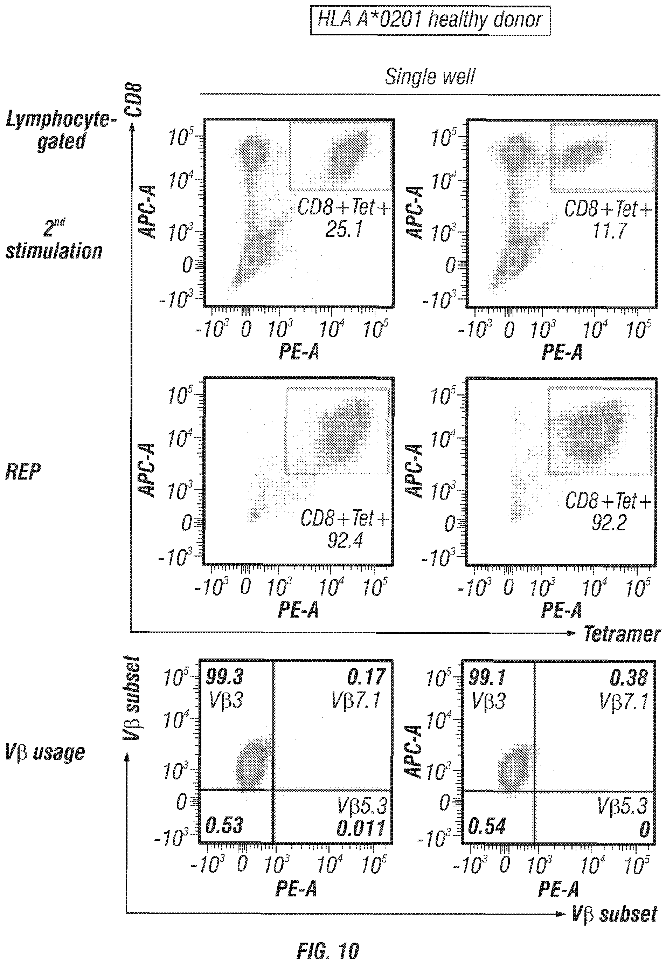

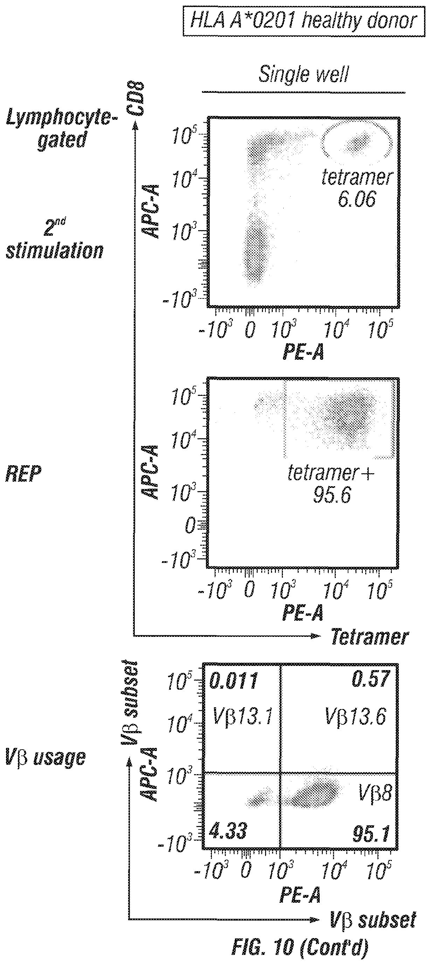

**FIG. 10****:** Generation of SLC45A2-specific CD8 T cells in PBMCs of HLA A*0201 -restricted healthy donors. Induction of SLC45A2-tetramer positive CD8 T cells. PBMC from two more HLA A*0201 restricted healthy donors was stimulated with autologous SLC45A2₃₈₂₋₃₉₀ peptide -pulsed DC. SLC45A2 tetramer-positive CD8 T cells were sorted after 2 times stimulation and the sorted SLC45A2-tetramer positive CD8 T cells were expanded according to REP. The expanded SLC45A2-tetramer positive CD8 T cells were used for other experiment. TCR repertoire analysis of SLC45A2-specific CD8 T cells was performed using the IOTest Beta Mark TCR-Vβ repertoire kit with Vβ antibodies corresponding to 24 different specificities.

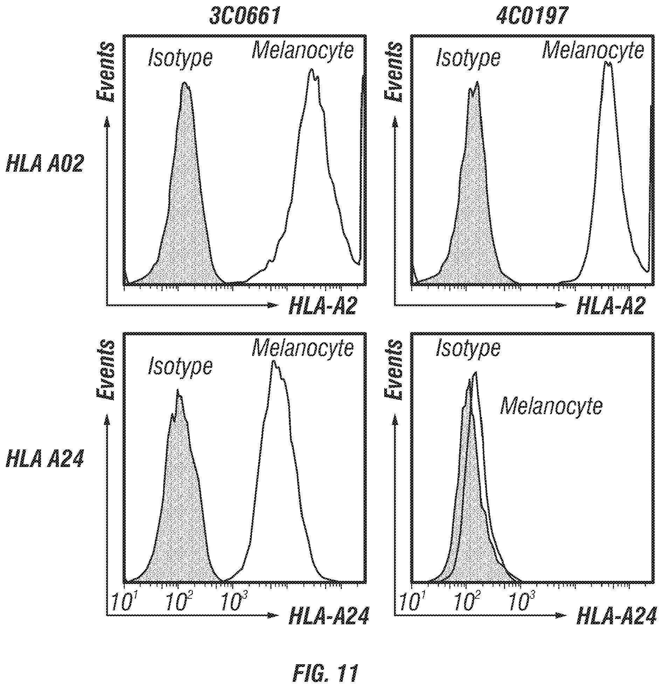

**FIG. 11****:** HLA expression in the melanocytes. HLA expression was assessed in melanocytes, 3C0661 and 4C0197 with different origin by staining with anti-HLA A2 antibody and anti HLA A24 antibody. 3C0661 expressed both HLA A2 and HLA A24. 4C0197 expressed HLA A2 but not HLA A24.

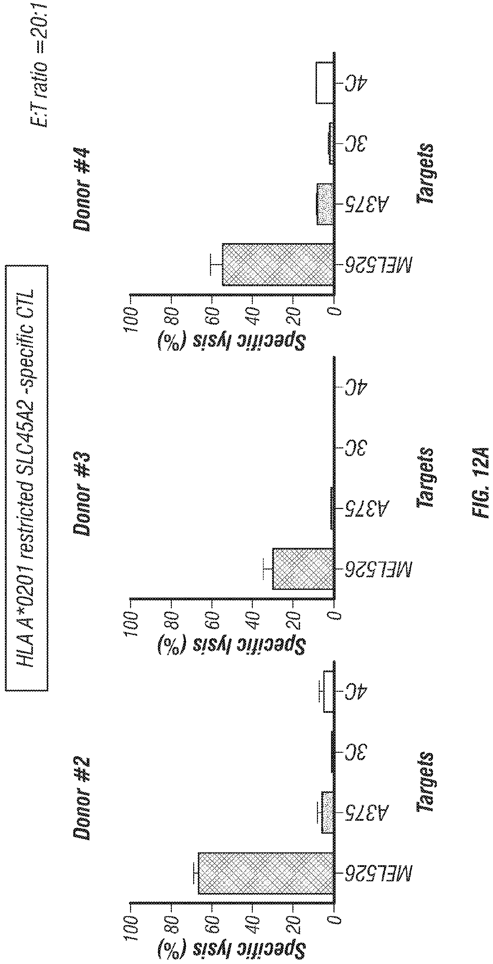

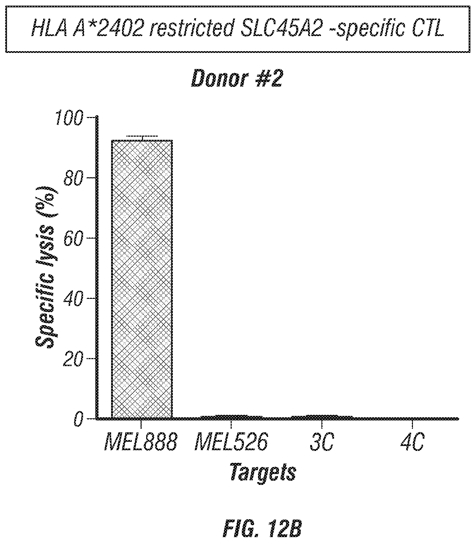

**FIGS. 12A****-B:** Cytotoxic activity of SLC45A2-specific CD8 T cells from other HLA-A*0201-restricted donors against melanocytes. **FIG. 12A****,** Cytotoxic activity of HLA-A*0201-restricted SLC45A2-specific CD8 T cells from other donors against melanocytes. SLC45A2-specific CD8 T cells didn't kill two kinds of melanocytes (HLA-A*0201+) well but kill melanoma cells with high cytotoxic effect. On the contrary, MART-1 and gp100-specific CD8 T cells killed melanocyte as well as melanoma cells. Standard ⁵¹Cr release assay was performed using HLA-A*0201 -restricted SLC45A2-specific CD8 T cells at E:T= 20:1 ratio. Mel526 (HLA-A*0201+/SLC45A2+) and A375 (HLA-A*0201+/SLC45A2-) was used as positive and negative control respectively. **FIG. 12B****,** Cytotoxic activity of HLA-A*2402-restricted SLC45A2-specific CD8 T cells from other donor against melanocytes.

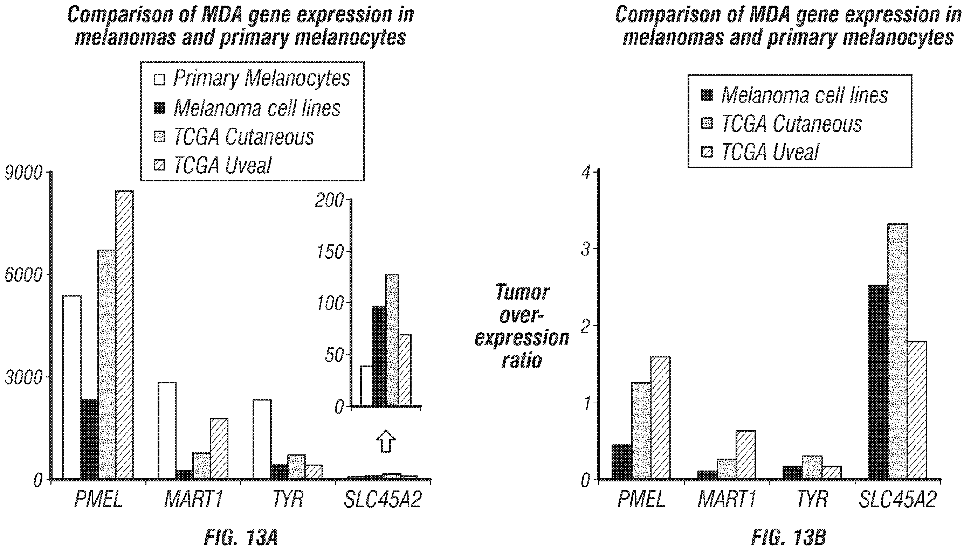

**FIGS. 13A****-B:** Comparison of MDA gene expression in melanomas and primary melanocytes. **FIG. 13A****,** RNAseq transcript expression of MART1, gp100, tyrosinase, and SLC45A2 in normal tissues (GTex Portal database), cutaneous and uveal melanoma tumors (TCGA database), melanoma cell lines (MD Anderson TIL lab database), or primary melanocytes. TPM, transcripts per million. **FIG. 13B****,** Tumor overexpression indices for MARTI, gp100, tyrosinase, and SLC45A2, as calculated by the formula{mean tumor transcript expression / mean primary melanocyte transcript expression}.

### DESCRIPTION

### I. IMMUNOTHERAPIES USING SLC45A2 PEPTIDES

A SLC45A2 peptide as described herein (e.g., comprising SEQ ID NO:1 or SEQ ID NO:2) may be used for immunotherapy of a cancer. For example, a SLC45A2 peptide may be contacted with or used to stimulate a population of T cells to induce proliferation of the T cells that recognize or bind the SLC45A2 peptide. A SLC45A2 peptide as described herein may be administered to a subject, such as a human patient, to enhance the immune response of the subject against a cancer. For tumors such as melanoma, the adoptive transfer of tumor-infiltrating lymphocytes (TILs) has been shown to result in significant patient benefit (Hawkins *et al*., 2010).

A SLC45A2 peptide may be included in an active immunotherapy (e.g., a cancer vaccine) or a passive immunotherapy (e.g., an adoptive immunotherapy). Active immunotherapies include immunizing a subject with a purified SLC45A2 peptide antigen or an immunodominant SLC45A2 peptide (native or modified); alternately, antigen presenting cells pulsed with a SLC45A2 peptide (or transfected with genes encoding the SLC45A2 antigen) may be administered to a subject. The SLC45A2 peptide may be modified or contain one or more mutations such as, *e.g.,* a substitution mutation. Passive immunotherapies include adoptive immunotherapies. Adoptive immunotherapies generally involve administering cells to a subject, wherein the cells *(e.g.,* cytotoxic T cells) have been sensitized *in vitro* to SLC45A2 (see, *e.g.,* US 7910109).

Flow cytometry may be used in the adoptive immunotherapy for rapid isolation of human tumor antigen-specific T-cell clones by using, *e.g.,* T-cell receptor (TCR) Vβ antibodies in combination with carboxyfluorescein succinimidyl ester (CFSE)-based proliferation assay. See, *e.g.,* Lee *et al.* (2008). Tetramer-guided cell sorting may be used such as, e.g., the methods described in Pollack *et al.* Various culture protocols are also known for adoptive immunotherapy and may be used according to the present disclosure; Cells may be cultured in conditions which do not require the use of antigen presenting cells *(e.g.,* Hida *et al*., 2002). Also, T cells may be expanded under culture conditions that utilize antigen presenting cells, such as dendritic cells (Nestle *et al*., 1998), and alsoartificial antigen presenting cells may be used for this purpose (Maus *et al*., 2002). Additional methods for adoptive immunotherapy are disclosed in Dudley *et al.* (2003) that may be used according to the present disclosure. Various methods are known and may be used for cloning and expanding human antigen-specific T cells (see, *e.g.,* Riddell *et al*., 1990).

The following protocol may be used to generate T cells that selectively recognize SLC45A2 peptides. Peptide-specific T-cell lines may be generated from HLA-A2⁺ normal donors and patients using methods previously reported (Hida *et al*., 2002). Briefly, PBMCs (1 × 10⁵ cells/well) can be stimulated with about 10 µg/ml of each peptide in quadruplicate in a 96-well, U-bottom-microculture plate (Corning Incorporated, Lowell, MA) in about 200 µl of culture medium. The culture medium may consist of 50% AIM-V medium (Invitrogen), 50% RPMI1640 medium (Invitrogen), 10% human AB serum (Valley Biomedical, Winchester, VA), and 100 IU/ml of interleukin-2 (IL-2). Cells may be restimulated with the corresponding peptide about every 3 days. After 5 stimulations, T cells from each well may be washed and incubated with T2 cells in the presence or absence of the corresponding peptide. After about 18 hours, the production of interferon (IFN)-γ may be determined in the supernatants by ELISA. T cells that secret large amounts of IFN-γ may be further expanded by a rapid expansion protocol (Riddell et al., 1990; Yee et al., 2002b).

An immunotherapy may utilize a SLC45A2 peptide as described herein that is associated with a cell penetrator, such as a liposome or a cell penetrating peptide (CPP). Antigen presenting cells (such as dendritic cells) pulsed with peptides may be used to enhance antitumour immunity (Celluzzi *et al*., 1996; Young *et al*., 1996). Liposomes and CPPs are described in further detail below. An immunotherapy may utilize a nucleic acid encoding a SLC45A2 peptideas described herein, wherein the nucleic acid is delivered, *e.g.,* in a viral vector or non-viral vector.

SLC45A2 is expressed in cancers such as melanomas. SLC45A2 (solute carrier family 45, member 2; also known as membrane-associated protein, MATP or AIM-1) is a melanocyte differentiation protein such as MART-1, gp100, tyrosinase and TRP-1 and transporter protein localized in melanosome membrane (Newton jm 2001). Although the exact function is unknown, it is likely linked to the production of melanin in either of two different roles. One is the proper processing and trafficking of tyrosinase to the melanosome (Costin 2003), and the other is the maintenance of a specific pH within the melanosomes (Graf et al., 2005; Lucotte et al., 2010). SLC45A2 has been implicated with dark skin, hair and eye pigmentation and in human, pathogenic mutation of SLC45A2 lead to type IV oculocutaneous albinism (OCA4) by disrupting melanin biosynthesis (Fukamachi 2001,Newton2001, du2002). Interestingly, SLC45A2 variants by mutation are associated with melanoma risk and its gene has been proposed as a melanoma susceptibility gene in light-skinned population (Guedj m2008, Fernandes lp 2008, ibarrola 2012).

A SLC45A2 peptide as disclosed herein may be used in an immunotherapy to treat a melanoma in a mammalian subject, such as a human patient. The melanoma may be, *e.g.,* a cutaneous melanoma, uveal melanoma, mucosal melanoma, or a metastatic melanoma. It is anticipated that any cancers that expresses SLC45A2 may be treated via an immunotherapy using a SLC45A2 peptide as described herein.

Circulating tumor antigen-specific T cells recognized melanocyte antigen (MART-1, gp100, tyrosinase) can be detected in melanoma patients, isolated from peripheral blood mononuclear cells (PBMCs) using tetramer-based technology, and expanded more than 20 fold using anti-CD3 and IL-2 with the irradiated feeder cells (Yee 2012). Tumor antigen-specific CD8 T cells can be induced in vitro by stimulating PBMCs using autologous dendritic cells (DCs) pulsed with peptide recognized CTL epitope. During priming, exposure of IL-21 leads to the increased frequencies and number of antigen-specific CTLs and drive to CTLs with memory-like phenotype, which experienced long-term in vivo persistence and mediated tumor regression (Li 2005; Chapuis 2013). Strategy using IL-21 in the *ex vivo* generation of potent tumor antigen-specific CTLs for adoptive transfer is very useful and was employed, as described in the below examples.

### II. SLC45A2 PEPTIDES

As used herein, the term "peptide" encompasses amino acid chains comprising 7-35 amino acids, preferably 8-35 amino acid residues, and even more preferably 8-25 amino acids, or 7, 8, 9, 10, 11, 12, 13, 14, 15, 16, 17, 18, 19, 20, 21, 22, 23, 24, 25, 26, 27, 28, 29, 30, 31, 32, 33, 34, or 35 amino acids in length, or any range derivable therein. For example, a SLC45A2 peptide as disclosed herein may comprise or consist of the SLC45A2 peptide of SEQ ID NO:1 or SEQ ID NO:2. Additional SLC45A2 peptides that may be used in accordance with the present disclosure are provided in Table 4. As used herein, an "antigenic peptide" is a peptide which, when introduced into a vertebrate, can stimulate the production of antibodies in the vertebrate, *i.e.,* is antigenic, and wherein the antibody can selectively recognize and/or bind the antigenic peptide. An antigenic peptide may comprise an immunoreactive SLC45A2 peptide, and may comprise additional sequences. The additional sequences may be derived from a native antigen and may be heterologous, and such sequences may, but need not, be immunogenic. A SLC45A2 peptide mayselectively bind with a HLA-A2 or HLA-A24. The SLC45A2 peptide may be 8, 9, 10, 11, 12, 13, 14, 15, 16, 17, 18, 19, 20, 21, 22, 23, 24, 25, 26, 27, 28, 29, 30, 31, 32, 33, 34, or 35 amino acids in length, or any range derivable therein. Preferably, the SLC45A2 peptide is from 8 to 35 amino acids in length. The SLC45A2 peptide may also be from 8 to 10 amino acids in length.

As would be appreciated by one of skill in the art, MHC molecules can bind peptides of varying sizes, but typically not full length proteins. While MHC class I molecules have been traditionally described to bind to peptides of 8-11 amino acids long, it has been shown that peptides 15 amino acids in length can bind to MHC class I molecules by bulging in the middle of the binding site or extending out of the MHC class I binding groove (Guo *et al*., 1992; Burrows *et al*., 2006; Samino *et al*., 2006; Stryhn *et al*., 2000; Collins *et al*., 1994; Blanchard and Shastri, 2008). Further, recent studies also demonstrated that longer peptides may be more efficiently endocytosed, processed, and presented by antigen-presenting cells (Zwaveling *et al*., 2002; Bijker *et al*., 2007; Melief and van der Burg, 2008; Quintarelli *et al*., 2011). As demonstrated in Zwaveling *et al.* (2002) peptides up to 35 amino acids in length may be used to selectively bind a class II MHC and are effective. As would be immediately appreciated by one of skill, a naturally occurring full-length SLC45A2 would not be useful to selectively bind a class II MHC such that it would be endocytosed and generate proliferation of T cells. Generally, the naturally occurring full-length SLC45A2 proteins do not display these properties and would thus not be useful for these immunotherapy purposes.

A SLC45A2 peptide is immunogenic or antigenic. As shown in the below examples, various disclosed SLC45A2 peptides can promote the proliferation of T cells. It is anticipated that such peptides may be used to induce some degree of protective immunity.

A SLC45A2 peptide may be a recombinant peptide, synthetic peptide, purified peptide, immobilized peptide, detectably labeled peptide, encapsulated peptide, or a vector-expressed peptide (*e.g.,* a peptide encoded by a nucleic acid in a vector comprising a heterologous promoter operably linked to the nucleic acid). A synthetic SLC45A2 peptide may be administered to a subject, such as a human patient, to induce an immune response in the subject. Synthetic peptides may display certain advantages, such as a decreased risk of bacterial contamination, as compared to recombinantly expressed peptides. A SLC45A2 peptide may also be comprised in a pharmaceutical composition such as, *e.g.,* a vaccine composition, which is formulated for administration to a mammalian or human subject.

### A. Cell Penetrating Peptides

A SLC45A2 peptide may also be associated with or covalently bound to a cell penetrating peptide (CPP). Cell penetrating peptides that may be covalently bound to a SLC45A2 peptide include, *e.g.,* HIV Tat, herpes virus VP22, the *Drosophila* Antennapedia homeobox gene product, signal sequences, fusion sequences, or protegrin I. Covalently binding a peptide to a CPP can prolong the presentation of a peptide by dendritic cells, thus enhancing antitumour immunity (Wang and Wang, 2002). A SLC45A2 peptide as described herein *(e.g.,* comprised within a peptide or polyepitope string) may be covalently bound (*e.g.,* via a peptide bond) to a CPP to generate a fusion protein. Furthermore, a SLC45A2 peptide or nucleic acid encoding a SLC45A2 peptide may be encapsulated within or associated with a liposome, such as a mulitlamellar, vesicular, or multivesicular liposome.

As used herein, "association" means a physical association, a chemical association or both. For example, an association can involve a covalent bond, a hydrophobic interaction, encapsulation, surface adsorption, or the like.

As used herein, "cell penetrator" refers to a composition or compound which enhances the intracellular delivery of the peptide/polyepitope string to the antigen presenting cell. For example, the cell penetrator may be a lipid which, when associated with the peptide, enhances its capacity to cross the plasma membrane. Alternatively, the cell penetrator may be a peptide. Cell penetrating peptides (CPPs) are known in the art, and include, *e.g.,* the Tat protein of HIV (Frankel and Pabo, 1988), the VP22 protein of HSV (Elliott and O'Hare, 1997) and fibroblast growth factor (Lin *et al*., 1995).

Cell-penetrating peptides (or "protein transduction domains") have been identified from the third helix of the *Drosophila* Antennapedia homeobox gene (Antp), the HIV Tat, and the herpes virus VP22, all of which contain positively charged domains enriched for arginine and lysine residues (Schwarze *et al*., 2000; Schwarze *et al*., 1999). Also, hydrophobic peptides derived from signal sequences have been identified as cell-penetrating peptides. (Rojas *et al*., 1996; Rojas *et al*., 1998; Du *et al*., 1998). Coupling these peptides to marker proteins such as β-galactosidase has been shown to confer efficient internalization of the marker protein into cells, and chimeric, in-frame fusion proteins containing these peptides have been used to deliver proteins to a wide spectrum of cell types both *in vitro* and *in vivo* (Drin *et al*., 2002). Fusion of these cell penetrating peptides to a SLC45A2 peptide as disclosed herein may enhance cellular uptake of the polypeptides.

Cellular uptake may be facilitated by the attachment of a lipid, such as stearate or myristilate, to the polypeptide. Lipidation has been shown to enhance the passage of peptides into cells. The attachment of a lipid moiety is another way that increases polypeptide uptake by the cell. Cellular uptake is further discussed below.

A SLC45A2 peptide as disclosed herein may be included in a liposomal vaccine composition. For example, the liposomal composition may be or comprise a proteoliposomal composition. Methods for producing proteoliposomal compositions that may be used in accordance with the present disclosure are described, *e.g.,* in Neelapu *et al.* (2007) and Popescu *et al.* (2007). Proteoliposomal compositions may be used to treat a melanoma.

By enhancing the uptake of a SLC45A2 polypeptide, it may be possible to reduce the amount of protein or peptide required for treatment. This in turn can significantly reduce the cost of treatment and increase the supply of therapeutic agent. Lower dosages can also minimize the potential immunogencity of peptides and limit toxic side effects.

A SLC45A2 peptide may be associated with a nanoparticle to form nanoparticle-polypeptide complex. The nanoparticle is a liposomes or other lipid-based nanoparticle such as a lipid-based vesicle (*e.g.,* a DOTAP:cholesterol vesicle). The nanoparticle is an iron-oxide based superparamagnetic nanoparticles. Superparamagnetic nanoparticles ranging in diameter from about 10 to 100 nm are small enough to avoid sequestering by the spleen, but large enough to avoid clearance by the liver. Particles this size can penetrate very small capillaries and can be effectively distributed in body tissues. Superparamagnetic nanoparticles-polypeptide complexes can be used as MRI contrast agents to identify and follow those cells that take up the SLC45A2 peptide. The nanoparticle may be a semiconductor nanocrystal or a semiconductor quantum dot, both of which can be used in optical imaging. The nanoparticle may also be be a nanoshell, which comprises a gold layer over a core of silica. One advantage of nanoshells is that polypeptides can be conjugated to the gold layer using standard chemistry. The nanoparticle may also be be a fullerene or a nanotube (Gupta *et al*., 2005).

Peptides are rapidly removed from the circulation by the kidney and are sensitive to degradation by proteases in serum. By associating a SLC45A2 peptide with a nanoparticle, the nanoparticle-polypeptide complexes as disclosed herein may protect against degradation and/or reduce clearance by the kidney. This may increase the serum half-life of polypeptides, thereby reducing the polypeptide dose need for effective therapy. Further, this may decrease the costs of treatment, and minimizes immunological problems and toxic reactions of therapy.

### B. Polyepitope Strings

A SLC45A2 peptide may be included or comprised in a polyepitope string. A polyepitope string is a peptide or polypeptide containing a plurality of antigenic epitopes from one or more antigens linked together. A polyepitope string may be used to induce an immune response in a subject, such as a human subject. Polyepitope strings have been previously used to target malaria and other pathogens (Baraldo *et al*., 2005; Moorthy *et al*., 2004; Baird *et al*., 2004). A polyepitope string may refer to a nucleic acid (*e.g.,* a nucleic acid encoding a plurality of antigens including a SLC45A2 peptide) or a peptide or polypeptide (*e.g*., containing a plurality of antigens including a SLC45A2 peptide). A polyepitope string may be included in a cancer vaccine composition.

### C. Biological Functional Equivalents

A SLC45A2 peptide in accordance with the present disclosure may be modified to contain amino acid substitutions, insertions and/or deletions that do not alter their respective interactions with HLA-A2 or HLA-A24 binding regions. Such a biologically functional equivalent of a SLC45A2 peptide could be a molecule having like or otherwise desirable characteristics, *e.g*., binding of HLA-A2 or HLA-A24. As a nonlimiting example, certain amino acids may be substituted for other amino acids in an SLC45A2 peptide disclosed herein without appreciable loss of interactive capacity, as demonstrated by detectably unchanged peptide binding to HLA-A2 or HLA-A24. The SLC45A2 may have a substitution mutation at an anchor reside, such as a substitution mutation at one, two, or all of positions: 1 (P1), 2 (P2), and/or 9 (P9). It is thus contemplated that an SLC45A2 peptide disclosed herein (or a nucleic acid encoding such a peptide) which is modified in sequence and/or structure, but which is unchanged in biological utility or activity remains within the scope of the disclosure.

It is also well understood by the skilled artisan that, inherent in the definition of a biologically functional equivalent peptide, is the concept that there is a limit to the number of changes that may be made within a defined portion of the molecule while still maintaining an acceptable level of equivalent biological activity. Biologically functional equivalent peptides are thus defined herein as those peptides in which certain, not most or all, of the amino acids may be substituted. Of course, a plurality of distinct peptides with different substitutions may easily be made and used in accordance with the disclosure.

The skilled artisan is also aware that where certain residues are shown to be particularly important to the biological or structural properties of a peptide, *e.g*., residues in specific epitopes, such residues may not generally be exchanged. This may be the case as a mutation in an SLC45A2 peptide disclosed herein could result in a loss of species-specificity and in turn, reduce the utility of the resulting peptide for use in the disclosed methods. Thus, peptides which are antigenic (*e.g*., bind HLA-A2 or HLA-A24 specifically) and comprise conservative amino acid substitutions are understood to be included in the present disclosure. Conservative substitutions are least likely to drastically alter the activity of a protein. A "conservative amino acid substitution" refers to replacement of amino acid with a chemically similar amino acid, *i.e.,* replacing nonpolar amino acids with other nonpolar amino acids; substitution of polar amino acids with other polar amino acids, acidic residues with other acidic amino acids, *etc.*

Amino acid substitutions, such as those which might be employed in modifying an SLC45A2 peptide disclosed herein are generally based on the relative similarity of the amino acid side-chain substituents, for example, their hydrophobicity, hydrophilicity, charge, size, and the like. An analysis of the size, shape and type of the amino acid side-chain substituents reveals that arginine, lysine and histidine are all positively charged residues; that alanine, glycine and serine are all a similar size; and that phenylalanine, tryptophan and tyrosine all have a generally similar shape. Therefore, based upon these considerations, arginine, lysine and histidine; alanine, glycine and serine; and phenylalanine, tryptophan and tyrosine; are defined herein as biologically functional equivalents. The mutation may enhance TCR-pMHC interaction and/or peptide-MHC binding.

Also envisaged are isoforms of the SLC45A2 peptides disclosed herein. An isoform contains the same number and kinds of amino acids as a peptide disclosed herein, but the isoform has a different molecular structure. The isoforms envisaged herein are those having the same properties as a peptide as described herein.

Nonstandard amino acids may be incorporated into proteins by chemical modification of existing amino acids or by de novo synthesis of a peptide disclosed herein. A nonstandard amino acid refers to an amino acid that differs in chemical structure from the twenty standard amino acids encoded by the genetic code.

Also envisaged is a chemical derivative of an SLC45A2 peptide disclosed herein. "Chemical derivative" refers to a peptide having one or more residues chemically derivatized by reaction of a functional side group, and retaining biological activity and utility. Such derivatized peptides include, for example, those in which free amino groups have been derivatized to form specific salts or derivatized by alkylation and/or acylation, p-toluene sulfonyl groups, carbobenzoxy groups, t-butylocycarbonyl groups, chloroacetyl groups, formyl or acetyl groups among others. Free carboxyl groups may be derivatized to form organic or inorganic salts, methyl and ethyl esters or other types of esters or hydrazides and preferably amides (primary or secondary). Chemical derivatives may include those peptides which comprise one or more naturally occurring amino acids derivatives of the twenty standard amino acids. For example, 4-hydroxyproline may be substituted for serine; and ornithine may be substituted for lysine.

It should be noted that all amino-acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino-acid residues. The amino acids described herein are preferred to be in the "L" isomeric form. However, residues in the "D" isomeric form can be substituted for any L-amino acid residue, as long as the desired functional properties set forth herein are retained by the protein.

Preferred SLC45A2 peptides or analogs thereof preferably specifically or preferentially bind a HLA-A2 or HLA-A24. Determining whether or to what degree a particular SLC45A2 peptide or labeled peptide, or an analog thereof, can bind an HLA-A2 or HLA-A24 and can be assessed using an *in vitro* assay such as, for example, an enzyme-linked immunosorbent assay (ELISA), immunoblotting, immunoprecipitation, radioimmunoassay (RIA), immunostaining, latex agglutination, indirect hemagglutination assay (IHA), complement fixation, indirect immnunofluorescent assay (FA), nephelometry, flow cytometry assay, chemiluminescence assay, lateral flow immunoassay, u-capture assay, mass spectrometry assay, particle-based assay, inhibition assay and/or an avidity assay.

### D. Nucleic Acids Encoding a SLC45A2 Peptide

Also provided is a nucleic acid encoding an isolated SLC45A2 peptide comprising a sequence that has at least about 90%, 91%, 92%, 93%, 94%, 95%, 96%, 97%, 98%, 99%, or 100% sequence identity to any of SEQ ID NOs. 1-2, or the peptide may have 1, 2, 3, or 4 point mutations (e.g., substitution mutations) as compared to SEQ ID NO:1 or SEQ ID NO:2. As stated above, such a SLC45A2 peptide may be, *e.g.,* from 8 to 35 amino acids in length, or any range derivable therein. The SLC45A2 peptide may correspond to a portion of the SLC45A2 protein (NM_016180 or NM_00101250; either of these splice variants may be used). The term "nucleic acid" is intended to include DNA and RNA and can be either double stranded or single stranded.

Also provided are recombinantly-produced SLC45A2 peptides which can specifically bind a HLA-A2 or HLA-A24. Accordingly, a nucleic acid encoding a SLC45A2 peptide may be operably linked to an expression vector and the peptide produced in the appropriate expression system using methods well known in the molecular biological arts. A nucleic acid encoding a SLC45A2 peptide disclosed herein may be incorporated into any expression vector which ensures good expression of the peptide. Possible expression vectors include but are not limited to cosmids, plasmids, or modified viruses (e.g. replication defective retroviruses, adenoviruses and adeno-associated viruses), so long as the vector is suitable for transformation of a host cell.

A recombinant expression vector being "suitable for transformation of a host cell", means that the expression vector contains a nucleic acid molecule as described herein and regulatory sequences selected on the basis of the host cells to be used for expression, which is operatively linked to the nucleic acid molecule. The terms, "operatively linked" or "operably linked" are used interchangeably, and are intended to mean that the nucleic acid is linked to regulatory sequences in a manner which allows expression of the nucleic acid.

Accordingly, a recombinant expression vector comprising nucleic acid encoding an SLC45A2 peptide, and the necessary regulatory sequences for the transcription and translation of the inserted protein-sequence is provided herein. Suitable regulatory sequences may be derived from a variety of sources, including bacterial, fungal, or viral genes (*e.g.,* see the regulatory sequences described in Goeddel (1990).

Selection of appropriate regulatory sequences is generally dependent on the host cell chosen, and may be readily accomplished by one of ordinary skill in the art Examples of such regulatory sequences include: a transcriptional promoter and enhancer or RNA polymerase binding sequence, a ribosomal binding sequence, including a translation initiation signal. Additionally, depending on the host cell chosen and the vector employed, other sequences, such as an origin of replication, additional DNA restriction sites, enhancers, and sequences conferring inducibility of transcription may be incorporated into the expression vector. It will also be appreciated that the necessary regulatory sequences may be supplied by the native protein and/or its flanking regions.

A recombinant expression vector may also contain a selectable marker gene which facilitates the selection of host cells transformed or transfected with a recombinant SLC45A2 peptide disclosed herein. Examples of selectable marker genes are genes encoding a protein such as G418 and hygromycin which confer resistance to certain drugs, β-galactosidase, chloramphenicol acetyltransferase, or firefly luciferase. Transcription of the selectable marker gene is monitored by changes in the concentration of the selectable marker protein such as β-galactosidase, chloramphenicol acetyltransferase, or firefly luciferase. If the selectable marker gene encodes a protein conferring antibiotic resistance such as neomycin resistance transformant cells can be selected with G418. Cells that have incorporated the selectable marker gene will survive, while the other cells die. This makes it possible to visualize and assay for expression of a recombinant expression vector, and in particular, to determine the effect of a mutation on expression and phenotype. It will be appreciated that selectable markers can be introduced on a separate vector from the nucleic acid of interest.

Recombinant expression vectors can be introduced into host cells to produce a transformant host cell. The term "transformant host cell" is intended to include prokaryotic and eukaryotic cells which have been transformed or transfected with a herein described recombinant expression vector. The terms "transformed with", "transfected with", "transformation" and "transfection" are intended to encompass introduction of nucleic acid (*e.g.* a vector) into a cell by one of many possible techniques known in the art. Suitable host cells include a wide variety of prokaryotic and eukaryotic host cells. For example, the proteins as described herein may be expressed in bacterial cells such as *E. coli,* insect cells (using baculovirus), yeast cells or mammalian cells.

A nucleic acid molecule as described herein may also be chemically synthesized using standard techniques. Various methods of chemically synthesizing polydeoxy-nucleotides are known, including solid-phase synthesis which, like peptide synthesis, has been fully automated in commercially available DNA synthesizers (See *e.g.,* Itakura et al. U.S. Pat. No. 4,598,049; Caruthers et al. U.S. Pat. No. 4,458,066; and Itakura U.S. Pat. Nos. 4,401,796 and 4,373,071).

### III. ANTIBODIES

One or more antibodies may be produced to a SLC45A2 peptide as described herein, a SLC45A2 peptide-HLA-A2 complex, or a SLC45A2 peptide-HLA-A24 complex. These antibodies may be used, *e.g.,* to treat a cancer or may be included in a cancer vaccine. An antibody that selectively recognizes a SLC45A2 peptide, a SLC45A2 peptide-HLA-A2 complex, or a SLC45A2 peptide-HLA-A24 complex may be administered to a subject, such as a human patient, to treat a melanoma.

There are methods of inducing dendritic cell- (DC) mediated cell killing against a target cell expressing a targeted cell surface polypeptide comprising: a) contacting the target cell with a polypeptide comprising an antibody that selectively binds a SLC45A2 peptide-HLA-A2 complex or a SLC45A2 peptide-HLA-A24 complex; and b) exposing the target cell to dendritic cells under conditions that promote killing of the target cell.

As used herein, the term "antibody" is intended to refer broadly to any immunologic binding agent such as IgG, IgM, IgA, IgD and IgE. Generally, IgG and/or IgM are preferred because they are typically the most common antibodies in the physiological situation and because they are easily made in a laboratory setting.

The term "antibody" is used to refer to any antibody-like molecule that has an antigen binding region, and includes antibody fragments such as Fab', Fab, F(ab')₂, single domain antibodies (DABs), Fv, scFv (single chain Fv), and the like. The techniques for preparing and using various antibody-based constructs and fragments are well known in the art. Means for preparing and characterizing antibodies are also well known in the art (See, *e.g.,* Antibodies: A Laboratory Manual, Cold Spring Harbor Laboratory, 1988).

"Mini-antibodies" or "minibodies" are also contemplated for use in accordance with the present disclosure. Minibodies are sFv polypeptide chains which include oligomerization domains at their C-termini, separated from the sFv by a hinge region. Pack *et al.* (1992). The oligomerization domain comprises self-associating .alpha.-helices, *e.g.,* leucine zippers, that can be further stabilized by additional disulfide bonds. The oligomerization domain is designed to be compatible with vectorial folding across a membrane, a process thought to facilitate *in vivo* folding of the polypeptide into a functional binding protein. Generally, minibodies are produced using recombinant methods well known in the art. See, *e.g.,* Pack *et al.* (1992); Cumber *et al.* (1992).

Antibody-like binding peptidomimetics are also contemplated herein. Liu *et al.* (2003) describe "antibody like binding peptidomimetics" (ABiPs), which are peptides that act as pared-down antibodies and have certain advantages of longer serum half-life as well as less cumbersome synthesis methods.

Monoclonal antibodies (MAbs) are recognized to have certain advantages, *e.g*., reproducibility and large-scale production, and their use is generally preferred. Accordingly, monoclonal antibodies of the human, murine, monkey, rat, hamster, rabbit and even chicken origin are provided herein. Due to the ease of preparation and ready availability of reagents, murine monoclonal antibodies will often be preferred.

"Humanized" antibodies are also contemplated, as are chimeric antibodies from mouse, rat, or other species, bearing human constant and/or variable region domains, bispecific antibodies, recombinant and engineered antibodies and fragments thereof. As used herein, the term "humanized" immunoglobulin refers to an immunoglobulin comprising a human framework region and one or more CDR's from a non-human (usually a mouse or rat) immunoglobulin. The non-human immunoglobulin providing the CDR's is called the "donor" and the human immunoglobulin providing the framework is called the "acceptor". A "humanized antibody" is an antibody comprising a humanized light chain and a humanized heavy chain immunoglobulin.

### A. Methods for Generating Monoclonal Antibodies

The methods for generating monoclonal antibodies (MAbs) generally begin along the same lines as those for preparing polyclonal antibodies. Briefly, a polyclonal antibody is prepared by immunizing an animal with a LEE or CEE composition in accordance with the present disclosure and collecting antisera from that immunized animal.

A wide range of animal species can be used for the production of antisera. Typically the animal used for production of antisera is a rabbit, a mouse, a rat, a hamster, a guinea pig or a goat. The choice of animal may be decided upon the ease of manipulation, costs or the desired amount of sera, as would be known to one of skill in the art. Antibodies described herein can also be produced transgenically through the generation of a mammal or plant that is transgenic for the immunoglobulin heavy and light chain sequences of interest and production of the antibody in a recoverable form therefrom. In connection with the transgenic production in mammals, antibodies can be produced in, and recovered from, the milk of goats, cows, or other mammals. See, *e.g.,* U.S. Pat. Nos. 5,827,690, 5,756,687, 5,750,172, and 5,741,957.

As is also well known in the art, the immunogenicity of a particular immunogen composition can be enhanced by the use of non-specific stimulators of the immune response, known as adjuvants. Suitable adjuvants include all acceptable immunostimulatory compounds, such as cytokines, chemokines, cofactors, toxins, plasmodia, synthetic compositions or LEEs or CEEs encoding such adjuvants.

Adjuvants that may be used include IL-1, IL-2, IL-4, IL-7, IL-12, γ-interferon, GMCSP, BCG, aluminum hydroxide, MDP compounds, such as thur-MDP and nor-MDP, CGP (MTP-PE), lipid A, and monophosphoryl lipid A (MPL). RIBI, which contains three components extracted from bacteria, MPL, trehalose dimycolate (TDM) and cell wall skeleton (CWS) in a 2% squalene/Tween 80 emulsion is also contemplated. MHC antigens may even be used. Exemplary, often preferred adjuvants include complete Freund's adjuvant (a non-specific stimulator of the immune response containing killed *Mycobacterium tuberculosis),* incomplete Freund's adjuvants and aluminum hydroxide adjuvant.

In addition to adjuvants, it may be desirable to coadminister biologic response modifiers (BRM), which have been shown to upregulate T cell immunity or downregulate suppressor cell activity. Such BRMs include, but are not limited to, Cimetidine (CIM; 1200 mg/d) (Smith/Kline, PA); low-dose Cyclophosphamide (CYP; 300 mg/m²) (Johnson/ Mead, NJ), cytokines such as γ-interferon, IL-2, or IL-12 or genes encoding proteins involved in immune helper functions, such as B-7.

The amount of immunogen composition used in the production of polyclonal antibodies varies upon the nature of the immunogen as well as the animal used for immunization. A variety of routes can be used to administer the immunogen including but not limited to subcutaneous, intramuscular, intradermal, intraepidermal, intravenous and intraperitoneal. The production of polyclonal antibodies may be monitored by sampling blood of the immunized animal at various points following immunization.

A second, booster dose (*e.g*., provided in an injection), may also be given. The process of boosting and titering is repeated until a suitable titer is achieved. When a desired level of immunogenicity is obtained, the immunized animal can be bled and the serum isolated and stored, and/or the animal can be used to generate MAbs.

For production of rabbit polyclonal antibodies, the animal can be bled through an ear vein or alternatively by cardiac puncture. The removed blood is allowed to coagulate and then centrifuged to separate serum components from whole cells and blood clots. The serum may be used as is for various applications or else the desired antibody fraction may be purified by well-known methods, such as affinity chromatography using another antibody, a peptide bound to a solid matrix, or by using, *e.g*., protein A or protein G chromatography.

MAbs may be readily prepared through use of well-known techniques, such as those exemplified in U.S. Patent 4,196,265. Typically, this technique involves immunizing a suitable animal with a selected immunogen composition, *e.g*., a purified or partially purified protein, polypeptide, peptide or domain, be it a wild-type or mutant composition. The immunizing composition is administered in a manner effective to stimulate antibody producing cells.

The methods for generating monoclonal antibodies (MAbs) generally begin along the same lines as those for preparing polyclonal antibodies. Rodents such as mice and rats are preferred animals, however, the use of rabbit, sheep or frog cells is also possible. The use of rats may provide certain advantages (Goding, 1986, pp. 60-61), but mice are preferred, with the BALB/c mouse being most preferred as this is most routinely used and generally gives a higher percentage of stable fusions.

The animals are injected with antigen, generally as described above. The antigen may be mixed with adjuvant, such as Freund's complete or incomplete adjuvant. Booster administrations with the same antigen or DNA encoding the antigen would occur at approximately two-week intervals.

Following immunization, somatic cells with the potential for producing antibodies, specifically B lymphocytes (B cells), are selected for use in the MAb generating protocol. These cells may be obtained from biopsied spleens, tonsils or lymph nodes, or from a peripheral blood sample. Spleen cells and peripheral blood cells are preferred, the former because they are a rich source of antibody-producing cells that are in the dividing plasmablast stage, and the latter because peripheral blood is easily accessible.

Often, a panel of animals will have been immunized and the spleen of an animal with the highest antibody titer will be removed and the spleen lymphocytes obtained by homogenizing the spleen with a syringe. Typically, a spleen from an immunized mouse contains approximately 5 × 10⁷ to 2 × 10⁸ lymphocytes.

The antibody-producing B lymphocytes from the immunized animal are then fused with cells of an immortal myeloma cell, generally one of the same species as the animal that was immunized. Myeloma cell lines suited for use in hybridoma-producing fusion procedures preferably are non-antibody-producing, have high fusion efficiency, and enzyme deficiencies that render then incapable of growing in certain selective media which support the growth of only the desired fused cells (hybridomas).

Any one of a number of myeloma cells may be used, as are known to those of skill in the art (Goding, pp. 65-66, 1986; Campbell, pp. 75-83, 1984). cites). For example, where the immunized animal is a mouse, one may use P3-X63/Ag8, X63-Ag8.653, NS1/1.Ag 4 1, Sp210-Ag14, FO, NSO/U, MPC-11, MPC11-X45-GTG 1.7 and S194/5XX0 Bul; for rats, one may use R210.RCY3, Y3-Ag 1.2.3, IR983F and 4B210; and U-266, GM1500-GRG2, LICR-LON-HMy2 and UC729-6 are all useful in connection with human cell fusions. See Yoo *et al.* (2002), for a discussion of myeloma expression systems.

One preferred murine myeloma cell is the NS-1 myeloma cell line (also termed P3-NS-1-Ag4-1), which is readily available from the NIGMS Human Genetic Mutant Cell Repository by requesting cell line repository number GM3573. Another mouse myeloma cell line that may be used is the 8-azaguanine-resistant mouse murine myeloma SP2/0 non-producer cell line.

Methods for generating hybrids of antibody-producing spleen or lymph node cells and myeloma cells usually comprise mixing somatic cells with myeloma cells in a 2:1 proportion, though the proportion may vary from about 20:1 to about 1:1, respectively, in the presence of an agent or agents (chemical or electrical) that promote the fusion of cell membranes. Fusion methods using Sendai virus have been described by Kohler and Milstein (1975; 1976), and those using polyethylene glycol (PEG), such as 37% (v/v) PEG, by Gefter *et al*., (1977). The use of electrically induced fusion methods is also appropriate (Goding pp. 71-74, 1986).

Fusion procedures usually produce viable hybrids at low frequencies, about 1 × 10⁻⁶ to 1 × 10⁻⁸. However, this does not pose a problem, as the viable, fused hybrids are differentiated from the parental, unfused cells (particularly the unfused myeloma cells that would normally continue to divide indefinitely) by culturing in a selective medium. The selective medium is generally one that contains an agent that blocks the *de novo* synthesis of nucleotides in the tissue culture media. Exemplary and preferred agents are aminopterin, methotrexate, and azaserine. Aminopterin and methotrexate block *de novo* synthesis of both purines and pyrimidines, whereas azaserine blocks only purine synthesis. Where aminopterin or methotrexate is used, the media is supplemented with hypoxanthine and thymidine as a source of nucleotides (HAT medium). Where azaserine is used, the media is supplemented with hypoxanthine.

Also, the HAT selection medium may be used. Generally, only the cells that capable of operating nucleotide salvage pathways are able to survive in HAT medium. The myeloma cells are defective in key enzymes of the salvage pathway, *e.g*., hypoxanthine phosphoribosyl transferase (HPRT), and they cannot survive. The B cells can operate this pathway, but they have a limited life span in culture and generally die within about two weeks. Therefore, the only cells that can survive in the selective media are those hybrids formed from myeloma and B cells.

This culturing can provide a population of hybridomas from which specific hybridomas may be selected. Typically, selection of hybridomas is performed by culturing the cells by single-clone dilution in microtiter plates, followed by testing the individual clonal supernatants (after about two to three weeks) for the desired reactivity. The assay should be sensitive, simple and rapid, such as radioimmunoassays, enzyme immunoassays, cytotoxicity assays, plaque assays, dot immunobinding assays, and the like.

The selected hybridomas may then be serially diluted and cloned into individual antibody-producing cell lines, which clones can then be propagated indefinitely to provide MAbs. The cell lines may be exploited for MAb production in two basic ways. First, a sample of the hybridoma can be injected (often into the peritoneal cavity) into a histocompatible animal of the type that was used to provide the somatic and myeloma cells for the original fusion (*e.g.,* a syngeneic mouse). Optionally, the animals are primed with a hydrocarbon, especially oils such as pristane (tetramethylpentadecane) prior to injection. The injected animal develops tumors secreting the specific monoclonal antibody produced by the fused cell hybrid. The body fluids of the animal, such as serum or ascites fluid, can then be tapped to provide MAbs in high concentration. Second, the individual cell lines could be cultured *in vitro,* where the MAbs are naturally secreted into the culture medium from which they can be readily obtained in high concentrations.

Further, expression of antibodies as described herein (or other moieties therefrom) from production cell lines can be enhanced using a number of known techniques. For example, the glutamine sythetase and DHFR gene expression systems are common approaches for enhancing expression under certain conditions. High expressing cell clones can be identified using conventional techniques, such as limited dilution cloning and Microdrop technology. The GS system is discussed in whole or part in connection with European Patent Nos. 0 216 846, 0 256 055, and 0 323 997 and European Patent Application No. 89303964.4.

MAbs produced by either means may be further purified, if desired, using filtration, centrifugation and various chromatographic methods such as HPLC or affinity chromatography. Fragments of the monoclonal antibodies as described herein can be obtained from the monoclonal antibodies so produced by methods which include digestion with enzymes, such as pepsin or papain, and/or by cleavage of disulfide bonds by chemical reduction. Alternatively, monoclonal antibody fragments encompassed by the present disclosure can be synthesized using an automated peptide synthesizer.

It is also contemplated that a molecular cloning approach may be used to generate monoclonal antibodies. In one embodiment, combinatorial immunoglobulin phagemid libraries are prepared from RNA isolated from the spleen of the immunized animal, and phagemids expressing appropriate antibodies are selected by panning using cells expressing the antigen and control cells. The advantages of this approach over conventional hybridoma techniques are that approximately 10⁴ times as many antibodies can be produced and screened in a single round, and that new specificities are generated by H and L chain combination which further increases the chance of finding appropriate antibodies. In another example, LEEs or CEEs can be used to produce antigens in vitro with a cell free system. These can be used as targets for scanning single chain antibody libraries. This would enable many different antibodies to be identified very quickly without the use of animals.

Another method for producing antibodies that may be used is found in U.S. Patent No. 6,091,001, which describes methods to produce a cell expressing an antibody from a genomic sequence of the cell comprising a modified immunoglobulin locus using Cre-mediated site-specific recombination. The method involves first transfecting an antibody-producing cell with a homology-targeting vector comprising a lox site and a targeting sequence homologous to a first DNA sequence adjacent to the region of the immunoglobulin loci of the genomic sequence which is to be converted to a modified region, so the first lox site is inserted into the genomic sequence via site-specific homologous recombination. Then the cell is transfected with a lox-targeting vector comprising a second lox site suitable for Cre-mediated recombination with the integrated lox site and a modifying sequence to convert the region of the immunoglobulin loci to the modified region. This conversion is performed by interacting the lox sites with Cre in vivo, so that the modifying sequence inserts into the genomic sequence via Cre-mediated site-specific recombination of the lox sites.

Alternatively, monoclonal antibody fragments encompassed by the present disclosure can be synthesized using an automated peptide synthesizer, or by expression of full-length gene or of gene fragments in *E. coli.*

### B. Antibody Conjugates

In addition, antibodies against a SLC45A2 peptide as described herein or a SLC45A2 peptide-HLA-A2 complex, generally of the monoclonal type, that are linked to at least one agent to form an antibody conjugate are provided herein. In order to increase the efficacy of antibody molecules as diagnostic or therapeutic agents, it is conventional to link or covalently bind or complex at least one desired molecule or moiety. Such a molecule or moiety may be, but is not limited to, at least one effector or reporter molecule. Effector molecules comprise molecules having a desired activity, *e.g*., cytotoxic activity. Non-limiting examples of effector molecules which have been attached to antibodies include toxins, anti-tumor agents, therapeutic enzymes, radio-labeled nucleotides, antiviral agents, chelating agents, cytokines, growth factors, and oligo- or poly-nucleotides. By contrast, a reporter molecule is defined as any moiety which may be detected using an assay. Non-limiting examples of reporter molecules which have been conjugated to antibodies include enzymes, radiolabels, haptens, fluorescent labels, phosphorescent molecules, chemiluminescent molecules, chromophores, luminescent molecules, photoaffinity molecules, colored particles or ligands, such as biotin.

Any antibody of sufficient selectivity, specificity or affinity may be employed as the basis for an antibody conjugate. Such properties may be evaluated using conventional immunological screening methodology known to those of skill in the art. Sites for binding to biological active molecules in the antibody molecule, in addition to the canonical antigen binding sites, include sites that reside in the variable domain that can bind pathogens, B-cell superantigens, the T cell co-receptor CD4 and the HIV-1 envelope (Sasso *et al*., 1989; Shorki *et al*., 1991; Silvermann *et al*., 1995; Cleary *et al*., 1994; Lenert *et al*., 1990; Berberian *et al*., 1993; Kreier *et al*., 1991). In addition, the variable domain is involved in antibody self-binding (Kang *et al*., 1988), and contains epitopes (idiotopes) recognized by anti-antibodies (Kohler *et al*., 1989).

Certain examples of antibody conjugates are those conjugates in which the antibody is linked to a detectable label. "Detectable labels" are compounds and/or elements that can be detected due to their specific functional properties, and/or chemical characteristics, the use of which allows the antibody to which they are attached to be detected, and/or further quantified if desired. Another such example is the formation of a conjugate comprising an antibody linked to a cytotoxic or anti-cellular agent, and may be termed "immunotoxins".

Antibody conjugates are generally preferred for use as diagnostic agents. Antibody diagnostics generally fall within two classes, those for use in *in vitro* diagnostics, such as in a variety of immunoassays, and/or those for use *in vivo* diagnostic protocols, generally known as "antibody-directed imaging".

Many appropriate imaging agents are known in the art, as are methods for their attachment to antibodies (see, for *e.g.,* U.S. Patent Nos. 5,021,236; 4,938,948; and 4,472,509). The imaging moieties used can be paramagnetic ions; radioactive isotopes; fluorochromes; NMR-detectable substances; X-ray imaging.

In the case of paramagnetic ions, one might mention by way of example ions such as chromium (III), manganese (II), iron (III), iron (II), cobalt (II), nickel (II), copper (II), neodymium (III), samarium (III), ytterbium (III), gadolinium (III), vanadium (II), terbium (III), dysprosium (III), holmium (III) and/or erbium (III), with gadolinium being particularly preferred. Ions useful in other contexts, such as X-ray imaging, include but are not limited to lanthanum (III), gold (III), lead (II), and especially bismuth (III).

In the case of radioactive isotopes for therapeutic and/or diagnostic application, one might mention astatine²¹¹, ¹⁴carbon, ⁵¹chromium, ³⁶chlorine, ⁵⁷cobalt, ⁵⁸cobalt, copper⁶⁷, ¹⁵²Eu, gallium⁶⁷, ³hydrogen, iodine¹²³, iodine¹²⁵, iodine¹³¹, indium¹¹¹, ⁵⁹iron, ³²phosphorus, rhenium¹⁸⁶, rhenium¹⁸⁸, ⁷⁵selenium, ³⁵sulphur, technicium^{99m} and/or yttrium⁹⁰. ¹²⁵I is often being preferred for use in certain applications, and technicium^{99m} and/or indium¹¹¹ are also often preferred due to their low energy and suitability for long range detection. Radioactively labeled monoclonal antibodies as described herein may be produced according to well-known methods in the art. For instance, monoclonal antibodies can be iodinated by contact with sodium and/or potassium iodide and a chemical oxidizing agent such as sodium hypochlorite, or an enzymatic oxidizing agent, such as lactoperoxidase. Monoclonal antibodies as described herein may be labeled with technetium^{99m} by ligand exchange process, for example, by reducing pertechnate with stannous solution, chelating the reduced technetium onto a Sephadex column and applying the antibody to this column. Alternatively, direct labeling techniques may be used, *e.g.,* by incubating pertechnate, a reducing agent such as SNCl₂, a buffer solution such as sodium-potassium phthalate solution, and the antibody. Intermediary functional groups which are often used to bind radioisotopes which exist as metallic ions to antibody are diethylenetriaminepentaacetic acid (DTPA) or ethylene diaminetetracetic acid (EDTA).

Among the fluorescent labels contemplated for use as conjugates include Alexa 350, Alexa 430, AMCA, BODIPY 630/650, BODIPY 650/665, BODIPY-FL, BODIPY-R6G, BODIPY-TMR, BODIPY-TRX, Cascade Blue, Cy3, Cy5,6-FAM, Fluorescein Isothiocyanate, HEX, 6-JOE, Oregon Green 488, Oregon Green 500, Oregon Green 514, Pacific Blue, REG, Rhodamine Green, Rhodamine Red, Renographin, ROX, TAMRA, TET, Tetramethylrhodamine, and/or Texas Red.

Another type of antibody conjugates contemplated herein are those intended primarily for use *in vitro,* where the antibody is linked to a secondary binding ligand and/or to an enzyme (an enzyme tag) that will generate a colored product upon contact with a chromogenic substrate. Examples of suitable enzymes include urease, alkaline phosphatase, (horseradish) hydrogen peroxidase or glucose oxidase. Preferred secondary binding ligands are biotin and/or avidin and streptavidin compounds. The use of such labels is well known to those of skill in the art and are described, for example, in U.S. Patents 3,817,837; 3,850,752; 3,939,350; 3,996,345; 4,277,437; 4,275,149 and 4,366,241.

Yet another known method of site-specific attachment of molecules to antibodies comprises the reaction of antibodies with hapten-based affinity labels. Essentially, hapten-based affinity labels react with amino acids in the antigen binding site, thereby destroying this site and blocking specific antigen reaction. However, this may not be advantageous since it results in loss of antigen binding by the antibody conjugate.

Molecules containing azido groups may also be used to form covalent bonds to proteins through reactive nitrene intermediates that are generated by low intensity ultraviolet light (Potter & Haley, 1983). In particular, 2- and 8-azido analogues of purine nucleotides have been used as site-directed photoprobes to identify nucleotide binding proteins in crude cell extracts (Owens & Haley, 1987; Atherton *et al*., 1985). The 2- and 8-azido nucleotides have also been used to map nucleotide binding domains of purified proteins (Khatoon *et al*., 1989; King *et al*., 1989; and Dholakia *et al*., 1989) and may be used as antibody binding agents.

Several methods are known in the art for the attachment or conjugation of an antibody to its conjugate moiety. Some attachment methods involve the use of a metal chelate complex employing, for example, an organic chelating agent such a diethylenetriaminepentaacetic acid anhydride (DTPA); ethylenetriaminetetraacetic acid; N-chloro-p-toluenesulfonamide; and/or tetrachloro-3α-6α-diphenylglycouril-3 attached to the antibody (U.S. Patent Nos. 4,472,509 and 4,938,948). Monoclonal antibodies may also be reacted with an enzyme in the presence of a coupling agent such as glutaraldehyde or periodate. Conjugates with fluorescein markers are prepared in the presence of these coupling agents or by reaction with an isothiocyanate. In U.S. Patent No. 4,938,948, imaging of breast tumors is achieved using monoclonal antibodies and the detectable imaging moieties are bound to the antibody using linkers such as methyl-p-hydroxybenzimidate or N-succinimidyl-3-(4-hydroxyphenyl)propionate.

Also derivatization of immunoglobulins by selectively introducing sulfhydryl groups in the Fc region of an immunoglobulin, using reaction conditions that do not alter the antibody combining site are contemplated. Antibody conjugates produced according to this methodology are disclosed to exhibit improved longevity, specificity and sensitivity (U.S. Pat. No. 5,196,066). Site-specific attachment of effector or reporter molecules, wherein the reporter or effector molecule is conjugated to a carbohydrate residue in the Fc region have also been disclosed in the literature (O'Shannessy *et al.,* 1987). This approach has been reported to produce diagnostically and therapeutically promising antibodies which are currently in clinical evaluation.

The anti-(SLC45A2 peptide) antibodies or the anti-(SLC45A2 peptide-HLA-A2) antibodies may be linked to semiconductor nanocrystals such as those described in U.S. Pat. Nos. 6,048,616; 5,990,479; 5,690,807; 5,505,928; 5,262,357; as well as PCT Publication No. 99/26299 (published May 27, 1999). In particular, exemplary materials for use as semiconductor nanocrystals in the biological and chemical assays disclosed herein include, but are not limited to those described above, including group II-VI, III-V and group IV semiconductors such as ZnS, ZnSe, ZnTe, CdS, CdSe, CdTe, MgS, MgSe, MgTe, CaS, CaSe, CaTe, SrS, SrSe, SrTe, BaS, BaSe, BaTe, GaN, GaP, GaAs, GaSb, InP, InAs, InSb, AlS, AlP, AlSb, PbS, PbSe, Ge and Si and ternary and quaternary mixtures thereof. Methods for linking semiconductor nanocrystals to antibodies are described in U.S. Patent Nos. 6,630,307 and 6,274,323.

Also disclosed are immunodetection methods for binding, purifying, removing, quantifying and/or otherwise generally detecting biological components such as T cells that selectively bind or recognize a SLC45A2 peptide or a SLC45A2 peptide-HLA-A2 complex. A tetramer assay may be used in accordance with the present disclosure. Tetramer assays generally involve generating soluble peptide-MHC tetramers that may bind antigen specific T lymphocytes, and methods for tetramer assays are described, *e.g.,* in Altman *et al.* (1996). Some immunodetection methods that may be used include, *e.g*., enzyme linked immunosorbent assay (ELISA), radioimmunoassay (RIA), immunoradiometric assay, fluoroimmunoassay, chemiluminescent assay, bioluminescent assay, tetramer assay, and Western blot. The steps of various useful immunodetection methods have been described in the scientific literature, such as, *e.g.,* Doolittle and Ben-Zeev, 1999; Gulbis and Galand, 1993; De Jager *et al*., 1993; and Nakamura *et al*., 1987.

### IV. PHARMACEUTICAL PREPARATIONS

It is contemplated that a SLC45A2 peptide as described herein may be comprised in a vaccine composition and administered to a subject to induce a therapeutic immune response in the subject towards a cancer, such as a melanoma, that expresses SLC45A2. A vaccine composition for pharmaceutical use in a subject may comprise a SLC45A2 peptide composition disclosed herein and a pharmaceutically acceptable carrier. Alternately, an antibody that selectively binds to a SLC45A2 peptide-HLA-A2 complex may be included in a pharmaceutically acceptable carrier.

The phrases "pharmaceutical," "pharmaceutically acceptable," or "pharmacologically acceptable" refers to molecular entities and compositions that do not produce an adverse, allergic or other untoward reaction when administered to an animal, such as, for example, a human, as appropriate. As used herein, "pharmaceutically acceptable carrier" includes any and all solvents, dispersion media, coatings, surfactants, antioxidants, preservatives (*e.g*., antibacterial agents, antifungal agents), isotonic agents, absorption delaying agents, salts, preservatives, drugs, drug stabilizers, gels, binders, excipients, disintegration agents, lubricants, sweetening agents, flavoring agents, dyes, such like materials and combinations thereof, as would be known to one of ordinary skill in the art (see, for example, Remington: The Science and Practice of Pharmacy, 21st edition, Pharmaceutical Press, 2011). Except insofar as any conventional carrier is incompatible with the active ingredient, its use in the vaccine compositions as described herein is contemplated.

As used herein, a "protective immune response" refers to a response by the immune system of a mammalian host to a cancer. A protective immune response may provide a therapeutic effect for the treatment of a cancer, *e.g*., decreasing tumor size, increasing survival, *etc.*

A vaccine composition as described herein may comprise a SLC45A2 peptide, an anti-(SLC45A2 peptide-HLA-A2 complex) antibody, or an anti-(SLC45A2 peptide-HLA-A24 complex) antibody as described herein. SLC45A2 peptides to be included in a pharmaceutical preparation may selectively bind HLA-A2 or HLA-A24. A vaccine composition comprising a SLC45A2 peptide or an antibody that selectively binds to either a SLC45A2 peptide-HLA-A2 complex or a SLC45A2 peptide-HLA-A24 complex may be used to induce a protective immune response against a cancer that expresses SLC45A2.

A person having ordinary skill in the medical arts will appreciate that the actual dosage amount of a vaccine composition administered to an animal or human patient can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

Vaccine compositions may comprise, for example, at least about 0.1% of a SLC45A2 peptide, anti-(SLC45A2 peptide-HLA-A2 complex) antibody, or anti-(SLC45A2 peptide-HLA-A24 complex) antibody. The active compound may also comprise between about 2% to about 75% of the weight of the unit, or between about 25% to about 60%, for example, and any range derivable therein. As with many vaccine compositions, frequency of administration, as well as dosage, will vary among members of a population of animals or humans in ways that are predictable by one skilled in the art of immunology. By way of nonlimiting example, the pharmaceutical compositions and vaccines may be administered by injection (*e.g.,* intracutaneous, intramuscular, intravenous or subcutaneous), intranasally (*e.g.,* by aspiration) or orally. Between 1 and 3 doses may be administered for a 1-36 week period. Preferably, 3 doses are administered, at intervals of 3-4 months, and booster vaccinations may be given periodically thereafter.

A "suitable dose" is an amount of an SLC45A2 peptide, anti-(SLC45A2 peptide-HLA-A2 complex) antibody, or anti-(SLC45A2 peptide-HLA-A24 complex) antibody that, when administered as described above, is capable of raising an immune response in an immunized patient against a cancer. In general, the amount of peptide present in a suitable dose (or produced in situ by the nucleic acid in a dose) may range from about 0.01-100 mg per kg of host, from about 0.01-100 mg, preferably about 0.05-50 mg and more preferably about 0.1-10 mg. A SLC45A2 peptide may be administered in a dose of from about 0.25 mg to about 1 mg per each vaccine dose.

A vaccine composition as described herein may comprise different types of carriers depending on whether it is to be administered in solid, liquid or aerosol form, and whether it needs to be sterile for such routes of administration as injection. A vaccine composition disclosed herein can be administered intravenously, intradermally, intraarterially, intraperitoneally, intralesionally, intracranially, intraarticularly, intraprostaticaly, intrapleurally, intratracheally, intranasally, intravitreally, intravaginally, intrarectally, topically, intratumorally, intramuscularly, intraperitoneally, subcutaneously, subconjunctivally, intravesicularlly, mucosally, intrapericardially, intraumbilically, intraocularly, orally, topically, locally, and by inhalation, injection, infusion, continuous infusion, lavage, and localized perfusion. A vaccine composition may also be administered to a subject *via* a catheter, in cremes, in lipid compositions, by ballistic particulate delivery, or by other method or any combination of the forgoing as would be known to one of ordinary skill in the art (see, for example, Remington: The Science and Practice of Pharmacy, 21st Ed. Lippincott Williams and Wilkins, 2005).

While any suitable carrier known to those of ordinary skill in the art may be employed in the pharmaceutical compositions as described herein, the type of carrier will vary depending on the mode of administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (e.g., polylactic galactide) may also be employed as carriers for the pharmaceutical compositions described herein. Suitable biodegradable microspheres are disclosed, for example, in U.S. Patents 4,897,268 and 5,075,109.

The vaccine composition may be administered by microstructured transdermal or ballistic particulate delivery. Microstructures as carriers for vaccine formulation are a desirable configuration for vaccine applications and are widely known in the art (Gerstel and Place 1976 (U.S. Patent 3,964,482); Ganderton and McAinsh 1974 (U.S. Patent 3,814,097); U.S. Patents 5,797,898, 5,770,219 and 5,783,208, and U.S. Patent Application 2005/0065463). Such a vaccine composition formulated for ballistic particulate delivery may comprise an isolated SLC45A2 peptide disclosed herein immobilized on a surface of a support substrate. A support substrate can include, but is not limited to, a microcapsule, a microparticle, a microsphere, a nanocapsule, a nanoparticle, a nanosphere, or a combination thereof.

Microstructures or ballistic particles that serve as a support substrate for an SLC45A2 peptide, anti-(SLC45A2 peptide-HLA-A2 complex) antibody, or anti-(SLC45A2 peptide-HLA-A24 complex) antibody disclosed herein may be comprised of biodegradable material and non-biodegradable material, and such support substrates may be comprised of synthetic polymers, silica, lipids, carbohydrates, proteins, lectins, ionic agents, crosslinkers, and other microstructure components available in the art. Protocols and reagents for the immobilization of a peptide described herein to a support substrate composed of such materials are widely available commercially and in the art.

A vaccine composition may also comprise an immobilized or encapsulated SLC45A2 peptide, anti-(SLC45A2 peptide-HLA-A2 complex) antibody, or anti-(SLC45A2 peptide-HLA-A24 complex) antibody disclosed herein and a support substrate. A support substrate can include, but is not limited to, a lipid microsphere, a lipid nanoparticle, an ethosome, a liposome, a niosome, a phospholipid, a sphingosome, a surfactant, a transferosome, an emulsion, or a combination thereof. The formation and use of liposomes and other lipid nano- and microcarrier formulations is generally known to those of ordinary skill in the art, and the use of liposomes, microparticles, nanocapsules and the like have gained widespread use in delivery of therapeutics (e.g., U.S. Patent 5,741,516). Numerous methods of liposome and liposome-like preparations as potential drug carriers, including encapsulation of peptides, have been reviewed (U.S. Patents 5,567,434; 5,552,157; 5,565,213; 5,738,868 and 5,795,587).

In addition to the methods of delivery described herein, a number of alternative techniques are also contemplated for administering the disclosed vaccine compositions. By way of nonlimiting example, a vaccine composition may be administered by sonophoresis (*i.e.,* ultrasound) which has been used and described in U.S. Patent 5,656,016 for enhancing the rate and efficacy of drug permeation into and through the circulatory system; intraosseous injection (U.S. Patent 5,779,708), or feedback-controlled delivery (U.S. Patent 5,697,899).

Any of a variety of adjuvants may be employed in the vaccines as described herein to nonspecifically enhance the immune response. Most adjuvants contain a substance designed to protect the antigen from rapid catabolism, such as aluminum hydroxide or mineral oil, and a nonspecific stimulator of immune responses, such as lipid A, *Bortadella pertussis* or *Mycobacterium tuberculosis.* Suitable adjuvants are commercially available as, for example, Freund's Incomplete Adjuvant and Freund's Complete Adjuvant (Difco Laboratories, Detroit, Mich.) and Merck Adjuvant 65 (Merck and Company, Inc., Rahway, N.J.). Other suitable adjuvants include alum, biodegradable microspheres, monophosphoryl lipid A and quil A.

A peptide may be formulated into a composition in a neutral or salt form. Pharmaceutically acceptable salts, include the acid addition salts (formed with the free amino groups of the protein) and which are formed with inorganic acids such as, for example, hydrochloric or phosphoric acids, or such organic acids such as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

In any case, the composition may comprise various antioxidants to retard oxidation of one or more component. Additionally, the prevention of the action of microorganisms can be brought about by preservatives such as various antibacterial and antifungal agents, including but not limited to parabens (e.g., methylparabens, propylparabens), chlorobutanol, phenol, sorbic acid, thimerosal or combinations thereof.

Sterile injectable solutions are prepared by incorporating the active peptides in the required amount in the appropriate solvent with various of the other ingredients enumerated above, as required, followed by filtered sterilization. Generally, dispersions are prepared by incorporating the various sterilized active ingredients into a sterile vehicle that contains the basic dispersion medium and/or the other ingredients. In the case of sterile powders for the preparation of sterile injectable solutions, suspensions or emulsion, the preferred methods of preparation are vacuum-drying or freeze-drying techniques which yield a powder of the active ingredient plus any additional desired ingredient from a previously sterile-filtered liquid medium thereof. The liquid medium should be suitably buffered if necessary and the liquid diluent first rendered isotonic prior to injection with sufficient saline or glucose. The preparation of highly concentrated compositions for direct injection is also contemplated, where the use of DMSO as solvent is envisioned to result in extremely rapid penetration, delivering high concentrations of the active agents to a small area.

The composition must be stable under the conditions of manufacture and storage, and preserved against the contaminating action of microorganisms, such as bacteria and fungi. It will be appreciated that endotoxin contamination should be kept minimally at a safe level, for example, less that 0.5 ng/mg protein.

Prolonged absorption of an injectable composition can be brought about by the use in the compositions of agents delaying absorption, such as, for example, aluminum monostearate, gelatin or combinations thereof.

### A. Detection and Vaccination Kits

A SLC45A2 peptide, an anti-(SLC45A2 peptide-HLA-A2 complex) antibody, anti-(SLC45A2 peptide-HLA-A24 complex) antibody, or an anti-SLC45A2 peptide antibody as described herein may be included in a kit. The SLC45A2 peptide or antibody in the kit may be detectably labeled or immobilized on a surface of a support substrate also comprised in the kit. The SLC45A2 peptide(s) or antibody may, for example, be provided in the kit in a suitable form, such as sterile, lyophilized, or both.

The support substrate comprised in a kit as described herein may be selected based on the method to be performed. By way of nonlimiting example, a support substrate may be a multi-well plate or microplate, a membrane, a filter, a paper, an emulsion, a bead, a microbead, a microsphere, a nanobead, a nanosphere, a nanoparticle, an ethosome, a liposome, a niosome, a transferosome, a dipstick, a card, a celluloid strip, a glass slide, a microslide, a biosensor, a lateral flow apparatus, a microchip, a comb, a silica particle, a magnetic particle, or a self-assembling monolayer.

As appropriate to the method being performed, a kit may further comprise one or more apparatuses for delivery of a composition to a subject or for otherwise handling a composition as described herein. By way of nonlimiting example, a kit may include an apparatus that is a syringe, an eye dropper, a ballistic particle applicator (e.g., applicators disclosed in U.S. Patents 5,797,898, 5,770,219 and 5,783,208, and U.S. Patent Application 2005/0065463), a scoopula, a microslide cover, a test strip holder or cover, and such like.

A detection reagent for labeling a component of the kit may optionally be comprised in a kit for performing a method as described herein. The labeling or detection reagent may be selected from a group comprising reagents used commonly in the art and including, without limitation, radioactive elements, enzymes, molecules which absorb light in the UV range, and fluorophores such as fluorescein, rhodamine, auramine, Texas Red, AMCA blue and Lucifer Yellow. In addition, a kit is provided comprising one or more container means and a BST protein agent already labeled with a detection reagent selected from a group comprising a radioactive element, an enzyme, a molecule which absorbs light in the UV range, and a fluorophore.

When reagents and/or components comprising a kit are provided in a lyophilized form (lyophilisate) or as a dry powder, the lyophilisate or powder can be reconstituted by the addition of a suitable solvent. The solvent may be a sterile, pharmaceutically acceptable buffer and/or other diluent. It is envisioned that such a solvent may also be provided as part of a kit.

When the components of a kit are provided in one and/or more liquid solutions, the liquid solution may be, by way of non-limiting example, a sterile, aqueous solution. The compositions may also be formulated into an administrative composition. In this case, the container means may itself be a syringe, pipette, topical applicator or the like, from which the formulation may be applied to an affected area of the body, injected into a subject, and/or applied to or mixed with the other components of the kit.

### V. EXAMPLES

### Example 1

### Materials and Methods

### Donors, cell lines and antibodies

Peripheral blood (PB) samples were obtained from healthy donors with HLA A*0201 and HLA A*2402.

Melanoma cell lines were maintained in RPMI1640 with 4mM L-glutamine, 1mM non-essential amino acids, 10mM sodium pyruvate and 50U/ml penicillin, 50mg/ml streptomycin and 10% FBS (TCB). Uveal melanomas were cultured with RPMI1640 including 10% FBS, 50U/ml penicillin, 50mg/ml and streptomycin. LCL used as feeder cells and cultured with RPMI 1640 containing 10% FBS, 50U/ml penicillin, 50mg/ml and streptomycin. CTL media for T cell culture contained 10% FBS, 2mM L-glutamine, β-mercaptoethanol, 50U/ml penicillin and 50mg/ml streptomycin.

### HLA immunoprecipitation and detection of bound peptides by tandem mass spectrometry

Tumor-associated peptides were directly eluted from HLA class I molecules isolated from fresh tumor tissue specimens or tumor cell lines. Tumor specimens (∼250 mg) were sliced into small pieces and digested in an enzymatic cocktail buffer in serum-free RPMI1640 medium until complete digestion, then washed and tumor cells lysed and supernatant collected. After measuring the protein concentration, tumor cell lysates were incubated with W6/32 antibody (specific for HLA-A, -B, and -C) then purified using resin beads. HLA class I-bound peptides were eluted and the presence of HLA was confirmed by Western Blot analysis. Positive elute fractions were analyzed by mass spectrometry, as described below.

For discovery phase tandem mass spectrometry (MS/MS), eluted MHC class I-bound peptides were injected onto a high-sensitivity HPLC system (Dionex 3000 RSLC), separated by reversed-phase chromatography in 0.1% formic acid water-acetonitrile on 1.8 micron C18 (Agilent Technologies) and analyzed on an Orbitrap Elite mass spectrometer (Thermo Scientific) using data-dependent acquisition. The Mascot algorithm searched acquired MS/MS spectra against the SwissProt complete human protein database using 10 ppm parent mass tolerance, 0.8 d fragment ion tolerance, Met oxidation, no enzyme selectivity. Search results were cross-referenced with the appropriate MHC-binding specificities using NetMHC 3.4 [101].

### Generation and expansion of SLC45A2-specific CD8 T cells

Tumor antigen-specific CTLs were generated with a manner previously described (Li 2005). Leukapheresis PBMCs positive for HLA-A*0201 were stimulated by autologous DC pulsed with tumor antigen peptide. For induction of dendritic cell, adherent PBMCs were cultured with GMCSF and IL-4 in AIM-V medium (Invitrogen Life Technologies) for 6 days and then added IL1b, IL-6, TNF-a an PGE2 for maturation. After 1 day, mature DCs were pulsed with 40ug/ml peptide at 2X10^6 cells/ml of 1% human serum albumin (HAS)/PBS in the present of 3ug/ml β2-microglubulin for 4hr at room temperature. After washing with 1%HSA/PBS, DCs were mixed with PBMCs at 1.5X10^6 cell /ml/well in 48 well plate. IL-21 (30ng/ml) was added initially and 3∼4 days after culture. IL-2 and IL-7 were added 1 day after secondary stimulation to expand activated -specific T cells.

Six days after secondary stimulation, cells were stained with SLC45A2382-390 peptide /MHC-PE-conjugated tetramer and CD8-APC antibody, and then CD8 and tetramer-positive cells were sorted by ARIA II. The sorted SLC45A2-specific CD8 T cells were expanded by Rapid Expansion Protocol (REP) with feeder cells of PBL and LCL underIL-21.

### Peptide -MHC tetramer staining

SLC45A2-specific CD8 T cells were confirmed by staining with tetramer of SLC45A2₃₈₂₋₃₉₀ peptide/MHC complex for HLA A*0201 or SLC45A2₃₉₃₋₄₀₂ peptide/MHC complex for HLA A*2402. CD8 T cells were incubated with PE-conjugated tetramer for 20 mins, washed and then stained with APC-conjugated CD8 antibody for 15mins in room temperature. After washing, cells were analyzed by flow cytometry (LSRFortessa X-20 Analyzer). Tetramers of HLA-A*A0201 and HLA-A*A2402 containing SLSC45A2₃₈₂₋₃₉₀ SLC45A2₃₉₃₋₄₀₂ respectively were purchased form Fred Hutchinson Cancer Research Center.

### TCR repertoire analysis of SLC45A2-specific CD8 T cells

To assess TCR V_{β} repertoire, the IOTest Beta Mark TCR-V_{β} Repertoire kit was used. This kit includes antibodies covering 24 TCR-V_{β} antigens of TCR-V_{β} regions and approximately 70% of the normal human TCR-V_{β} repertoire: V_{β} 1, V_{β} 2, V_{β} 3, V_{β} 4, V_{β} 5.1, V_{β} 5.2, V_{β} 5.3, V_{β} 7.1, V_{β} 7.2, V_{β} 8, V_{β} 9, V_{β} 11, V_{β} 12, V_{β} 13.1, V_{β} 13.2, V_{β} 13.6, V_{β} 14, V_{β} 16, V_{β} 17, V_{β} 18, V_{β} 20, V_{β} 21.3, V_{β} 22, and V_{β} 23. These antibodies were conjugated with Fluorescein isothiocyanate (FITC) or phycoerythrin (PE). When TCR-V_{β} repertoire assay was performed, anti-CD8 allophycocyanin (APC) were added.

### ₅₁Chromium release assay

SLC45A2-specific CD8 T cells were assayed for specific lysis of SLC45A2 -expressing or not expressing targets using standard ₅₁Chromium (₅₁Cr) release assay. Targets were labeled with 100 uCi of ⁵¹Cr for 2hrs and after three times washing, the labeled targets plated triplicated well at a 2000 targets per well. Effector cells were incubated with targets as various effector: target (E: T) ratio. After 4hours, 30ul of supernatant was collected from each well and the ⁵¹Cr was measured by a gamma counter. The percentage of specific lysis was calculated.

### Peptide binding assay

SLC45A2, Mart-1 and g100-specific CD8 T cells (1X10^5 cells) were incubated with T2 cells (4X10^4 cell) pre-incubated with SLC45A2₃₈₂₋₃₉₀, M₂₇₋₃₅ or G₁₅₄₋₁₆₂ peptide respectively at various concentrations (100, 10, 1, 0.1, 0.01, 0 nM). 48 hours after incubation, IFN-γ production was measured by ELISA assay.

### RT-PCR and quantitative RT-PCR

For analysis of mRNA expression of melanocyte differentiation antigen, RT-PCR was performed. Briefly, total cellular RNA was extracted by guanidine-isothiocyanate/cesium chloride procedure. cDNA from 1ug of RNA was synthesized with high-capacity cDNA reverse transcription kits and amplified by 30 cycles of PCR with primers specific for SLC45A2, MART-1, gp100, and tyrosinase. Primer sequences are listed in supplemental table1. PCR production was run on a 2% agarose gel and visualized by Gel Red.

Real time PCR was done with primers of SLC45A2, MART-1, gp100, and tyrosinase using power syber green PCR master mix (Applied biosystems life technologies). Values were normalized by the amount of the gene encoding GAPDH.

Mel526 and A375 (BRAF V600E+) and MeWo (BRAF wild type) were treated with BRAF V600E inhibitor, dabrafenib (50nM), or MEK inhibitor, Trametinib (50nM) (GlaxoSmithKline) or both for 48 hours. Untreated melanomas were used as control.

### RNAseq Analysis

Whole Transcriptome Seq (RNA-Seq) was performed by the Avera Institute for Human Genetics on tumor samples using the Illumina TruSeq Stranded Total RNA kit with Ribo-Zero Gold. Approximately 200 million Paired-End reads were used for each tumor RNA sample. BCL (raw output of Illumina HigSeq) files was processed using ISIS v2.4.60 for demultiplexing and conversion to FASTQ format. FASTQ files and sequence reads were aligned to the genome (Hg19) using BWA using parameters suitable for a specific run (for example, 3 mis-matches with 2 in the first 40 seed regions for a 51 bases sequencing run). The aligned BAM files were then subjected to mark duplication, re-alignment, and recalibration using Picard and GATK programs before any downstream analyses. RNASeq data was processed using TopHat, TopHat-Fusion, and Cufflinks algorithms.

### Statistical analysis

Data analysis was performed using GraphPad prism version 6.0e. Normally distributed data were analyzed using parametric tests (Anova or unpaired t-test). Statistical test differences were considered significant if p values were <0.05.

### Example 2

### Expression of SLC45A2 is highly restricted to melanomas

The expression of SLC45A2 was evaluated in various melanoma cells including cutaneous, uveal and mucosal melanoma cells. SLC45A2 mRNA expression was analyzed in tumor cells of various types including melanoma cell lines by RT-PCR (Table 1). SLC45A2 mRNA was detected in most of the melanoma cells, but not in tumor cells of other types (FIGS. 1A-C). The expression of SLC45A2 was also examined in metastatic melanoma cells which originated from different sites (Table 2). 11 of the 16 metastatic melanoma cells tested expressed SLC45A2 mRNA (FIG. 1A). The expression ratio of SLC45A2 was compared with that of other melanoma differentiation antigens (MDA) such as MART-1, gp100 and tyrosinase in various melanoma cells. It was found that the different melanoma differentiation antigens showed a similar expression ratio, 78.7∼84.8% (Table 3). Comparison of MDA gene expression in melanomas and primary melanocytes is shown in FIGS. 13A-B.

**Table 1. Human MDA gene primer sequences for RT-PCR.**

| **Gene** | **Sense** | **Anti-sense** | **size** |
|---|---|---|---|
| SLC45A2 | CTGGCCGCCACATCTATAAAT (SEQ ID NO: 3) | GTAGCAGAACTCTCTTCCGAAC (SEQ ID NO: 4) | 125 bp |
| MART-1 | ACAGTGATCCTGGGAGTCTTAC (SEQ ID NO: 5) | TTGAAGAGACACTTTGCTGTCC (SEQ ID NO: 6) | 168 bp |
| gp100 | AGGTGCCTTTCTCCGTGAG (SEQ ID NO: 7) | GCTTCAGCCAGATAGCCACT (SEQ ID NO: 8) | 128bp |
| Tyrosinase | GCAAAGCATACCATCAGCTCA (SEQ ID NO: 9) | GCAGTGCATCCATTGACACAT (SEQ ID NO: 10) | 145bp |
| GAPDH | AAT CCC ATC ACC ATC TTC CA (SEQ ID NO: 11) | TGG ACT CCA CGA CGT ACT CA (SEQ ID NO: 12) | 94bp |

**Table 2. SLC45A2 expression and HLA type in melanoma cells.**

| **Melanoma name** | **SLC45A2 expression** | **HLA Type** |
|---|---|---|
| **Melanoma** | | |
| Mel 888 | + | A01/A24 |
| Mel 888+A2 | + | A01/A24 |
| Mel 526 | + | A02/A03 |
| Mel 624 | + | A02/A03 |
| A375 | - | A01/A02 |
| WM793 | - | A02/A |
| MeWo | + | A02/A |
| FM88 | + | A02/A |
| FM6 | + | A02/A |
| A2058 | + | A02/A |

| **Uveal melanoma** | | |
|---|---|---|
| 202 | + | A01/A03 |
| 92.1 | + | A03 |
| OMM1 | + | A02 |
| UPMD1 | + | A02 |
| UPMD2 | + | A24/A68 |

| **Mucosal melanoma** | | |
|---|---|---|
| Mel2170 | + | A02/A01 |
| Mel2042 | + | A03/A11 |
| Melanoma name | SLC45A2 expression | HLA Type |

| **Metastatic melanoma** | | |
|---|---|---|
| Mel2381 | + | A02/A68 |
| Mel2508 | + | A02/A24 |
| Mel2400 | + | A02/A29 |
| Mel2412 | + | A02/A03 |
| Mel2382 | + | A02/A26 |
| Mel2559 | + | A02/A29 |
| Mel2461 | - | A02/A01 |
| Mel2333 | - | A02/A68 |
| Mel2216 | + | A02/A24 |
| Mel2391 | + | A02/A24 |
| Mel2423 | - | A03/A24 |
| Mel2492 | + | A24/A26 |
| Me12792 | - | A24/A11 |
| Mel2297 | + | A03/A11 |
| Mel2425 | + | A01/A11 |
| Mel2698 | - | A11/A31 |

**Table 3. Comparison of the MDA expression ratio in melanoma cell lines.**

| **mRNA expression** | **Positive #** | **Negative #** | **Total #** | **Ratio (%)** |
|---|---|---|---|---|
| **SLC45A2** | 26 | 7 | 33 | 78.7 |
| **MART-1** | 28 | 5 | 33 | 84.8 |
| **Gp100** | 28 | 5 | 33 | 84.8 |
| **Tyrosinase** | 26 | 7 | 33 | 78.7 |

The relative gene expression of SLC45A2 was analyzed in normal tissues and cancer tissues using Genotype-Tissue Expression (GTEx) and The Cancer Genome Atlas (TCGA) portal data. Expression of the SLC45A2 gene was either not detected or detected at a low level in various normal tissues (FIG. 8). However, MART-1 and gp100 showed significantly higher expression in many normal tissue samples, particularly normal skin. SLC45A2 showed high expression in cutaneous and uveal melanoma tissues along with the other MDAs (FIG. 8). SLC45A2 was expressed in most of the cutaneous melanoma cells including metastatic melanoma cells, but not in tumor cells of other types, indicating that the expression of SLC45A2 is melanoma-specific.

### Example 3

### Identification of HLA A*0201 and A*2402 restricted SLC45A2-derived CD8 T cell epitope

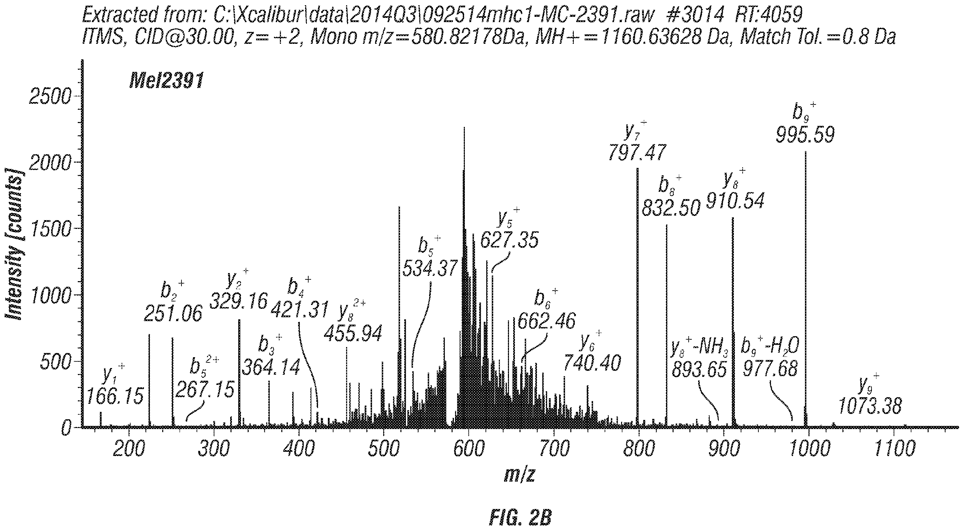

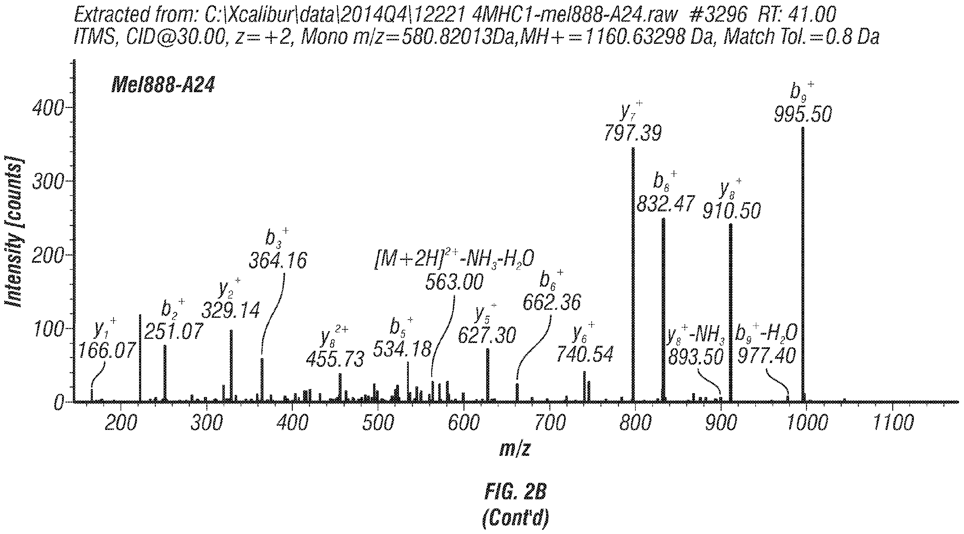

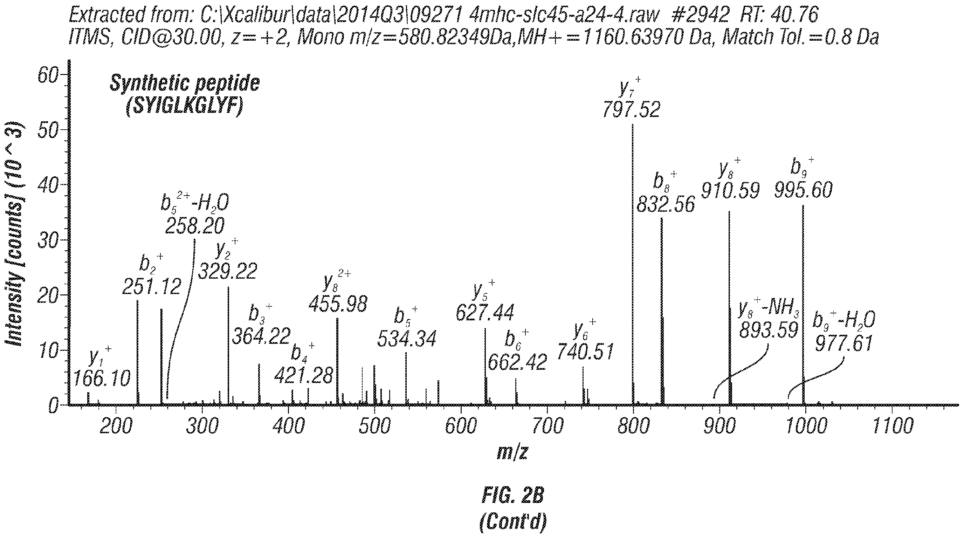

Several MDACC patient-derived melanoma cell lines and fresh melanoma tumor specimens were analyzed for surface HLA class I bound peptides using immunoprecipitation of HLA-A,B,C, acid elution, and tandem mass spectrometry (MS/MS). Six different SLC45A2-derived peptides predicted to bind 4 different HLA alleles were detected from multiple specimens, demonstrating that they constitute shared tumor-associated antigens (Table 4). Additional evidence of these peptides being naturally processed and presented is shown in Table 5: Mel888 melanoma cells were transduced with lentiviral vectors to express either HLA-A*0201, A*2402, or A*0301. Immunopeptidome analysis was performed on parental and transduced Mel888 cells as described above. In this way, 5 of the 6 peptides identified in Table 4 were also detected in the transduced cells, but only if they expressed the appropriate HLA allele (Table 5 and FIG. 2). As shown, novel SLC45A2₃₈₂₋₃₉₀ (SLYSYFQKV; SEQ ID NO:1) and SLC45A2₃₉₃₋₄₀₂ (SYIGLKGLYF, SEQ ID NO:2) peptides were confirmed by elution from Mel888 transduced with HLA A*0201 or A*2402 using MS analysis.

**Table 4. SLC45A2-derived peptides detected by mass spectrometric analysis of melanoma cell lines.**

| **SLC45A2 peptide** | **Peptide position** | **Number of melanomas in which peptide was detected** | **HLA restriction element** | **Worldwide HLA prevalence** | **Predicted HLA binding affinity (nM)** |
|---|---|---|---|---|---|
| **SLYSYFQKV** (SEQ ID NO: 1) | 382 - 390 | 6 | A*0201 | 28% | 7 |
| **RLLGTEFQV** (SEQ ID NO: 13) | 209 - 217 | 8 | | | 11 |
| **SYIGLKGLYF** (SEQ ID NO: 2) | 393 - 402 | 3 | A*2402 | 34% | 51 |
| **VWFLSPILGF** (SEQ ID NO: 14) | 73 - 82 | 2 | | | 76 |
| **ALIANPRRK** (SEQ ID NO: 15) | 129 - 137 | 2 | A*0301 | 8% | 108 |
| **SGQAGRHIY** (SEQ ID NO: 16) | 5 - 13 | 2 | A*3002 | 3% | 41 |

**Table 5. Confirmation of natural peptide processing and presentation by HLA transduction.**

| | **HLA-transduced SLC45A2+ melanoma cell line** | | | | | **Predicted HLA binding affinity (nM)** | | | | |
|---|---|---|---|---|---|---|---|---|---|---|
| **SLC45A2 peptide** | **Mel888 PARENTAL** | **Mel888 A*0201** | **Mel888 A*0301** | **Mel888 A*1101** | **Mel888 A*2402** | **A*0101** | **A*0201** | **A*0301** | **A*1101** | A***02401** |
| **SLYSYFQKV** (SEQ ID NO: 1) | - | 20 | - | - | - | 19814 | **7** | 4450 | 10314 | 12016 |
| **RLLGTEFQV** (SEQ ID NO: 13) | - | 27 | - | - | - | 21492 | **11** | 12802 | 17100 | 14058 |
| **SYIGLKGLYF** (SEQ ID NO: 2) | 34 | 23 | 31 | 34 | 61 | 12377 | 24841 | 24562 | 26349 | **51** |
| **VWFLSPILGF** (SEQ ID NO: 14) | - | - | - | - | 17 | 11843 | 18995 | 24560 | 28804 | **76** |
| **ALIANPRRK** (SEQ ID NO: 15) | - | - | 24 | - | - | 21477 | 22597 | **108** | 320 | 30124 |

The Ion score of the detected peptide is listed on the left side of the table below the specific HLA-transduced SLC45A2 melanoma cell line. A "-" in the table indicates that the peptide was not detected.

### Example 4

### Induction of SLC45A2-specific CD8 T cells

SLC45A2-specific CD8 T cells were generated by stimulating autologous HLA-A*0201 or A*2402 positive PBMCs with SLC45A2₃₈₂₋₃₉₀ or SLC45A2₃₉₃₋₄₀₂ peptide-pulsed-dendritic cells (DCs) treated with IL-21. According to the time schedule depicted in FIG. 3A, HLA-A*0201 and A*2402 restricted PBMCs were stimulated by SLC45A2₃₈₂₋₃₉₀ and SLC45A2₃₉₃₋₄₀₂ peptide-pulsed-DC, respectively, treated with IL-21. After a secondary stimulation, SLC45A2-tetramer-positive CD8 T cells were induced at a frequency of about 2-25% of the lymphocyte-gated population and 6.68-30% of the CD8-gated population (FIG. 3B top panel). SLC45A2-tetramer-positive CD8 T cells were sorted and expanded by the Rapid Expansion Protocol (REP). After REP, the frequency of the SLC45A2-tetramer positive population was 91-99% of the lymphocyte-gated population and 97-99% of the CD8-gated population (FIG.3B middle panel). SLC45A2-tetramer positive CD8 T cells were barely observed in these healthy donor PBMCs. SLC45A2-specific CD8 T cells were also successfully induced from PBMCs of two other HLA A*0201 or A*2402-restricted healthy donors (FIG. 10).

To investigate the clonality of the SLC45A2-specific CD8 T cells, the TCR Vβ repertoire was analyzed using Vβ antibodies corresponding to 24 different specificities. The Vβ-chain of the SLC45A2-specific CD8 T cells induced from each donor included Vβ3, Vβ14, Vβ18, Vβ21.3, and Yβ23 (FIG.3B bottom panel, FIG. 10). SLC45A2 tetramer-positive CD8 T cells, sorted from a single well and expanded by REP, displayed one major population of Vβ with a range of 92-99%.

For phenotype analysis of the induced SLC45A2-specific CD8 T cells, expression of CD45RA, CCR7, CD62L and CD28 was tested in SLC45A2-tetramer positive CD8 T cells by flow cytometry. SLC45A2-specific CD8 T cells did not express CD45RA, CCR7, and CD62L, but did express CD28, suggesting they have a phenotype similar to effector memory T cells (FIG.3C).

### Example 5

### Antigen specific recognition and function of SLC45A2-specific CD8 T cells

To determine the ability of SLC45A2-specific CD8 T cells to recognize and kill SLC45A2-expressing melanoma cells, a standard ⁵¹Cr release assay was performed using SLC45A2-expressing melanoma cell lines at various E:T ratios. Mel526, Mel624, FM6, and MeWo cells were used as targets, which express both SLC45A2 and HLA A*0201 (Table 2). It was found that SLC45A2-specific CD8 T cells effectively killed the various melanoma cell lines (FIG. 4A). This showed that the SLC45A2-specific CD8 T cells could recognize the SLC45A2 epitope SLYSYFQKV (SEQ ID NO: 1) endogenously processed by melanoma cells. As controls, HLA-A*0201 melanoma cells negative for SLC45A2 expression (A375) and melanoma cells that expressed SLC45A2 but were HLA-A*0201-negative (Mel888) were not lysed by SLC45A2-specific CD8 T cells. Transduction of Mel888 cells with HLA-A*0201 rendered the cells susceptible to lysis by SLC45A2-specific CD8 T cells, indicating the SLC45A2-specific CD8 T cells have an HLA-A*0201 restricted response. Cytotoxic activity of the SLC45A2-specific CD8 T cells was also examined against metastatic melanoma cells derived from different tissues. All metastatic melanoma cells that were used in this study were positive for HLA-A*0201.

In addition, it was found that T cells specific for the HLA-A*2402-restricted epitope SYIGLKGLYF (SEQ ID NO: 2) lysed a panel of melanoma cell lines expressing HLA-A*2402: Similar to the HLA-A*0201 restricted T cells, the A*2402-restricted T cells effectively lysed Mel888, Mel2381, Mel2508, Mel2400, Mel2412 and Mel2559 cells expressing SLC45A2 protein; however, A*2402-positive Mel2461 and Mel2333 cells that did not express SLC45A2 protein were not lysed (FIG. 4B).

### Example 6

### Functional avidity of SLC45A2-specific CTLs

To evaluate the functional avidity for target recognition of SLC45A2-specific CD8 T cells, the ability of SLC45A2-specific CTLs to produce IFN-γ was examined in the presence of T2 cells pre-incubated with SLC45A2₃₈₂₋₃₉₀ peptide (SLYSYFQKV, SEQ ID NO: 1) at various concentrations using an ELISA assay. T2 cells pre-incubated with HLA-A*0201 binding control peptides MART-1₂₇₋₃₅ (AAGIGILTV, SEQ ID NO: 17) and gp100₁₅₄₋₁₆₂ (KTWGQYWQV, SEQ ID NO: 18) were used to confirm a peptide-specific response of the SLC45A2-specific CD8 T cells. The binding capacity of the respective peptides was compared between SLC45A2-, MART-1- and gp100-specific CTLs (Fig. 4C). Interestingly, SCL45A2-specific CD8 T cells were capable of high IFN-γ production in response to T2 cells pulsed with peptide as low as 0.1ug/ml and showed decreased IFN-gamma production at a peptide concentration of 0.01ug/ml (FIG. 4C upper panel). IFN-γ production by MART-1-specific CD8 T cells was significantly less than the IFN-γ production by SCL45A2-specific CD8 T cells at the same peptide concentrations (FIG.4C bottom panel). In addition, the binding affinity of the gp100-specific CD8 T cells was similar to the binding affinity of SLC45A2-specific CD8 T cells. These data suggest that SLC45A2-specific CD8 T cells could respond with a high affinity to targets expressing SLC45A2. These results are consistent with the predicted HLA binding affinities of the SLC45A2, gp100, and MART-1 peptides for HLA-A*0201, which are 7 nM, 9 nM, and 2498 nM, respectively.

### Example 7

### Low expression of SLC45A2 and low cytotoxic activity of SLC45A2-specific CD8 T cells towards melanocytes

Since SLC45A2 is a melanocyte differentiation protein, SLC45A2 expression was investigated in normal melanocytes and the cytotoxic activity of SLC45A2-specific CD8 T cells against normal melanocytes was determined. Human epidermal melanocytes, 3C0661 and 4C0197, were isolated from lightly pigmented neonatal skin. 3C0661 expressed both HLA A*0201 and A*2402 and 4C0197 was HLA A*0201-positive and A*2402-negative (FIG. 11). As shown in FIG.5A, SLC45A2 was expressed by these melanocytes, but the expression was significantly less in the melanocytes compared with melanoma cells. However, other melanocyte differentiation proteins such as MART-1, gp100 and tyrosinase were expressed in melanocytes at a level similar to their expression in melanoma cells (Fig. 5A). In addition, the RNA expression levels of SLC45A2, MART-1, gp100 and tyrosinase were compared between melanocytes and melanoma cells using RNA sequencing and TCGA data. It was found that the expression of SLC45A2 was lower in melanocytescompared with melanoma, whereas the expression of MART-1 and gp100 in melanocytes was significantly higher and was similar to their expression in melanoma cells (Table 6).

**Table 6. Melanocyte differentiation antigen expression in melanomas and normal tissues.**

| | **Tissue expression (RNAseq, mean TPM)** | **PMEL** | **MART1** | **TYR** | **SLC45A2** |
|---|---|---|---|---|---|
| ***Gtex normal tissue*** | **Heart** | 0.81 | 0.29 | 0.05 | **0.07** |
| | **Brain** | 0.15 | 0.21 | 0.09 | **0.27** |
| | **Skin** | 42.9 | 11.1 | 8.63 | **0.55** |
| | | | | | |
| ***Cell lines*** | **Melanomas (SLC45A2⁺)** | 2323 | 267 | 386 | **97** |
| | **Primary Melanocytes** | 5354 | 2804 | 2268 | **39** |
| | | | | | |
| ***TCGA tumors*** | **Cutaneous Melanoma** | 6706 | 754 | 675 | **128** |
| | **Uveal melanoma** | 8466 | 1777 | 393 | **69** |

The cytotoxicity of SLC45A2-, MART-1- and gp100-specific CD8 T cells was investigated against primary melanocytes derived from 2 HLA-A*0201-positive healthy donors. MART-1 and gp100-specific CD8 T cells showed 35-42% and 55-65% cytotoxicity against melanocytes, respectively, but surprisingly, SLC45A2-specific CD8 T cells showed less than 8% cytotoxicity against the same melanocytes (FIG. 5B). In addition, SLC45A2-specific CD8 T cells generated from other healthy donors also had low toxicity against melanocytes (FIGS. 12A-B). In addition, HLA A*2402-restricted SLC45A2-specific CD8 T cells were found not to be cytotoxic against A*2402 positive melanocytes, 3C0661 (FIG. 5C). These results suggest that, unlike other MDAs such as MART-1 and gp100, SLC45A2-specific CD8 T cells can effectively kill melanoma cells without destruction of normal melanocytes.

### Example 8

### SLC45A2 expression and cytotoxicity of SLC45A2-specific CD8 T cells against uveal and mucosal melanoma cells

Uveal melanoma cells 202, 92.1, UPMD1 and UPMD2 were derived from primary tumors and OMM1 cells were derived from a metastatic tumor. All uveal melanoma cells that were used in this study expressed SLC45A2 mRNA (FIG. 6A).

The cytotoxicity of SLC45A2-specific CD8 T cells against uveal melanoma cells was investigated. OMM1 cells and 202 cells positive for SLC45A2 were observed to be killed by HLA A*0201 restricted SLC45A2-specific CD8 T cells (FIG. 6B). Next, the cytoxicity of MART-1- and gp100-specific CD8 T cells was examined against uveal melanomas. MART-1-specific CD8 T cells showed less cytotoxicity for uveal melanoma cells than SLC45A2- and gp100-specific CD8 T cells. A*2402-restricted SLC45A2-specific CD8 T cells killed UPMD2 cells expressing SLC45A2 and HLA A*2402. When UPMD2 cells were pulsed with A*2402- restricted peptide, SLC45A2₃₉₃₋₄₀₂, the cytotoxicity of SLC45A2-specific CD8 T cells was increased. UPMD1 cells positive for SLC45A2 but negative HLA A*2402 were not lysed by A*2402-restricted SLC45A2-specific CD8 T cells (FIG. 6C).

In mucosal melanoma, SLC45A2 expression and cytotoxicity by SLC45A2-specific CD8 T cells was tested. Mucosal melanoma cells expressed SLC45A2 at levels similar to the expression of other MDAs (FIG. 6D). A*0201 restricted SLC45A2-specific CD8 T cells lysed 2170 mucosal melanoma cells expressing SLC45A2 and HLA A*0201, but not 2042 cells expressing SLC45A2 and not HLA A*0201 (FIG. 6E). Cytotoxic activity against mucosal melanoma cells was similar between SLC45A2, MART-1 and gp100 -specific CD8 T cells. These results indicate that SLC45A2-specific CD8 T cells can effectively kill uveal melanoma cells and mucosal melanoma cells expressing SLC45A2 and the appropriate HLA type.

### Example 9

### Enhanced SLC45A2 expression and killing of melanoma cells following treatment with MAPK pathway inhibitors

Like other melanocyte differentiation proteins such as MART-1, gp100 and tyrosinase, the SLC45A2 gene is regulated by the MITF transcription factor (J Biol Chem, 2002, 277:402-406, Gen Soci Ame, 2008 178:761-769). MITF protein levels are suppressed by oncogenic BRAF through ERK-mediated phosphorylation and degradation (J Cell Biol, 2005, 170:703-708). Thus, it was next investigated whether BRAF or MEK inhibitors can modulate SLC45A2 expression. Melanoma cells were treated with a specific inhibitor of BRAF V600E, dabrafenib (50nM), or a MEK inhibitor, Trametinib (50nM), or both for 48 hours and mRNA expression of SLC45A2 and MART-1 was analyzed by RT-PCR. As shown in FIG. 7A, a significant increase of SLC45A2 and MART-1 was observed in Mel526 cells (expressing mutated BRAF V600E) after treatment with a BRAF or MEK inhibitors , while MeWo cells (expressing wild type BRAF) did not show an increased expression of SLC45A2 and MART-1. To assess the killing effect of SCL45A2-specific CD8 T cells against melanoma cells after treatment with BRAF and MEK inhibitors, a ⁵¹Cr release assay was performed in melanoma cells treated with a BRAF inhibitor, a MEK inhibitor or both for 48 hours. SCL45A2-specific CD8 T cells showed increased cytotoxicity in melanoma cells treated with a BRAF inhibitor, a MEK inhibitor, or both compared with that of untreated melanoma cells (FIG. 7B). These data indicated that MAPK pathway inhibition increases expression of SLC45A2 regulated by MITF, which enhances target recognition and the cytotoxicity of SLC45A2-specific CD8 T cells.

Conclusions: Importantly, SLC45A-specific CD8 T cells effectively killed melanoma cells, but not normal melanocytes, whereas MART-1 and gp100-specific CD8 T cells killed both melanocytes and melanoma cells. In addition, treatment with BRAF and MEK inhibitors increased SLC45A expression, target recognition and cytoxicity of SLC45A2-specific CD8 T cells in melanoma cells. Collectively, this study identified novel HLA-A*0201 and A*2402-restricted peptides SLC45A2₃₈₂₋₃₉₀ and SLC45A2₃₉₃₋₄₀₂, respectively, and showed that SLC45A2, as a melanoma differentiation antigen, can be an effective target with high efficacy and low toxicity for immunotherapy in melanoma. This finding of SLC45A2 as a melanoma-specific antigen could be important for the development of adoptive T cell immunotherapy.

### REFERENCES

U.S. Patent 3,814,097
U.S. Patent 3,817,837
U.S. Patent 3,850,752
U.S. Patent 3,939,350
U.S. Patent 3,964,482
U.S. Patent 3,996,345
U.S. Patent 4,196,265
U.S. Patent 4,275,149
U.S. Patent 4,277,437
U.S. Patent 4,366,241
U.S. Patent 4,373,071
U.S. Patent 4,401,796
U.S. Patent 4,458,066
U.S. Patent 4,472,509
U.S. Patent 4,598,049
U.S. Patent 4,897,268
U.S. Patent 4,938,948
U.S. Patent 5,021,236
U.S. Patent 5,075,109
U.S. Patent 5,196,066
U.S. Patent 5,262,357
U.S. Patent 5,505,928
U.S. Patent 5,552,157
U.S. Patent 5,565,213
U.S. Patent 5,567,434
U.S. Patent 5,656,016
U.S. Patent 5,690,807
U.S. Patent 5,697,899
U.S. Patent 5,738,868
U.S. Patent 5,741,516
U.S. Patent 5,741,957
U.S. Patent 5,750,172
U.S. Patent 5,756,687
U.S. Patent 5,770,219
U.S. Patent 5,770,219
U.S. Patent 5,779,708
U.S. Patent 5,783,208
U.S. Patent 5,795,587
U.S. Patent 5,797,898
U.S. Patent 5,827,690
U.S. Patent 5,990,479
U.S. Patent 6,048,616
U.S. Patent 6,091,001
U.S. Patent 6,274,323
U.S. Patent 6,630,307
U.S. Patent 7,910,109
U.S. Patent Appln. 2005/0065463
Aggarwal et al., Mod. Pathol., 22:206-215, 2008.
Altman et al. Science 274(5284):94-6, 1996.
Antibodies: A Laboratory Manual, Cold Spring Harbor Laboratory, 1988.
Atherton et al., Biol. of Reproduction, 32, 155-171, 1985.
Baird et al., Scand. J. Immunol., 60(4):363-71, 2004.
Baraldo et al., Infect. Immun., 73(9):5835-41, 2005.
Bendandi et al., Nat. Med., 5:1171-1177, 1999.
Berberian et al., Science, 261:1588-1591, 1993.
Bertinetti et al., Cancer Res., 66:4496-4502, 2006.
Bijker et al., J. Immunol., 179:5033-5040, 2007.
Blanchard and Shastri, Curr. Opin. Immunol., 20:82-88, 2008.
Burrows et al., Trends Immunol., 27:11-16, 2006.
Campbell, In: Monoclonal Antibody Technology, Laboratory Tech. Biochem. Molec. Biol., Vol. 13, Burden and Von Knippenberg (Eds.), 75-83, Amsterdam, Elsevier, 1984.
Celluzzi et al., J. Exp. Med., 183 283-287, 1996.
Chapuis, A. G., et al. (2016). "T-Cell Therapy Using IL-21 Primed CTL Combined with CTLA-4 Blockade Results in Longterm Cell Persistence and Durable Tumor Regression " J Clin Oncol, in press.
Cheson and Leonard, N. Eng. J. Med., 359:613-626, 2008.
Cleary et al., J. Biol. Chem., 269(29):18747-9, 1994.
Coiffier et al., N. Eng. J. Med., 346:235-242, 2002.
Collins et al., Nature, 371:626-629, 1994.
Coulie, P. G., et al. (1994). "A new gene coding for a differentiation antigen recognized by autologous cytolytic T lymphocytes on HLA-A2 melanomas [see comments]." Journal of Experimental Medicine 180(1): 35-42.
Cumber et al., J. Immunol.., 149B:120-126, 1992.
De Jager et al., Semin. Nucl. Med. 23(2), 165-179, 1993.
Dholakia et al., J. Biol. Chem., 264, 20638-20642, 1989.
Di Nicola et al., Blood, 113:18-27, 2009.
Doolittle and Ben-Zeev, Methods Mol. Biol., 109, :215-237, 1999.
Drin et al., AAPS Pharm. Sci., 4(4):E26, 2002.
Du et al., J. Pept. Res., 51:235-243, 1998.
Dudley et al., J. Immunol., 26(4):332-342, 2003.
Elliott and O'Hare, Cell, 88:23-233, 1997.
European Patent 0 216 846
European Patent 0 256 055
European Patent 0 323 997
European Patent Appl. 89303964.4
Frankel and Pabo, Cell, 55:189-1193, 1988.
Gefter et al., Somatic Cell Genet., 3:231-236, 1977.
Goding, In: Monoclonal Antibodies: Principles and Practice, 2d ed., Orlando, Fla., Academic Press, 60-61, 65-66, 71-74, 1986.
Goeddel, Methods Enzymol., 185:3-7, 1990.
Gritti et al., Blood, 92:368-373, 1998.
Gulbis and Galand, Hum. Pathol. 24(12), 1271-1285, 1993.
Guo et al., Nature, 360:364-366, 1992.
Gupta et al., Biomaterials, 26:3995-4021, 2005.
Harada et al., Canc. Res. 61:1089-1094.
Hawkins et al., Hum. Gene Ther., 21(6):665-72, 2010.
Herling et al., Am. J. Surg. Pathol., 31:1123-1129, 2007.
Herling et al., Blood, 114(21):4675-4686, 2009.
Hida et al., Cancer Immunol. Immunotherapy, 51:219-228, 2002.
Houot and Levy, Blood Rev., 23:137-142, 2009.
Hoyer et al., J. Immunol., 175:864-873, 2005.
Hoyer et al., Proc. Natl. Acad. Sci. USA, 99:14392-14397, 2002.
Inoges et al., J. Natl. Cancer Inst., 98:1292-1301, 2006.
Irvine et al., Nature, 419:845-849, 2002.
Kang et al., Blood, 105:1288-1294, 2005.
Kang et al., Science, 240:1034-1036, 1988.
Kawakami, Y., et al. (1995). "Recognition of multiple epitopes in the human melanoma antigen gp100 by tumor-infiltrating T lymphocytes associated with in vivo tumor regression." Journal of Immunology 154(8): 3961-3968.
Khatoon et al., Ann. of Neurology, 26, 210-219, 1989.
King et al., J. Biol. Chem., 269, 10210-10218, 1989.
Kohler and Milstein, Eur. J. Immunol., 6, 511-519, 1976.
Kohler and Milstein, Nature, 256, 495-497, 1975.
Kohler et al., Methods Enzymol., 178:3, 1989.
Kreier et al., In: Infection, Resistance and Immunity, Harper and Row, New York, 1991.
Kwak et al., N. Eng. J. Med., 327:1209-1215, 1992.
Laine et al., Molec. Cell, 6:395-407, 2000.
Lee et al., J. Immunol. Methods, 331:13-26, 2008.
Lenert et al., Science, 248:1639-1643, 1990.
Liddy N. et al., Monoclonal TCR-redirected tumor cell killing. Nat Med 18(6):980-7, 2012.
Lin et al., Immunol. Cell Biol., 86:353-362, 2008.
Lin et al., J. Biol. Chem., 270:4255-14258, 1995.
Liu et al., Cell Mol. Biol., 49(2):209-216, 2003.
Malyguine et al., J. Transl. Med., 2:9, 2004.
Marcus et al., Blood, 105:1417-1423, 2005.
Maus et al., Nat. Biotech., 20:143-148, 2002.
McKee et al., J Transl Med. 3:35, 2005.
McLaughlin et al., J. Clin. Oncol., 16:2825-2833, 1998.
Melief and van der Burg, Nat. Rev. Cancer, 8:351-360, 2008.
Moorthy et al., PLoS Med., 1(2):e33, 2004.
Nakamura et al., In: Enzyme Immunoassays: Heterogeneous and Homogeneous Systems, Chapter 27, 1987.
Narducci et al., Cancer Res., 57:5452-5456, 1997b.
Narducci et al., Cancer Res., 60:2095-2100, 2000.
Narducci et al., Oncogene, 15:919-926, 1997a.
Narducci et al., Proc. Natl. Acad. Sci. USA, 99:11712-11717, 2002.
Navarrete et al., Blood, 117:1483-1491, 2011.
Neelapu et al., Nat. Med., 11:986-991, 2005.
Neelapu et al., Blood, 15:109(12):5160-5163, 2007.
Nestle et al., Nat. Med., 4:328, 1998.
Oates J. et al. ImmTACs for targeted cancer therapy: Why, what, how, and which (2015) Mol Immunol. 2015 Oct;67(2 Pt A):67-74.
O'Shannessy et al., J. Immun. Meth., 99, 153-161, 1987.
Owens and Haley, J. Biol. Chem., 259, 14843-14848, 1987.
Pack et al., Biochem., 31:1579-1584, 1992.
Park and Neelapu, Br. J. Haemat., 142:179-191, 2008.
PCT Publn. WO 99/26299
Pekarsky et al., Proc. Natl. Acad. Sci. USA, 105:19643-19648, 2008.
Pollack, S. M., R. L. Jones, E. A. Farrar, S. R. Riddell, and C. Yee. 2014. Tetramer Guided Cell Sorter Assisted Production of Clinical Grade Autologous NY-ESO-1 Specific CD8+ T Cells. Journal of Immunotherapy of Cancer in press.
Popescu et al. Blood, 15:109(12):5407-5410, 2007.
Potter and Haley, Meth. Enzymol., 91, 613-633, 1983.
Quintarelli et al., Blood, 117:3353-3362, 2011.
Ramuz et al., Int. J Oncol., 26:151-157, 2005.
Remington, In: The Science and Practice of Pharmacy, 21st Ed., Lippincott Williams and Wilkins, 2005.
Remington, In: The Science and Practice of Pharmacy, 21st Ed., Pharmaceutical Press, 2011.
Ribas et al., Trends Immunol.,24:58-61, 2003.
Riddell et al., J. Immunol., 128(2):189-201, 1990.
Rojas et al., J. Biol. Chem., 271:27456-27461, 1996.
Rojas et al., Proc. West. Pharmacol. Soc., 41:55-56, 1998.
Said et al., Lab. Invest. 81:555-564, 2001.
Samino et al., J. Biol. Chem., 281:6358-6365, 2006.
Sasso et al., J. Immunol., 142:2778-2783, 1989.
Seaman BJ, et al. Audiovestibular dysfunction associated with adoptive cell immunotherapy for melanoma. Otolaryngology--head and neck surgery; Otolaryngol Head Neck Surg May 17, 2012.
Schuster et al., J. Clin. Oncol., 29(20):2787-94, 2011.
Schwarze et al., Trends in Cell Biol., 10:290-295, 2000.
Schwenzer et al., J. Biol. Chem., 274:19368-19374, 1999.
Shorki et al., J. Immunol., 146:936-940, 1991.
Silvermann et al., J. Clin. Invest., 96:417-426, 1995.
Skull and Kemp, Arch. Dis. Child., 74:527-530, 1996.
Stryhn et al., Eur. J. Immunol., 30:3089-3099, 2000.
Teitell, Nat. Rev. Cancer, 5:640-648, 2005.
Timmerman et al., Blood, 99:1517-1526, 2002.
Virgilio et al., Proc. Natl. Acad. Sci. USA, 95:3885-3889, 1998.
Wakim et al., Nature, 471:629-632, 2011.
Wang and Wang, Nat. Biotechnol., 20:149-154, 2002.
Yee et al., J. Immunol. Methods, 261(1-2):1-20, 2002.
Yee, C., et al.,. Adoptive T cell therapy using antigen-specific CD8+ T cell clones for the treatment of patients with metastatic melanoma: in vivo persistence, migration, and antitumor effect of transferred T cells. Proc Natl Acad Sci U S A 99: 16168-16173, 2002b.
Yee et al. The use of endogenous T cells for adoptive transfer. Immunological reviews 257: 250-263. 2014.
Young et al., J. Exp. Med., 183:-11, 1996.
Zwaveling et al., J. Immunol., 169:350-358, 2002.

## Claims

1. An isolated peptide 35 amino acids in length or less for use in the treatment of melanoma excluding uveal melanoma, wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402.

2. A cell culture media comprising the peptide as defined in claim 1.

3. A pharmaceutical composition comprising a peptide 35 amino acids in length or less for use in the treatment of melanoma excluding uveal melanoma, wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402 and an excipient.

4. The pharmaceutical composition for use of claim 3, wherein
- the pharmaceutical preparation is formulated for parenteral administration, intravenous injection, intramuscular injection, inhalation, or subcutaneous injection,
- the pharmaceutical preparation is formulated for injection or inhalation as a nasal spray, and/or
- the peptide is comprised in a liposome, lipid-containing nanoparticle, or in a lipid-based carrier.

5. The peptide as defined in claim 1 or a pharmaceutical composition as defined in claim 3 or 4 for use in the treatment of melanoma excluding uveal melanoma, wherein a second anticancer therapy is administered to the subject to be treated and wherein the second anticancer therapy is preferably chosen from the list of: a chemotherapy, a radiotherapy, an immunotherapy, or a surgery, wherein the peptide or the pharmaceutical composition is administered to said subject in an amount effective to promote cytotoxic T lymphocytes (CTL) in the subject to lyse or kill cancerous cells in the subject.

6. An *in vitro* method for inducing a population of T cells to proliferate, comprising contacting T cells *in vitro* with a peptide in an amount sufficient to bind a HLA-A*0201 or a HLA-A2 in the T cells and promote proliferation of one or more of the T cells, preferably wherein the T cells are cytotoxic T lymphocytes (CTL) and/or CD8+ T cells, wherein the peptide is 35 amino acids in length or less and wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402.

7. The T cells as obtained by the method of claim 6 for use in the treatment of melanoma excluding uveal melanoma, wherein the T cells are administered to a subject, preferably wherein the subject is a human.

8. An antibody that selectively binds to a peptide or that selectively binds to a peptide - HLA-A2 complex for use in the treatment of melanoma excluding uveal melanoma, preferably wherein said antibody is a monoclonal antibody, is comprised in polyclonal antisera, is an antibody fragment, is a human or humanized antibody, or is comprised in a fusion construct, a soluble fusion construct, an ImmTAC, or an immunotoxin, wherein the peptide is 35 amino acids in length or less and wherein the peptide comprises the sequence of SLC45A2₃₈₂₋₃₉₀ (SEQ ID NO:1) or SLC45A2₃₉₃₋₄₀₂ (SEQ ID NO:2) or a sequence having at least 90 % identity to SLC45A2₃₈₂₋₃₉₀ (SEQ ID NO:1) or SLC45A2₃₉₃₋₄₀₂ (SEQ ID NO:2), wherein the peptide selectively binds HLA-A2, HLA-A*0201, HLA-A24, or HLA-A*2402.

9. The peptide for use of claim 1 or 5, the cell culture medium as defined in claim 2, the pharmaceutical composition for use of claim 3, 4 or 5, the method as defined in claim 6, or the antibody for use of claim 8, wherein the peptide is 30 amino acids in length or less, 25 amino acids in length or less, 20 amino acids in length or less or 15 amino acids in length or less.

10. The peptide for use of claim 1 or 5, the cell culture medium as defined in claim 2, the pharmaceutical composition for use of claim 3, 4 or 5, the method as defined in claim 6, or the antibody for use of claim 8, wherein
- the peptide comprises or consists of SLC45A2₃₈₂₋₃₉₀ (SEQ ID NO:1) and wherein the peptide selectively binds HLA-A2 or HLA-A*0201, or
- the peptide comprises or consists of SLC45A2₃₉₃₋₄₀₂ (SEQ ID NO:2) and wherein the peptide selectively binds HLA-A24 or HLA-A*2402.

11. The peptide for use of claim 1 or 5, or the pharmaceutical composition for use of claim 3, 4 or 5, wherein the melanoma is cutaneous melanoma, mucosal melanoma or metastatic melanoma.

## Patentansprüche

1. Isoliertes Peptid mit einer Länge von 35 Aminosäuren oder weniger zur Verwendung bei der Behandlung eines Melanoms, mit Ausnahme des Uvealmelanoms, wobei das Peptid die Sequenz von SLC45A2₃₈₂₋₃₉₀ (SEQ ID NO:1) oder SLC45A2₃₉₃₋₄₀₂ (SEQ ID NO:2) oder eine Sequenz, die mindestens 90% Identität mit SLC45A2₃₈₂₋₃₉₀ (SEQ ID NO:1) oder SLC45A2₃₉₃₋₄₀₂(SEQ ID NO:2) aufweist, umfasst, wobei das Peptid selektiv HLA-A2, HLA-A*0201, HLA-A24 oder HLA-A*2402 bindet.

2. Zellkulturmedium, das das wie in Anspruch 1 definierte Peptid umfasst.

3. Pharmazeutische Zusammensetzung, die ein Peptid mit einer Länge von 35 Aminosäuren oder weniger zur Verwendung bei der Behandlung eines Melanoms, mit Ausnahme des Uvealmelanoms, und einen Exzipienten umfasst, wobei das Peptid die Sequenz von SLC45A2₃₈₂₋₃₉₀ (SEQ ID NO:1) oder SLC45A2₃₉₃₋₄₀₂ (SEQ ID NO:2) oder eine Sequenz, die mindestens 90% Identität mit SLC45A2₃₈₂₋₃₉₀ (SEQ ID NO:1) oder SLC45A2₃₉₃₋₄₀₂(SEQ ID NO:2) aufweist, umfasst, wobei das Peptid selektiv HLA-A2, HLA-A*0201, HLA-A24 oder HLA-A*2402 bindet.

4. Pharmazeutische Zusammensetzung zur Verwendung nach Anspruch 3, wobei
- die pharmazeutische Formulierung für parenterale Verabreichung, intravenöse Injektion, intramuskuläre Injektion, Inhalation oder subkutane Injektion formuliert ist,
- die pharmazeutische Formulierung für die Injektion oder Inhalation als Nasenspray formuliert ist, und/oder
- das Peptid in einem Liposom, einem Lipid-enthaltenden Nanopartikel oder in einem Träger auf Lipidbasis umfasst ist.

5. Peptid wie in Anspruch 1 definiert oder pharmazeutische Zusammensetzung wie in Anspruch 3 oder 4 definiert zur Verwendung bei der Behandlung eines Melanoms, mit Ausnahme des Uvealmelanoms, wobei dem zu behandelnden Individuum eine zweite Antikrebstherapie verabreicht wird und wobei die zweite Antikrebstherapie bevorzugt ausgewählt ist aus der Liste aus: einer Chemotherapie, einer Strahlentherapie, einer Immuntherapie oder einem chirurgischen Eingriff, wobei das Peptid oder die pharmazeutische Zusammensetzung dem Individuum in einer Menge verabreicht wird, die wirksam ist, cytotoxische T-Lymphocyten (cytotoxic T lymphocytes; CTL) in dem Individuum zu fördern, um Krebszellen in dem Individuum zu lysieren oder abzutöten.

6. *In vitro*-Verfahren, um eine T-Zell-Population zur Proliferation zu induzieren, umfassend das Inkontaktbringen von T-Zellen *in vitro* mit einem Peptid in einer Menge, die ausreichend ist, um ein HLA-A*0201 oder ein HLA-A2 in den T-Zellen zu binden und die Proliferation einer oder mehrerer der T-Zellen zu fördern, bevorzugt wobei die T-Zellen cytotoxische T-Lymphocyten (CTL) und/oder CD8⁺-T-Zellen sind, wobei das Peptid eine Länge von 35 Aminosäuren oder weniger aufweist und wobei das Peptid die Sequenz von SLC45A2₃₈₂₋₃₉₀ (SEQ ID NO:1) oder SLC45A2₃₉₃₋₄₀₂ (SEQ ID NO:2) oder eine Sequenz, die mindestens 90% Identität mit SLC45A2₃₈₂₋₃₉₀ (SEQ ID NO:1) oder SLC45A2₃₉₃₋₄₀₂ (SEQ ID NO:2) aufweist, umfasst, wobei das Peptid selektiv HLA-A2, HLA-A*0201, HLA-A24 oder HLA-A*2402 bindet.

7. T-Zellen wie durch das Verfahren nach Anspruch 6 erhalten zur Verwendung bei der Behandlung eines Melanoms, mit Ausnahme des Uvealmelanoms, wobei die T-Zellen einem Individuum verabreicht werden, bevorzugt wobei das Individuum ein Mensch ist.

8. Antikörper, der selektiv an ein Peptid bindet oder der selektiv an einen Peptid-HLA-A2-Komplex bindet, zur Verwendung bei der Behandlung eines Melanoms, mit Ausnahme des Uvealmelanoms, bevorzugt wobei der Antikörper ein monoclonaler Antikörper ist, in polyclonalen Antiseren umfasst ist, ein Antikörperfragment ist, ein humaner oder humanisierter Antikörper ist oder in einem Fusionskonstrukt, einem löslichen Fusionskonstrukt, einem ImmTAC oder einem Immuntoxin umfasst ist, wobei das Peptid eine Länge von 35 Aminosäuren oder weniger aufweist und wobei das Peptid die Sequenz von SLC45A2₃₈₂₋₃₉₀ (SEQ ID NO:1) oder SLC45A2₃₉₃₋₄₀₂ (SEQ ID NO:2) oder eine Sequenz, die mindestens 90% Identität mit SLC45A2₃₈₂₋₃₉₀ (SEQ ID NO:1) oder SLC45A2₃₉₃₋₄₀₂(SEQ ID NO:2) aufweist, umfasst, wobei das Peptid selektiv HLA-A2, HLA-A*0201, HLA-A24 oder HLA-A*2402 bindet.

9. Peptid zur Verwendung nach Anspruch 1 oder 5, Zellkulturmedium wie in Anspruch 2 definiert, pharmazeutische Zusammensetzung zur Verwendung nach Anspruch 3, 4 oder 5, Verfahren wie in Anspruch 6 definiert oder Antikörper zur Verwendung nach Anspruch 8, wobei das Peptid eine Länge von 30 Aminosäuren oder weniger, eine Länge von 25 Aminosäuren oder weniger, eine Länge von 20 Aminosäuren oder weniger oder eine Länge von 15 Aminosäuren oder weniger aufweist.

10. Peptid zur Verwendung nach Anspruch 1 oder 5, Zellkulturmedium wie in Anspruch 2 definiert, pharmazeutische Zusammensetzung zur Verwendung nach Anspruch 3, 4 oder 5, Verfahren wie in Anspruch 6 definiert oder Antikörper zur Verwendung nach Anspruch 8, wobei
- das Peptid SLC45A2₃₈₂₋₃₉₀ (SEQ ID NO:1) umfasst oder daraus besteht und wobei das Peptid selektiv HLA-A2 oder HLA-A*0201 bindet, oder
- das Peptid SLC45A2₃₉₃₋₄₀₂(SEQ ID NO:2) umfasst oder daraus besteht und wobei das Peptid selektiv HLA-A24 oder HLA-A*2402 bindet.

11. Peptid zur Verwendung nach Anspruch 1 oder 5 oder pharmazeutische Zusammensetzung zur Verwendung nach Anspruch 3, 4 oder 5, wobei das Melanom kutanes Melanom, mukosales Melanom oder metastasierendes Melanom ist.

## Revendications

1. Peptide isolé d'une longueur inférieure ou égale à 35 acides aminés à utiliser dans le traitement du mélanome à l'exception du mélanome uvéal, le peptide comprenant la séquence de SLC45A2₃₈₂₋₃₉₀ (SEQ ID NO:1) ou de SLC45A2₃₉₃₋₄₀₂ (SEQ ID NO:2) ou une séquence présentant au moins 90 % d'identité avec SLC45A2₃₈₂₋₃₉₀ (SEQ ID NO:1) ou SLC45A2₃₉₃₋₄₀₂ (SEQ ID NO:2), le peptide se liant sélectivement à HLA-A2, à HLA-A*0201, à HLA-A24 ou à HLA-A*2402.

2. Milieux de culture cellulaire comprenant le peptide selon la revendication 1.

3. Composition pharmaceutique comprenant un peptide d'une longueur inférieure ou égale à 35 acides aminés à utiliser dans le traitement du mélanome à l'exception du mélanome uvéal, dans laquelle le peptide comprend la séquence de SLC45A2₃₈₂₋₃₉₀ (SEQ ID NO:1) ou de SLC45A2₃₉₃₋₄₀₂ (SEQ ID NO:2) ou une séquence présentant au moins 90 % d'identité avec SLC45A2₃₈₂₋₃₉₀ (SEQ ID NO:1) ou SLC45A2₃₉₃₋₄₀₂ (SEQ ID NO:2), dans laquelle le peptide se lie sélectivement à HLA-A2, à HLA-A*0201, à HLA-A24 ou à HLA-A*2402 et à un excipient.

4. Composition pharmaceutique à utiliser selon la revendication 3, dans laquelle
- la préparation pharmaceutique est formulée pour une administration parentérale, une injection intraveineuse, une injection intramusculaire, une inhalation, ou une injection sous-cutanée,
- la préparation pharmaceutique est formulée pour une injection ou une inhalation sous forme de pulvérisation nasale, et/ou
- le peptide est compris dans un liposome, une nanoparticule contenant un lipide ou un support à base de lipide.

5. Peptide selon la revendication 1 ou composition pharmaceutique selon la revendication 3 ou 4 à utiliser dans le traitement du mélanome à l'exception du mélanome uvéal, dans lequel une seconde thérapie anticancéreuse est administrée au sujet à traiter et dans lequel la seconde thérapie anticancéreuse est de préférence choisie dans la liste suivante : une chimiothérapie, une radiothérapie, une immunothérapie ou une chirurgie, le peptide ou la composition pharmaceutique étant administré audit sujet dans une quantité efficace pour favoriser la prolifération des lymphocytes T cytotoxiques (LTC) chez le sujet afin de lyser ou d'éliminer les cellules cancéreuses chez le sujet.

6. Procédé *in vitro* pour induire la prolifération d'une population de cellules T, comprenant la mise en contact de cellules T *in vitro* avec un peptide dans une quantité suffisante pour lier un HLA-A*0201 ou un HLA-A2 dans les cellules T et favoriser la prolifération d'une ou de plusieurs des cellules T, de préférence dans lequel les cellules T sont des lymphocytes T cytotoxiques (LTC) et/ou des cellules T CD8+, dans lequel le peptide a une longueur inférieure ou égale à 35 acides aminés et dans lequel le peptide comprend la séquence de SLC45A2₃₈₂₋₃₉₀ (SEQ ID NO:1) ou de SLC45A2₃₉₃₋₄₀₂ (SEQ ID NO:2) ou une séquence présentant au moins 90 % d'identité avec SLC45A2₃₈₂₋₃₉₀ (SEQ ID NO:1) ou SLC45A2₃₉₃₋₄₀₂ (SEQ ID NO:2), dans lequel le peptide se lie sélectivement à HLA-A2, à HLA-A*0201, à HLA-A24 ou à HLA-A*2402.

7. Cellules T telles qu'obtenues par le procédé selon la revendication 6 à utiliser dans le traitement du mélanome à l'exception du mélanome uvéal, les cellules T étant administrées à un sujet, le sujet étant de préférence un sujet humain.

8. Anticorps qui se lie sélectivement à un peptide ou qui se lie sélectivement à un complexe peptide-HLA-A2 à utiliser dans le traitement du mélanome à l'exception du mélanome uvéal, ledit anticorps étant de préférence un anticorps monoclonal, étant inclus dans un antisérum polyclonal, étant un fragment d'anticorps, étant un anticorps humain ou humanisé ou étant inclus dans une construction de fusion, une construction de fusion soluble, un ImmTAC ou une immunotoxine, dans lequel le peptide a une longueur inférieure ou égale à 35 acides aminés et dans lequel le peptide comprend la séquence de SLC45A2₃₈₂₋₃₉₀ (SEQ ID NO:1) ou de SLC45A2₃₉₃₋₄₀₂ (SEQ ID NO:2) ou une séquence présentant au moins 90 % d'identité avec SLC45A2₃₈₂₋₃₉₀ (SEQ ID NO:1) ou SLC45A2₃₉₃₋₄₀₂ (SEQ ID NO:2), dans lequel le peptide se lie sélectivement à HLA-A2, à HLA-A*0201, à HLA-A24 ou à HLA-A*2402.

9. Peptide à utiliser selon la revendication 1 ou 5, milieu de culture cellulaire selon la revendication 2, composition pharmaceutique à utiliser selon la revendication 3, 4 ou 5, procédé selon la revendication 6 ou anticorps à utiliser selon la revendication 8, le peptide ayant une longueur inférieure ou égale à 30 acides aminés, inférieure ou égale à 25 acides aminés, inférieure ou égale à 20 acides aminés ou inférieure ou égale à 15 acides aminés.

10. Peptide à utiliser selon la revendication 1 ou 5, milieu de culture cellulaire selon la revendication 2, composition pharmaceutique à utiliser selon la revendication 3, 4 ou 5, procédé selon la revendication 6 ou anticorps à utiliser selon la revendication 8,
- le peptide comprenant ou étant constitué de SLC45A2₃₈₂₋₃₉₀ (SEQ ID NO:1) et le peptide se liant sélectivement à HLA-A2 ou à HLA-A*0201, ou
- le peptide comprenant ou étant constitué de SLC45A2₃₉₃₋₄₀₂ (SEQ ID NO:2) et le peptide se liant sélectivement à HLA-A24 ou à HLA-A*2402.

11. Peptide à utiliser selon la revendication 1 ou 5 ou composition pharmaceutique à utiliser selon la revendication 3, 4 ou 5, dans lequel le mélanome est un mélanome cutané, un mélanome mucosal ou un mélanome métastatique.
